# EUROPEAN PATENT APPLICATION

(11) **EP 1 734 116 A2**
(43) Date of publication of application: **20.12.2006**
(21) Application number: 05721270.6
(22) Date of filing: 22.03.2005
(51) Int. Cl.: C12N 9/74, C12N 15/12, A61K 38/55, A61P 7/00, A61P 29/00

(54) **THROMBIN DERIVATIVES AND MEDICINAL COMPOSITION CONTAINING THE SAME**

(30) Priority: 19.03.2004 JP 2004080950; 08.06.2004 JP 2004170346; 26.07.2004 JP 2004217834; 29.10.2004 JP 2004315631; 04.02.2005 JP 2005029360
(71) Applicant: Chisso Corporation, Osaka-shi Osaka 530-0055 (JP); FUJIMORI KOGYO CO., LTD., Tokyo 103 (JP)
(72) Inventor: HOSOKAWA, Kazuya; c/o FUJIMORI KOGYO CO., LTD., 4-, Tokyo ;1030002 (JP); KASHIMA, Kosuke; c/o FUJIMORI KOGYO CO., LTD., 4-1, Tokyo ,1030002 (JP)
(74) Representative: Arth, Hans-Lothar
(86) International application number: PCT/JP2005/005170
(87) International publication number: WO 2005/089070

(57) **Abstract**

The present invention provides a thrombin derivative, comprising an A chain and a B chain, the B chain having an amino acid sequence in which one or more kinds of active center amino acids selected from the group consisting of serine at position 205, glycine at position 203, aspartic acid at position 99, and histidine at position 43 in an amino acid sequence of a thrombin B chain are substituted, having the properties: (1) cleaving a thrombin substrate at the ratio of 10% or less when it is reacted with the thrombin substrate in 50 mM Tris-HCl (pH 7.4) containing 0.1 M NaCl at 37°C for 3 hours, and (2) maintaining a structure of exosite I.

## Description

### [Technical Field]

The present invention relates to a thrombin derivative and to a pharmaceutical composition containing the same, particularly to an antithrombotic agent or an anti-inflammatory agent.

### [Background Art]

Thrombin is a trypsin-like serine protease which has an extremely high homology to trypsin and plays roles in a platelet aggregation response, an inflammatory response, and the like. For instance, Non-patent Document 1 describes that thrombin activates a thrombin receptor serving as a substrate, to thereby cause a platelet aggregation response, activation of vascular endothelial cells, and an imflammatory response.

Physiological actions of thrombin have been reported as follows. Non-patent Document 2 (Japanese Journal of Thrombosis and Hemostasis, vol. 10, 1999, Nos. 2 and 3) describes that an exosite I region plays an important role in substrate recognition relating to blood coagulation caused mostly by thrombin. Non-patent Document 3 (Biochemical J. (2001) 354. 309-313) describes that anhydrothrombin which is an inactivated thrombin having a binding ability to a thrombin substrate protein (hereinafter, also referred to as "thrombin substrate") has, like a wild-type thrombin, a high binding ability to a thrombin substrate capable of binding to the exosite I. In addition, Non-patent Document 3 describes that addition of a substance such as benzamidine which provides steric hindrance to an active center eliminates the binding ability of the AHT. Non-patent Document 4 (Voet: Biochemistry, 1st volume, 1996, p331-340, Tokyo Kagaku Dojin) describes that: serine proteases such as thrombin have serine, histidine, and aspartic acid in an active center thereof; a charge relay system of those three amino acids causes the expression of protease activity; and glycine at position 193 (the number 193 indicates the position of an amino acid in chymotrypsinogen and corresponds to glycine at position 203 in a thrombin B chain) is involved in transition of a Michaelis complex of the serine proteases to a tetrahedral complex.

On the basis of those reports, various modifications and alterations of thrombin have been attempted to develop an antithrombotic agent and the like. Patent Document 1 (WO 01/03740) discloses a serine protease inhibitor containing a substance (hereinafter, also referred to as "reaction inhibiting substance") which binds to a substrate of a serine protease competitively with the serine protease to thereby inhibit the reaction between the serine protease and the substrate. Further, Patent Document 1 describes that the serine protease inhibitor effectively serves as an antithrombotic agent (i.e., thrombogenesis inhibitor). Patent Document 1 also discloses as a specific example of the reaction inhibiting substance a thrombin derivative (i.e., anhydrothrombin or hereinafter, also referred to as "AHT") with a significantly decreased serine protease activity, which is obtained by reacting a serine protease thrombin with an inhibitor such as phenyl methyl sulfonyl fluoride (hereinafter, also referred to as "PMSF") to convert serine existing in an active site into dehydroalanine (hereinafter, also referred to as "anhydration").

Meanwhile, Patent Document 2 (WO 02/077031) discloses an anhydrothrombin (AHT) derivative (hereinafter, also referred to as "M-AHT") obtained by chemically modifying AHT through a reaction of a carboxyl group of the AHT with an imido. M-AHT has a selectively low binding ability to fibrinogen (hereinafter, also referred to as "Fbgn"), which exists in blood in a large amount, and can increase an effect of prolonging activated partial thromboplastin time (hereinafter, also referred to as "APTT") as compared to the AHT, and has a high antithrombotic effect.

Meanwhile, studies on thrombin derivatives having amino acid substitutions have been made. Several discussions as described below have been conducted on recombinats obtained by substituting an amino acid in the active center through gene recombination of thrombin. For instance, Non-patent Document 5 (Experimental cell research 219, 650-656 (1995)) describes the influence of a thrombin derivative, in which the active center serine is substituted by alanine, on leukocytes. In addition, Non-patent Document 5 describes that the enzymatic activity of the thrombin derivative is lost.

Non-patent Document 6 (Biochimica et Biophyscia Acta 1451 (1999) 173-186) describes: a thrombin derivative in which the glycine at position 203 in the thrombin B chain is substituted by alanine; a thrombin derivative in which the active center serine is substituted by alanine or threonine; a thrombin derivative in which the active center histidine is substituted by asparagine; and a thrombin derivative in which the active center aspartic acid is substituted by asparagine. Non-patent Document 6 describes about the enzymatic activities of those thrombin derivatives. That is, the thrombin derivative in which the active center serine is substituted by threonine, the thrombin derivative in which the active center histidine is substituted by asparagine, and the thrombin derivative in which the active center aspartic acid is substituted by asparagine each have an enzymatic activity which is decreased to be one several thousandth to several-ten thousandth as compared to that of the wild-type thrombin. In addition, the enzymatic activities of the thrombin derivative in which the glycine at position 203 in the thrombin B chain is substituted by alanine and a thrombin derivative in which the active center serine is substituted by alanine are completely lost.
However, the thrombin derivatives described in Non-patent Documents 5 and 6 still have enzymatic activities (thrombin substrate-cleaving activity) at a level which cannot be detected by the assay methods described in the respective documents, and have significantly decreased thrombin substrate-binding abilities, or have high binding abilities to Fbgn which exists in blood in a large amount. Thus, it is difficult to state that the thrombin derivatives each have an activity sufficient to serve as an antithrombotic or anti-inflammatory agent.

Patent Document 3 (JP-A-09-509045), Non-patent Document 7 (J. Biol. Chem Vol. 275, 39827-39830), Non-patent Document 8 (J. Biol. Chem Vol. 279, 26387-26394), and Non-patent Document 9 (J. Biol. Chem Vol. 277, 27581-27584) describe about thrombin derivatives each having an enzymatic activity (thrombin substrate-cleaving activity) and an anti-blood coagulation effect, obtained by the substitution of an amino acid. Those thrombin derivatives each have a maintained or enhanced binding ability to thrombomodulin (hereinafter, also referred to as "TM") and a significantly decreased Fbgn-cleaving ability, and thus each of them is a thrombin derivative which specifically binds to TM to activate protein C, to thereby exhibit an antithrombotic effect.

Patent Document 4 (JP-A-11-507542) discloses a prothrombin derivative which has a substituted active center amino acid and is intended to be used for neutralizing an anticoagulation activity of a hirudin C-terminal peptide when problems such as bleeding occurs by administration of the hirudin C-terminal peptide as an antithrombotic agent to a patient.
Patent Documents 5 and 6 describe that a thrombin derivative in which the active center serine was substituted by alanine and a thrombin derivative in which the active center serine was substituted by alanine and the active center aspartic acid was substituted by asparagine inhibited the stimulation of a thrombin receptor by thrombin in a washed platelet suspension.
However, those thrombin derivatives each have a strong binding ability to Fbgn which exists in blood in a large amount. When those thrombin derivatives are administered into blood, most of them bind to the Fbgn. Therefore, a large amount of a thrombin derivative has to be administered in order to attain an anti-thrombin receptor effect. Thus, it has been practically impossible to use each of the thrombin derivatives as an anti-thrombin receptor agent (i.e., antiplatelet agent) in blood (see Experimental Example 3 herein).
[Patent Document 1] WO 01/03740
[Patent Document 2] WO 02/077031
[Patent Document 3] WO 95/13385
[Patent Document 4] WO 96/41868
[Patent Document 5] WO 92/14750
[Patent Document 6] US 5,256,766
[Non-patent Document 1] J. Biol. Chem. 261 (1986) 15928-15933
[Non-patent Document 2] Japanese Journal of Thrombosis and Hemostasis, vol. 10, Nos. 2 and 3 (1999)
[Non-patent Document 3] Biochemical J. (2001) 354. 309-313
[Non-patent Document 4] Voet: Biochemistry, first volume of the textbook that has been translated into Japanese, 1996, p331-340, Tokyo Kagaku Dojin
[Non-patent Document 5] Experimental Cell Research 219, 650-656 (1995)
[Non-patent Document 6] Biochimica et BiophysciaActa 1451 (1999) 173-186
[Non-patent Document 7] J. Biol. Chem Vol. 275, 39827-39830
[Non-patent Document 8] J. Biol. Chem Vol. 279, 26387-26394
[Non-patent Document 9] J. Biol. Chem Vol. 277, 27581-27584

### [Disclosure of the Invention]

Thrombin derivatives such as AHT and M-AHT obtained by chemical procedures have antithrombotic effects or anti-inflammatory effects, however, the serine protease activities left therein varied because of the chemical procedures. Further, multiple steps including an alkali treatment, a regeneration treatment, and the like are necessary upon conversion into AHT, and yields thereof are from 50 to 60%. In the AHT and M-AHT, the remaining enzymatic activity (thrombin substrate-cleaving activity) of trace level decreases the anticoagulation ability of the AHT and M-AHT in some cases. In particular, it occurs more likely in the AHT.

Meanwhile, it is hardly to state that the thrombin derivative having the substituted active center amino acids of thrombin has an activity sufficient for serving as an antithrombotic agent or anti-inflammatory agent. The reason may be because each of those thrombin derivatives has a substantial thrombin substrate-cleaving activity when used as an antithrombotic agent or anti-inflammatory agent, or has a significantly decreased thrombin substrate-binding ability due to the structural change in mainly exosite I caused by the amino acid substitutions.
For instance, Non-patent Document 6 (Biochimica et BiophysciaActa 1451 (1999) 173-186) reported that a thrombin derivative in which glycine at position 203 in the thrombin B chain is substituted by alanine has completely lost the thrombin substrate-cleaving activity. However, the thrombin derivative still has a substantial thrombin substrate-cleaving activity when used as an antithrombotic agent or anti-inflammatory agent. Further, the substitutions of glycine at position 203, the active center histidine and aspartic acid in the thrombin B chain as described in Non-patent Document 6 decreased the thrombin substrate-cleaving activity to a lower level, but some combination of the substitutions caused the structural change in the exosite I, thereby leading to impairment of the thrombin substrate-binding ability.

Meanwhile, from the perspective of current status of medical treatments, three major causes of death of the Japanese are cancer standing first (30%), cardiac diseases standing second (15%), and cerebrovascular disorders standing third (13%), which account for about 60 percent of the total. Most of the cardiac diseases are coronary diseases such as cardiac infarction and angina pectoris. At present, the total of angiopathy of heart and brain accounts for almost equal percentage of cause of death to that of the cancer. However, the percentage of angiopathy will further increase in future, and therefore the development of an effective therapeutic agent is becoming a significant social demand. In addition, cerebral infarction often leaves aftereffects even when death is avoided, leading to social concerns such as ' significant burden of nursing care and medical payment.

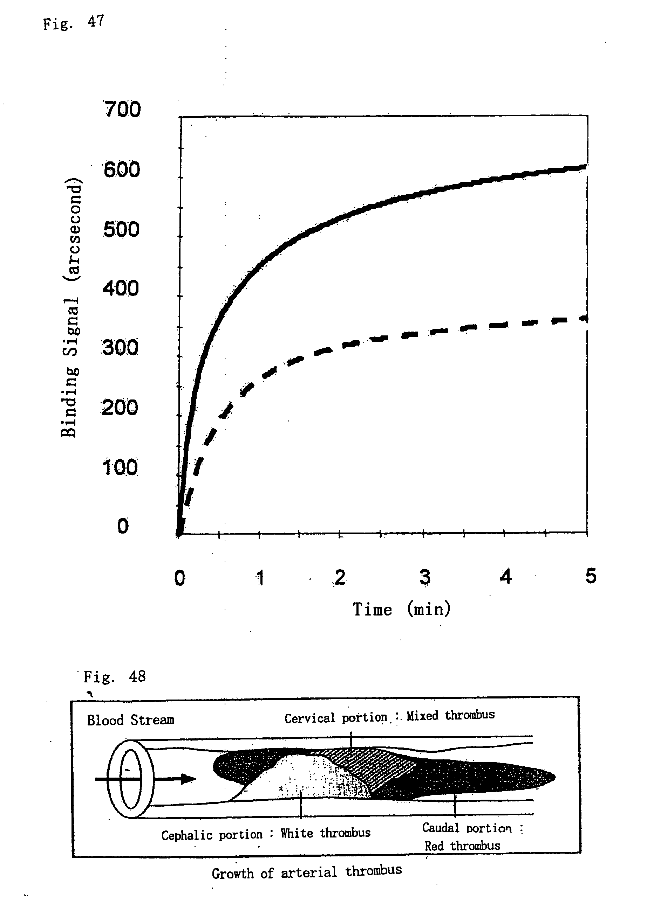

Thrombosis including cardiac infarction, angina pectoris, cerebral infarction, and the like result from combination of multiple factors. As shown in Fig. 48, in an actual cardiac infarction, it is reported that there are observed a white thrombus, subsequently a mixed thrombus at a middle position in blood flow, and a red thrombus at downstream of the blood flow.

Most of the vascular disorders in brain or heart are thrombosis. Major causes thereof include abnormal blood flow, abnormality in a coagulating component, and abnormality in a vascular endothelium, but in actuality, they in combination cause and induce thrombosis. A coagulase thrombin is the one which is involved in all of the causes and plays central roles in thrombogenesis. Thrombin produces fibrin aggregates in a final step of coagulation cascade, and at the same time, thrombin accelerates the coagulation cascade by activating factors XI, V, and VIII. Further, thrombin causes platelet aggregation and activation of endothelial cells through PAR1 which is a receptor on a platelet and a vascular endothelium. It has been reported that the activation of endothelial cells promotes coagulation of a vascular wall and the thrombogenesis proceeds owing to a negative chain reaction. In actual pathological anatomy of an arterial thrombus, the white, mixed, and red thrombi are observed in most cases, so it is revealed that inhibition of coagulation and platelets together is important in an antithrombotic treatment. Nonetheless, all of existing antithrombotic agents are classified into anticoagulants or antiplatelet agents, and the treatment is conducted by using respective drugs.
In consideration of the above-mentioned circumstances, it is desired to develop an antithrombotic agent which can be used as an antithrombotic agent and an anti-inflammatory agent which effectively inhibit (i) activation and promotion of coagulation, (ii) aggregation and adhesion of platelets, and (iii) ability of activating the vascular endothelial cells.

The inventors of the present invention have found the following facts.
That is, the inventors have found that thrombin derivatives, which comprises substitutions of one or more kinds of amino acids selected from the group consisting of serine at position 205, glycine at position 203, aspartic acid at position 99, and histidine at position 43 in a thrombin B chain, and has the thrombin substrate-cleaving activity decreased to a level at which a thrombin substrate is not substantially cleaved and in which the substitutions of the active center amino acids do not impair the structures of the exosite I and exosite II of an unsubstituted thrombin, can provide an antithrombotic effect mainly including an APTT-prolonging effect similar to that of AHT; and that the APTT-prolonging effect is one surely prolonging the APTT without being affected by incubation which results from a remaining thrombin activity (see, Experimental Example 21). Further, the inventors have found that the thrombin derivatives having a binding ability more specific to a thrombin substrate, which plays an important role in thrombogenesis, can more surely provide an antithrombotic effect or anti-inflammatory effect equal to or larger than that of AHT.

Furthermore, the inventors of the present invention have found that the binding ability to Fbgn existing in blood in a large amount can be relatively decreased as compared to the binding ability to a thrombin receptor or a blood coagulation factor 8 (hereinafter, also referred to as "FVIII") by the substitutions of an amino acids except the active center amino acids, to thereby obtain a good antithrombotic effect even with a small dosage. In addition, it has been found that the substitutions of the amino acids except the active center amino acids provide thrombin derivatives having various aspects of the effects, such as a thrombin derivative which has a high APTT-prolonging effect but with a low antiplatelet effect and a thrombin derivative which has a high APTT-prolonging effect and a high antiplatelet (only a PAR1-inhibiting) effect.

The inventors of the present invention have found that the substitutions of some additional amino acids except the active center amino acids do not impair the antithrombotic effect held by the thrombin derivative of the present invention and specifically decrease the binding ability of the thrombin derivative to TM. When the thrombin derivatives of the present invention in which amino acids except the active center amino acids are substituted are administered into a living body, the thrombin derivatives do not inhibit the activation of protein C caused by thrombin on TM. In other words, the thrombin derivatives of the present invention in which amino acids except the active center amino acids are substituted do not inhibit the antithrombotic ability inherently held by thrombin.

The inventors of the present invention have found that when the carboxyl groups of the thrombin derivatives of the present invention are modified according to the method described in Patent Document 2 (WO 02/077031), not only the APTT is prolonged but the antiplatelet effect, particularly the PAR1 activation and ristocetin-induced platelet aggregation-inhibiting effect can be imparted or controlled depending on the substitutions of the amino acids. To be specific, there can be obtained thrombin derivatives having a controlled balance of drug efficacy, such as: thrombin derivatives having an extremely high APTT-prolonging effect and a high antiplatelet effect; or thrombin derivatives having a moderate level of an APTT-prolonging effect and a strong antiplatelet effect. That is, the modification of the carboxyl groups of the thrombin derivatives of the present invention can provide thrombin derivatives having an anti-blood coagulation effect and an antiplatelet effect which can be applied to various thrombosis.
According to those findings, the inventors of the present invention thus have completed the invention.

That is, the present invention is as described hereinafter.
(1) A thrombin derivative comprising an A chain and a B chain, wherein the B chain has an amino acid sequence in which one or more kinds of active center amino acids selected from the group consisting of serine at position 205, glycine at position 203, aspartic acid at position 99, and histidine at position 43 in the amino acid sequence of a thrombin B chain are substituted, and wherein
   1) the thrombin derivative cleaves a thrombin substrate at the ratio of 10% or less when it is reacted with the thrombin substrate in 50 mM Tris-HCl (pH 7.4) containing 0.1 M NaCl at 37°C for 3 hours, and
   2) the thrombin derivative maintains a structure of exosite I.
(2) A thrombin derivative comprising an A chain and a B chain, wherein the B chain has an amino acid sequence in which one or more kinds of active center amino acids selected from the group consisting of serine at position 205, glycine at position 203, aspartic acid at position 99, and histidine at position 43 in the amino acid sequence of a thrombin B chain are substituted, wherein
   1) the thrombin derivative cleaves a thrombin substrate at the ratio of 10% or less when it is reacted with the thrombin substrate in 50 mM Tris-HCl (pH 7.4) containing 0.1 M NaCl at 37°C for 3 hours, and
   2) the thrombin derivative maintains a binding ability to hirudin C-terminal peptide-immobilized gel.
(3) The thrombin derivative according to (2), further maintaining a binding ability to heparin.
(4) The thrombin derivative according to (1) or (2), wherein the thrombin substrate is a blood coagulation factor 13.
(5) The thrombin derivative according to (1) or (2), wherein the thrombin substrate is fibrinogen.
(6) The thrombin derivative according to any one of (1) to (5), wherein the substitutions of the active center amino acids are substitutions of two or more kinds of amino acids selected from the group consisting of serine at position 205, glycine at position 203, aspartic acid at position 99, and histidine at position 43 in a thrombin B chain.
(7) The thrombin derivative according to any one of (1) to (6), wherein the substitutions of the active center amino acids comprises a substitution of serine at position 205.
(8) The thrombin derivative according to any one of (1) to (7), wherein the substitution of the active center amino acids comprises substitutions of serine at position 205 and histidine at position 43.
(9) The thrombin derivative according to (8), wherein the histidine at position 43 is substituted by alanine or serine.
(10) The thrombin derivative according to any one of (6) to (9), wherein the serine at position 205 is substituted by alanine, threonine, or glycine.
(11) The thrombin derivative according to any one of (6) to (9), wherein the serine at position 205 is substituted by alanine.
(12) The thrombin derivative according to (8), wherein the serine at position 205 and the histidine at position 43 are substituted by alanine.
(13) The thrombin derivative according to any one of (1) to (12), wherein its blood coagulation factor 8-binding ability is 10% or more of that of anhydrothrombin.
(14) The thrombin derivative according to any one of (1) to (12), wherein its blood coagulation factor 8-binding ability is 80% or more of that of anhydrothrombin.
(15) The thrombin derivative according to any one of (1) to (14), wherein VIIIA/FA, a ratio between a blood coagulation factor 8-binding ability (VIIIA) and a fibrinogen-binding ability (FA) of the thrombin derivative is 1.1 folds or more of VIIIa/Fa, a ratio between a blood coagulation factor 8-binding ability (VIIIa) and a fibrinogen-binding ability (Fa) of anhydrothrombin.
(16) The thrombin derivative according to any one of (1) to (14), wherein VIIIA/FA, a ratio between a blood coagulation factor 8-binding ability (VIIIA) and a fibrinogen-binding ability (FA) of the thrombin derivative is 1.2 folds or more of VIIIa/Fa, a ratio between a blood coagulation factor 8-binding ability (VIIIa) and a fibrinogen-binding ability (Fa) of anhydrothrombin.
(17) The thrombin derivative according to any one of (1) to (12), wherein an activated partial thromboplastin time of the thrombin derivative is 1.1 folds or more of that of anhydrothrombin.
(18) The thrombin derivative according to any one of (1) to (12), wherein its fibrinogen-binding ability has been decreased by 10% or more and its activated partial thromboplastin time has been prolonged 1.1 folds or more owing to the amino acid substitutions of the active center amino acids.
(19) A thrombin derivative comprising an A chain and a B chain, wherein the B chain has an amino acid sequence in which serine at position 205 and one or more kinds of amino acids selected from the group consisting of glycine at position 203, aspartic acid at position 99, and histidine at position 43, in the amino acid sequence of a thrombin B chain are substituted.
(20) A thrombin derivative comprising an A chain and a B chain, wherein the B chain has an amino acid sequence in which serine at position 205 and histidine at position 43 in the amino acid sequence of a thrombin B chain are substituted.
(21) The thrombin derivative according to any one of (1) to (20), wherein the B chain has an amino acid sequence in which amino acids except the active center amino acids are further substituted.
(22) The thrombin derivative according to (21), wherein the amino acids except the active center amino acids are amino acids in an exosite I region of thrombin.
(23) The thrombin derivative according to (22), wherein the amino acids in the exosite I region of thrombin are basic amino acids.
(24) The thrombin derivative according to (22), wherein the amino acids in the exosite I region of thrombin are one or more amino acids selected from the group consisting of glutamine at position 24, lysine at position 65, and lysine at position 77 in a thrombin B chain.
(25) The thrombin derivative according to any one of (21) to (24), wherein VIIIA/FA, a ratio between a blood coagulation factor 8-binding ability (VIIIA) and a fibrinogen-binding ability (FA) of the thrombin derivative is 1.1 folds or more of VIIIa/Fa, a ratio between a blood coagulation factor 8-binding ability (VIIIa) and a fibrinogen-binding ability (Fa) of a thrombin derivative before the amino acids except the active center amino acids are substituted.
(26) The thrombin derivative according to any one of (21) to (24), wherein VIIIA/FA, a ratio between a blood coagulation factor 8-binding ability (VIIIA) and a fibrinogen-binding ability (FA) of the thrombin derivative is 1.5 folds or more of VIIIa/Fa, a ratio between a blood coagulation factor 8-binding ability (VIIIa) and a fibrinogen-binding ability (Fa) of a thrombin derivative before the amino acids except the active center amino acids are substituted.
(27) The thrombin derivative according to any one of (21) to (24), said thrombin derivative having:
   any one of the effects selected from the group consisting of an APTT-prolonging effect, a thrombin receptor activation-inhibiting effect, and a ristocetin-induced platelet aggregation-inhibiting effect; and having
   a decreased thrombomodulin-binding ability as compared to that of a thrombin derivative before the amino acids except the active center amino acids are substituted.
(28) The thrombin derivative according to any one of (21) to (24), said thrombin derivative having:
   any one of the effects selected from the group consisting of an APTT-prolonging effect, a thrombin receptor activation-inhibiting effect, and a ristocetin-induced platelet aggregation-inhibiting effect; and having
   a thrombomodulin-binding ability decreased by 10% or more as compared to that of a thrombin derivative before the amino acids except the active center amino acids are substituted.
(29) The thrombin derivative according to any one of (21) to (24), wherein VIIIA/TMA, a ratio between a blood coagulation factor 8-binding ability (VIIIA) and a thrombomodulin-binding ability (TMA) of the thrombin derivative is 1.1 folds or more of VIIIa/TMa, a ratio between a blood coagulation factor 8-binding ability (VIIIa) and a thrombomodulin-binding ability (TMa) of a thrombin derivative before the amino acids except the active center amino acids are substituted.
(30) The thrombin derivative according to any one of (21) to (24), wherein VIIIA/TMA, a ratio between a blood coagulation factor 8-binding ability (VIIIA) and a thrombomodulin-binding ability (TMA) of the thrombin derivative is 1.5 folds or more of VIIIa/TMa, a ratio between a blood coagulation factor 8-binding ability (VIIIa) and a thrombomodulin-binding ability (TMa) of a thrombin derivative before the amino acids except the active center amino acids are substituted.
(31) The thrombin derivative according to (21), wherein the amino acids except the active center amino acids are amino acids in an exosite II region of thrombin, and its antithrombotic ability is maintained while its heparin-binding ability is decreased.
(32) The thrombin derivative according to (31), wherein the amino acids in the exosite II of thrombin are one or more kinds of amino acids selected from the group consisting of arginine at position 98, arginine at position 245, lysine at position 248, and lysine position 252 in a thrombin B chain.
(33) The thrombin derivative according to (31) or (32), wherein the heparin-binding ability of the thrombin derivative is 90% or less as compared to that of a thrombin before the amino acids except the active center amino acids are substituted.
(34) The thrombin derivative according to any one of (21) to (33), wherein one or more kinds of antithrombotic effects selected from the group consisting of an activated partial thromboplastin time-prolonging effect, an modified thrombin-induced platelet aggregation-inhibiting effect, and a ristocetin-induced platelet aggregation-inhibiting effect are enhanced.
(35) The thrombin derivative according to any one of (21) to (33), wherein the activated partial thromboplastin time of the thrombin derivative is 1.1 folds or more as compared to that of anhydrothrombin.
(36) The thrombin derivative according to (31) or (32), wherein the activated partial thromboplastin time of the thrombin derivative is 1.5 folds or more as compared to that of a thrombin before the amino acids except the active center amino acids are substituted, and its thrombomodulin-binding ability is decreased to 50% or less as compared to that of a thrombin before the amino acids except the active center amino acids are substituted.
(37) The thrombin derivative according to any one of (1) to (36), wherein the amino acid sequence of the thrombin B chain is an amino acid sequence of a B chain of human wild-type thrombin.
(38) The thrombin derivative according to (37), wherein the amino acid sequence of the B chain of the human wild-type thrombin is an amino acid sequence from position 50 to position 308 of SEQ ID NO:2.
(39) The thrombin derivative according to any one of (1) to (38), wherein its carboxyl group is modified.
(40) The thrombin derivative according to (39), wherein the carboxyl group is modified by an ester of an amino acid.
(41) The thrombin derivative according to (39), wherein the carboxyl group is modified by polyethylene glycol.
(42) The thrombin derivative according to (39), wherein the carboxyl group is modified by polyethylene glycol having an amino group.
(43) The thrombin derivative according to (41) or (42), wherein the polyethylene glycol is polyethylene glycol having a molecular weight of 1,000 or less.
(44) The thrombin derivative according to (39), wherein the carboxyl group is modified by carbodiimide.
(45) The thrombin derivative according to (39), wherein at least 3 carboxyl groups per molecule are modified.
(46) The thrombin derivative according to (39), wherein 25 or less carboxyl groups per molecule are modified.
(47) The thrombin derivative according to (39), wherein at least a carboxyl group of glutamic acid at position 25 in the B chain is modified.
(48) The thrombin derivative according to any one of (1) to (47), having a PAR1 activation-inhibiting effect and/or a ristocetin-induced platelet aggregation-inhibiting effect.
(49) A DNA encoding the thrombin derivative according to any one of (1) to (38).
(50) A pharmaceutical composition, comprising the thrombin derivative according to any one of (1) to (48).
(51) The pharmaceutical composition according to (50), which is an antithrombotic agent.
(52) The pharmaceutical composition according to (50), which is an anti-inflammatory agent.
(53) The pharmaceutical composition according to (50), which is a platelet aggregation-inhibiting agent.
(54) The pharmaceutical composition according to (50), which is a platelet adhesion-inhibiting agent.
(55) The pharmaceutical composition according to (50), which is an endogenous blood coagulation-inhibiting agent.
(56) The pharmaceutical composition according to (50), which is a thrombin receptor activation-inhibiting agent.
(57) The pharmaceutical composition according to (50), having both an anti-blood coagulation effect and an antiplatelet effect.

### [Brief Description of the Drawings]

[Fig. 1] A drawing showing the antiplatelet effect of carboxyl group-modified 203A205G thrombin (37 µg/ml) on PRP in which the platelet aggregation has been induced by 5 mg/ml ristocetin. "009" and "010" denote a control and the carboxyl group-modified 203A205G thrombin, respectively. The vertical axis represents the transmittance (%) and the horizontal axis represents the time (minute): the same holds true for FIGS. 2 to 12, 15, 21 and 22, and 25 to 35.
[Fig. 2] A drawing showing the antiplatelet effect of carboxyl group-modified 203A205G thrombin (37 µg/ml) on PRP in which the platelet aggregation has been induced by 1 µg/ml M-thrombin (i.e., carboxyl group-modified thrombin). "002" and "001" denote the control and the carboxyl group-modified 203A205G thrombin, respectively.
[Fig. 3] A drawing showing the antiplatelet effect of 205A43A thrombin (30 µg/ml) on PRP in which the platelet aggregation has been induced by 5 mg/ml ristocetin. "079" and "080" denote the control and the 205A43A thrombin, respectively.
[Fig. 4] A drawing showing the antiplatelet effect of 205A43A thrombin (30 µg/ml) on PRP in which the platelet aggregation has been induced by 1 µg/ml M-thrombin. "083" and "084" denote the control and the 205A43A thrombin, respectively.
[Fig. 5] A drawing showing the antiplatelet effect of carboxyl group-modified 205A43A thrombin (30 µg/ml) on PRP in which the platelet aggregation has been induced by 5 mg/ml ristocetin. "056" and "055" denote the control and the carboxyl group-modified 205A43A thrombin, respectively.
[Fig. 6] A drawing showing the antiplatelet effect of carboxyl group-modified 205A43A thrombin (15 µg/ml) on PRP in which the platelet aggregation has been induced by 5 mg/ml ristocetin. "042" and "041" denote the control and the carboxyl group-modified 205A43A thrombin, respectively.
[Fig. 7] A drawing showing the antiplatelet effect of carboxyl group-modified 205A43A thrombin (7.5 µg/ml) on PRP in which the platelet aggregation has been induced by 5 mg/ml ristocetin. "058" and "057" denote the control and the carboxyl group-modified 205A43A thrombin, respectively.
[Fig. 8] A drawing showing the antiplatelet effect of carboxyl group-modified 205A43A thrombin (30 µg/ml) on PRP in which the platelet aggregation has been induced by 1 µg/ml M-thrombin. "064" and "063" denote the control and the carboxyl group-modified 205A43A thrombin, respectively.
[Fig. 9] A drawing showing the antiplatelet effect of carboxyl group-modified 205A43A thrombin (15 µg/ml) on PRP in which the platelet aggregation has been induced by 1 µg/ml M-thrombin. "067" and "066" denote the control and the carboxyl group-modified 205A43A thrombin, respectively.
[Fig. 10] A drawing showing the antiplatelet effect of carboxyl group-modified 205A43A thrombin (7.5 µg/ml) on PRP in which the platelet aggregation has been induced by 1 µg/ml M-thrombin. "070" and "069" denote the control and the carboxyl group-modified 205A43A thrombin, respectively.
[Fig. 11] A drawing showing the antiplatelet effect of the 203A205G thrombin (80 µg/ml) on PRP in which the platelet aggregation has been induced by 5 mg/ml ristocetin. "104" and "095" denote the control and the 203A205G thrombin, respectively.
[Fig. 12] A drawing showing the antiplatelet effect of the 203A205G thrombin (80 µg/ml) on PRP in which the platelet aggregation has been induced by 1 µg/ml M-thrombin. "097" and "098" denote the control and the 203A205G thrombin, respectively.
[Fig. 13] A drawing showing the binding specificity of the 203A205G thrombin to Fbgn and FVIII. The dashed line denotes 1.0 x 10⁻⁷ M Fbgn and the continuous line denotes 1.0 x 10⁻⁷ M FVIII.
[Fig. 14] A drawing showing the binding specificity of AHT to Fbgn and FVIII. The dashed line denotes 1.0 x 10⁻⁷ M Fbgn and the continuous line denotes 1.0 x 10⁻⁷ M FVIII.
[Fig. 15] A drawing showing the antiplatelet effects of the 205A43A thrombin ("007" and "008" denote concentrations of 3.26 µM and 0.81 µM, respectively) on PRP in which the platelet aggregation has been induced by 1 µg/ml M-thrombin. "009" denotes the control. [Fig. 16] A drawing showing the binding specificity of the 205A43A thrombin derivative to Fbgn and FVIII. The dashed line denotes 1.0 x 10⁻⁷ M Fbgn and the continuous line denotes 1.0 x 10⁻⁷ M FVIII.
[Fig. 17] A drawing showing the binding specificity of the 205A43S thrombin derivative to Fbgn and FVIII. The dashed line denotes 1.0 x 10⁻⁷ M Fbgn and the continuous line denotes 1.0 x 10⁻⁷ M FVIII.
[Fig. 18] A drawing showing the binding specificity of the 24E205A43A thrombin to Fbgn and FVIII. The dashed line denotes 1.0 x 10⁻⁷ M Fbgn and the continuous line denotes 1.0 x 10⁻⁷ M FVIII.
[Fig. 19] A drawing showing the TM-binding abilities of 205A43A thrombin and 24E205A43A thrombin. D and E denote the 205A43A thrombin and the 24E205A43A thrombin, respectively.
[Fig. 20] A drawing (photograph) of electrophoresis showing the thrombin substrate-cleaving activity of 203A thrombin. Lane 1 denotes a molecular weight marker, lanes 2 and 5 each denote a blank lane, lane 3 denotes only FXIII, and lane 4 denotes FXIII + the 203A thrombin. In addition, lanes 6 to 10 denote the results obtained by adding the 203A thrombin to FXIII and then allowing to react for 0, 0.5, 1, 3, and 6 hours, respectively.
[Fig. 21] A drawing showing the antiplatelet effect of carboxyl group-modified 205A thrombin (100 µg/ml) on PRP in which the platelet aggregation has been induced by 1 µg/ml M-thrombin. "016" and "015" denote the control and the carboxyl group-modified 205A thrombin, respectively.
[Fig. 22] A drawing showing the antiplatelet effect of carboxyl group-modified 205A thrombin (100 µg/ml) on PRP in which the platelet aggregation has been induced by 5 mg/ml ristocetin. "003" and "004" denote the control and the carboxyl group-modified 205A thrombin, respectively.
[Fig. 23] A drawing showing the binding specificity of the 205A thrombin derivative to Fbgn and FVIII. The dashed line denotes 1.0 x 10⁻⁷ M Fbgn and the continuous line denotes 1.0 x 10⁻⁷ M FVIII.
[Fig. 24] A drawing showing the binding specificity of the 205A203A thrombin derivative to Fbgn and FVIII. The dashed line denotes 1.0 x 10⁻⁷ M Fbgn and the continuous line denotes 1.0 x 10⁻⁷ M FVIII.
[Fig. 25] A drawing showing the antiplatelet effect of carboxyl group-modified 77E203A205G thrombin (50 µg/ml) on PRP in which the platelet aggregation has been induced by 5 mg/ml ristocetin. "001" and "002" denote the control and the carboxyl group-modified 77E203A205G thrombin, respectively.
[Fig. 26] A drawing showing the antiplatelet effect of carboxyl group-modified 77E203A205G thrombin (50 µg/ml) on PRP in which the platelet aggregation has been induced by 1 µg/ml M-thrombin. "014" and "013" denote the control and the carboxyl group-modified 77E203A205G thrombin, respectively.
[Fig. 27] A drawing showing the antiplatelet effect of 77E205A43A thrombin (100 µg/ml) on PRP in which the platelet aggregation has been induced by 1 µg/ml M-thrombin. "019" and "020" denote the control and the 77E205A43A thrombin, respectively.
[Fig. 28] A drawing showing the antiplatelet effect of carboxyl group-modified 77E205A43A thrombin (30 µg/ml) on PRP in which the platelet aggregation has been induced by 5 mg/ml ristocetin. "005" and "006" denote the control and the carboxyl group-modified 77E205A43A thrombin, respectively.
[Fig. 29] A drawing showing the antiplatelet effect of 65A205A43A thrombin (100 µg/ml) on PRP in which the platelet aggregation has been induced by 1 µg/ml M-thrombin. "020" and "019" denote the control and the 65A205A43A thrombin, respectively.
[Fig. 30] A drawing showing the antiplatelet effects of 65A205A43A thrombin (25 or 50 µg/ml) on PRP in which the platelet aggregation has been induced by 1 µg/ml M-thrombin. "021" and "022" denote the 65A205A43A thrombin at concentrations of 25 µg/ml and 50 µg/ml, respectively.
[Fig. 31] A drawing showing the antiplatelet effect of 65A205A43A thrombin (10 µg/ml) on PRP in which the platelet aggregation has been induced by 1 µg/ml M-thrombin. "023" and "024" denote the control and the 65A205A43A thrombin, respectively.
[Fig. 32] A drawing showing the antiplatelet effect of carboxyl group-modified 65A205A43A thrombin (36 µg/ml) on PRP in which the platelet aggregation has been induced by 1 µg/ml M-thrombin. "038" and "033" denote the control and the carboxyl group-modified 65A205A43A thrombin, respectively.
[Fig. 33] A drawing showing the antiplatelet effects of carboxyl group-modified 65A205A43A thrombin (9 or 18 µg/ml) on PRP in which the platelet aggregation has been induced by 1 µg/ml M-thrombin. "035" and "036" denote 18 µg/ml and 9 µg/ml, respectively.
[Fig. 34] A drawing showing the antiplatelet effect of carboxyl group-modified 65A205A43A thrombin (4.5 µg/ml) on PRP in which the platelet aggregation has been induced by 1 µg/ml M-thrombin.
[Fig. 35] A drawing showing the antiplatelet effect of carboxyl group-modified 65A205A43A thrombin (36 µg/ml) on PRP in which the platelet aggregation has been induced by 5 mg/ml ristocetin. "041" and "042" denote the control and the carboxyl group-modified 77E205A43A thrombin, respectively.
[Fig. 36] A drawing showing the binding specificity of the 65A205A43A thrombin derivative to Fbgn and FVIII. The dashed line denotes 1.0 x 10⁻⁷ M Fbgn and the continuous line denotes 1.0 x 10⁻⁷ M FVIII.
[Fig. 37] A drawing showing the TM-binding ability of 65A205A43A thrombin.
[Fig. 38]Adawing (photograph) showing an electrophoresis pattern of 205A43A thrombin in a purification process by using a hirudin C-terminal peptide column. SDS-PAGE was performed without reduction and then CBB staining was performed. Lanes 1 and 2 each denote a standard thrombin, lane 3 denotes a fraction before application to the column, lane 4 denotes a flow-flow-through fraction, and lanes 5 to 10 each denote an eluted fraction.
[Fig. 39] A drawing (photograph) showing an electrophoresis pattern of 205A thrombin in the purification process by using a hirudin C-terminal peptide column. SDS-PAGE was performed without reduction and then CBB staining was performed. Lanes 1 to 3 each denote a standard thrombin, lane 4 denotes a fraction before application to the column, lane 5 denotes a flow-flow-through fraction, and lanes 6 to 11 each denote an eluted fraction.
[Fig. 40] A drawing (photograph) of electrophoresis showing the thrombin substrate-cleaving activity of 205A thrombin. Lane 1 denotes a marker, lanes 2 and 5 each denote a lane without a sample, lane 3 denotes only FXIII, and lane 4 denotes FXIII + thrombin. Lanes 6 to 10 denote FXIII which was reacted with the 195A thrombin for 0, 1, 3, 6, and 24 hours, respectively.
[Fig. 41] A drawing (photograph) of electrophoresis showing the thrombin substrate-cleaving activity of 203A205G thrombin. Lane 1 denotes a marker, lane 2 denotes only FXIII, and lane 3 denotes FXIII + thrombin. Lanes 4 to 8 denote FXIII which was reacted with the 203A205G thrombin for 0, 1, 3, 6, and 24 hours, respectively.
[Fig. 42] A drawing (photograph) of electrophoresis showing the thrombin substrate-cleaving activities of respective derivatives. Lane 1 denotes a marker, lane 3 denotes only FXIII, and lanes 2 and 4 each denote FXIII + thrombin. Lanes 5 to 7 denote FXIII which was reacted with the 205G thrombin for 0, 1, and 3 hours, respectively. Lanes 8 and 9 denote FXIII which was reacted with the 205A43A thrombin for 0 and 3 hours, respectively. Lanes 10 and 11 denote FXIII which was reacted with the 203A205G thrombin for 0 and 3 hours, respectively.
[Fig. 43] A drawing (photograph) of electrophoresis showing the thrombin substrate-cleaving activities of respective derivatives. Lane 1 denotes a marker, lane 2 denotes only FXIII, and lane 3 denotes FXIII + thrombin. Lanes 4 to 8 denote FXIII which was reacted with the 203A205A thrombin for 0, 1, 3, 6, and 24 hours, respectively. Lanes 9 to 13 denote FXIII which was reacted with the 203A205A99N thrombin for 0, 1, 3, 6, and 24 hours, respectively.
[Fig. 44] A drawing (photograph) showing an electrophoresis pattern of 205V thrombin in the purification process by using a hirudin C-terminal peptide column. SDS-PAGE was performed after S-S bonds were reduced and Western blotting was performed by using an anti-human thrombin polyclonal antibody to confirm bands. Lane 1 denotes a marker, lanes 2 and 3 each denote standard thrombin, lane 4 denote a fraction before application to the column, lane 5 denotes a flow-flow-through fraction, and lane 6 denotes an eluted fraction.
[Fig. 45] A drawing (photograph) showing an electrophoresis pattern of 205D thrombin in the purification process by using a hirudin C-terminal peptide column. SDS-PAGE was performed after S-S bonds were reduced and Western blotting was performed by using an anti-human thrombin polyclonal antibody to confirm bands. Lane 1 denotes a marker, lanes 2 and 3 each denote standard thrombin, lane 4 denote a fraction before application to the column, lane 5 denotes a flow-flow-through fraction, and lane 6 denotes an eluted fraction.
[Fig. 46] A drawing showing the effect of an anti-PAR1 antibody on the platelet aggregation effect of 1 µg/ml M-thrombin. "002" and "001" denote 1 µg/ml M-thrombin and 1 µg/ml M-thrombin + anti-PAR1 antibody, respectively.
[Fig. 47] A drawing showing the binding specificity of the 19A205A43A thrombin derivative to Fbgn and FVIII. The dashed line denotes 1.0 x 10⁻⁷ Fbgn and the continuous line denotes 1.0 x 10⁻⁷ VIII.
[Fig. 48] A schematic drawing showing the growth of an arterial thrombus.

### [DECRRIPTION OF THE PREFERRED EMBODIMENTS]

Hereinafter, the present invention will be described in detail.
1. <Thrombin>
A thrombin derivative to be used in the present invention is a thrombin derivative which comprises an A chain and a B chain, and in which a particular amino acid in the B chain is substituted. The thrombin derivative to be used in the present invention is not particularly limited as long as it can form a tertiary structure in which the A chain and the B chain are cross-linked to each other through an S-S bond in a living body. The A chain and the B chain are each produced by processing of a thrombin precursor protein, so the thrombin derivative of the present invention may be administered to a living body in a form of a precursor protein such as prethrombin or prothrombin so as to be processed in the living body to form the tertiary structure. Meanwhile, the A chain and the B chain may be separately produced by gene recombination, chemical synthesis, or the like, and then the S-S bonds between them were allowed to be formed in vitro, or they may be separately administered so as to form the tertiary structure, in which the A chain and the B chain are cross-linked to each other through the S-S bonds, in a living body.
Here, the A chain is a region corresponding to amino acid numbers 1 to 49 in SEQ ID NO:2 in the case of human wild-type thrombin. The B chain is a region corresponding to amino acid numbers 50 to 308 in SEQ ID NO:2 in the case of human wild-type thrombin.
In human thrombin, 13 amino acid residues at an N-terminal of the A chain are cleaved out therefrom by autolysis. Therefore, the A chain may be a sequence in which the 13 amino acid residues (e.g., amino acid numbers 1 to 13 in SEQ ID NO:2) at the N terminal of the A chain have been cleaved out. Further, thrombin precursor proteins such as prothrombin and prethrombin each of which can form the tertiary structure as described above in a living body are also encompassed in the thrombin derivative of the present invention. The amino acid sequence of human wild-type prothrombin is disclosed in the database of Swissprot with an accession number of P00734.
The thrombin derivatives to be obtained in the present invention can be administered into a living body in a form of prothrombin. In this case, the thrombin derivative is activated to form thrombin having an antithrombotic effect at a thrombotic site, and an antithrombotic effect is exerted at the area where a thrombus is formed, thereby attaining a more site-directed antithrombotic effect in a living body.

### 2. <Thrombin substrate-cleaving activity>

When the thrombin derivative of the present invention is reacted with the blood coagulation factor 13 (FXIII), fibrinogen (Fbgn), a synthetic substrate (available from SIGMA) such as S2238, a thrombin receptor, or a thrombin substrate such as protein C, in 50 mM Tris-HCl (pH 7.4) containing 0.1 M NaCl at 37°C for 3 hours, the percentage of a cleaved thrombin substrate is 10% or less. As long as the thrombin substrate-cleaving activity is within this range, the activation of the thrombin substrate by the thrombin derivative from after the administration into a living body to the metabolism of the thrombin derivative does not cause any problems. Of the thrombin substrates, FXIII is relatively stable to heat and can be easily observed by electrophoresis, so FXIII is preferably used in the present invention. The cleavage is preferably determined at a concentration of the thrombin derivative with respect to FXIII of 1:1 or more in terms of a molar ratio.
The presence or absence of the substrate-cleaving abilities of thrombin can be confirmed by observing a band of the activated substrate in electrophoresis for FXIII, and the presence or absence of fibrinogen can be confirmed by observing the presence or absence of a small amount of fibrin clots. According to the method of assaying the thrombin substrate-cleaving activity, a derivative which is obtained by substituting alanine for the glycine at position 203 in the B chain and which is reported to have completely lost its activity in Non-patent Document 6 (Biochimica et Biophyscia Acta, 1451 (1999), 173-186) is found to still have the thrombin substrate-cleaving activity left therein.

The thrombin derivatives to be obtained in the present invention have an antithrombotic mechanism different from those of thrombin derivatives disclosed in Patent Document 3 (JP-A-09-509045), Non-patent Document 7 (J. Biol. Chem Vol. 275, 39827-39830), Non-patent Document 8 (J. Biol. Chem Vol. 279, 26387-26394), and Non-patent Document 9 (J. Biol. Chem Vol. 277, 27581-27584). In other words, the thrombin derivatives disclosed in the documents have a maintained or improved TM specificity and thus preferentially activate protein C to thereby exert an antithrombotic effect. On the other hand, the thrombin derivative of the present invention has no ability of activating the thrombin substrate including protein C. The thrombin derivative of the present invention binds more specifically to a thrombin substrate which is important in thrombogenesis, without activating the thrombin substrate, and inhibits the activation of those thrombin substrates caused by thrombin which exists in the blood, thereby exerting an antithrombotic effect. However, it is desirable that the thrombin derivative of the present invention does not inhibit the TM-binding of thrombin which exists in a living body while having a decreased TM-binding ability.

### 3. <Substitution of amino acid and modification of carboxyl group>

A first aspect of the present invention is a thrombin derivative in which one or more amino acids (active center amino acids) selected from the group consisting of serine at position 205, glycine at position 203, aspartic acid at position 99, and histidine at position 43 in a thrombin B chain are substituted, in which (1) the ratio of a thrombin substrate which is cleaved by the thrombin derivative when the thrombin substrate is reacted in 50 mM Tris-HCl (pH 7.4) containing 0.1 M NaCl at 37°C for 3 hours is 10% or less, and (2) a structure of an exosite I is maintained. The first aspect of the present invention takes the constitutions, to thereby exert an antithrombotic effect mainly composed of an effect of prolonging activated partial thromboplastin time (APTT) equal to or larger than that of anhydrothrombin (AHT). The APTT-prolonging effect surely prolongs the APTT without being affected by incubation.

A second aspect of the present invention is a thrombin derivative in which amino acids except the active center amino acids are further substituted. The thrombin derivative is a derivative having an improved antithrombotic ability by decreasing the binding ability to Fbgn which exists in blood in a large amount. The decrease in the binding ability to Fbgn enables efficient binding to a target thrombin substrate which promotes blood coagulation, thereby inhibiting the activation of the thrombin substrate.
Examples of the thrombin substrate which promotes the blood coagulation include FV, FVIII, FXI, a thrombin receptor (e.g., PAR1), and PAR4. It is more preferable to specifically inhibit the thrombin receptor or FVIII in order to improve the antithrombotic ability more efficiently. That is, a preferable example of the thrombin derivative of the present invention is one which has an Fbgn-binding ability relatively lower than the binding ability to a thrombin receptor or FVIII, and thus can provide a good antithrombotic effect even with a decreased dosage. In addition, the additional substitution of the amino acid except the active center amino acids specifically decreases the binding ability to TM without eliminating the antithrombotic effect held by the thrombin derivative of the first aspect of the present invention.
Further, according to the second aspect of the present invention, there can be obtained thrombin derivatives having various aspects of the expression of the effects, such as a thrombin derivative which has a high APTT-prolonging effect but with a low antiplatelet effect and a thrombin derivative which has a high APTT-prolonging effect and a high antiplatelet effect (only a PAR1-inhibiting effect).
Examples of such amino acid except the active center amino acids include: amino acids in the exosite I region such as glutamine at position 24, lysine at position 65, and lysine at position 77 in the B chain; amino acids in the exosite II region such as arginine at position 98, arginine at position 245, lysine at position 248, and lysine at position 252 in the B chain; and amino acids in a region which does not belong to either exosites such as arginine at position 197 in the B chain and phenylalanine at position 19 in the B chain.

A third aspect of the present invention is a thrombin derivative obtained by modifying carboxyl groups of the thrombin derivative according to the first or second aspect of the present invention by a method described in Patent Document 2 (WO 02/077031). The thrombin derivative not only prolongs the APTT but imparts or controls the antiplatelet effect, inhibiting effect of particularly the PAR1 activation and ristocetin-induced platelet aggregation depending on the substitution of the amino acids. To be specific, there can be obtained: a thrombin derivative having an extremely strong APTT-prolonging effect and a strong antiplatelet effect; a thrombin derivative having a moderate level of an APTT-prolonging effect and a strong antiplatelet effect; and the like. That is, according to the third aspect of the present invention, a thrombin derivative having an anti-blood coagulation effect and an antiplatelet effect which can be applied to various thromboses can be obtained.
Hereinafter, for facilitating understanding of the effects and implications of the amino acid substitution, the present invention will be described by dividing it into parts: the substitution of the active center amino acids (the first aspect of the present invention); the substitution of amino acids except the active center amino acids (the second aspect of the present invention); and the modification of carboxyl groups (the third aspect of the present invention).

It should be noted that the thrombin derivative of the present invention may be a thrombin derivative having an amino acid sequence in which one or several amino acids are substituted, deleted, inserted, or added in the B chain (amino acid numbers 50 to 308 in SEQ ID NO:2) in addition to the particular amino acid as described above, as long as the effect of the present invention is not impaired. Here, the term "several" means 2 to 20, preferably 2 to 10, and more preferably 2 to 5. The thrombin derivative of the present invention may be a thrombin derivative having an amino acid sequence in which one or several amino acids are substituted, deleted, inserted, or added also in the A chain (amino acid numbers 1 to 49 in SEQ ID NO:2). Here, the term "several" means 2 to 10, preferably 2 to 5, and more preferably 2 to 3.
Furthermore, the thrombin derivative of the present invention may be one having additional modification such as substitution, deletion, insertion, or addition at a position other than the amino acid numbers 1 to 49 in SEQ ID NO:2 of the A chain and the amino acid numbers 50 to 308 in SEQ ID NO:2 of the B chain while each of the chains has a homology of a certain level or higher, as long as the ratio of a thrombin substrate which is cleaved by the thrombin derivative when the thrombin substrate is reacted in 50 mM Tris-HCl (pH 7.4) containing 0.1 M NaCl at 37°C for 3 hours is 10% or less and a structure of an exosite I is maintained. Here, the term "homology of a certain level or higher" refers to a homology of 80% or higher, preferably 90% or higher, and particularly preferably 95% or higher.

The thrombin derivative of the present invention is obtained by creating a DNA encoding each derivative by means of site-directed mutagenesis, incorporating the DNA into a vector or the like, and expressing the DNA in a mammalian cell or the like. Such a DNA may encode both the A chain and the B chain as described above, or each of the chains may be separately expressed. The method for site-directed mutagenesis is not particularly limited, but may be performed by using Quick Change Site-Directed Mutagenesis Kit (manufactured by Stratagene) or the like. The thrombin derivative can also be obtained by chemical synthesis.

### 3-1. <Substitution of active center amino acids; hereinafter, also referred to as the first aspect of the present invention>

The thrombin derivative of the present invention is a thrombin derivative in which one or more amino acids (active center amino acids) selected from the group consisting of serine at position 205 (position 205 in B chain; serine corresponding to serine at position 254 in SEQ ID NO:2), glycine at position 203 (position 203 in B chain; glycine corresponding to glycine at position 252 in SEQ ID NO:2), histidine at position 43 (position 43 in B chain; histidine corresponding to histidine at position 92 in SEQ ID NO:2), and aspartic acid at position 99 (position 99 in B chain; aspartic acid corresponding to aspartic acid at position 148 in SEQ ID NO:2) in the thrombin B chain.

Herein, the terms "position 205 in B chain", "position 203 in B chain", and the like each refer to the number of an amino acid counted from the first amino acid in the B chain, for example, isoleucine at amino acid number 50 in SEQ ID NO:2. The position of the substituted amino acid as described above may be shifted to back and forth depending on the deletion, insertion, addition, or the like of the amino acid. For instance, when an amino acid residue is inserted to the N terminal, the serine residue which originally exists at position 205 becomes the 206th residue. In the present invention, such serine residue corresponding to the serine residue at position 205 is also called a serine residue at position 205.

In order that the thrombin derivative of the present invention expresses the effect of the present invention, the structure of the exosite I of thrombin before the substitution has to be maintained even after the substitution of the active center amino acids is performed.
The exosite I of thrombin is reported as an essential region which plays the most important role in the binding to many of thrombin substrates each having a thrombogenesis effect, inflammatory effect, or the like (Journal of Biological Chemistry, 1989, Vol. 264, 8692-8698). A hirudin C-terminal peptide is known to specifically bind to the exosite I (Journal of Biological Chemistry, 1991, Vol. 266, 23633-23636). In particular, a thrombin receptor which plays an important role in the thrombogenesis through the activation of platelets is reported to have a high homology with the hirudin C-terminal region and strongly bind to the exosite I. Therefore, it is thought that a thrombin derivative having lost the binding ability to the hirudin C-terminal peptide has the structure of the exosite I destructed, and thus the binding ability to many of the thrombin substrates including the thrombin receptor has been lost. Accordingly, in the present invention, judgement on whether the binding ability to the hirudin C-terminal peptide (e.g., SEQ ID NO:3) is maintained or not is used for a first step of screening for judging whether the structure of the exosite I is maintained or not.

The judgement on whether the binding ability to the hirudin C-terminal peptide is maintained or not can be made by determining the binding ratio of a thrombin derivative to hirudin C-terminal peptide-immobilized gel as described in the examples as described below.
The thrombin derivative of the present invention preferably maintains the binding ability to the hirudin C-terminal peptide. A preferable example of the thrombin derivative of the present invention is a thrombin derivative 10% or more of which, preferably 50% or more of which added in the reaction system binds to the hirudin C-terminal peptide-immobilized gel when it is allowed to react with the hirudin C-terminal peptide-immobilized gel.

It should be noted that the determination of the binding ratio of the thrombin derivative to the hirudin C-terminal peptide-immobilized gel is performed only for confirming whether the structure of the exosite I is maintained or not before and after the substitution of the active center amino acid. Therefore, in the second aspect of the present invention, when the substitution of the amino acid except the active center amino acids is further performed, the FVIII-binding ability may be maintained and the binding ability to the hirudin C-terminal peptide-immobilized gel may be lost or significantly decreased owing to the further amino acid substitution, even when the structure of the exosite I is maintained.

Furthermore, the thrombin derivative of the present invention is preferably one which does not lose the heparin-binding ability owing to the substitution of the active center amino acids. A thrombin derivative which has maintained 80% or more of the heparin-binding ability with respect to the heparin-binding ability of thrombin before the substitution of the active center amino acids is more preferable. The active center exists in a region distant from the heparin-binding site or exosite II in the tertiary structure of thrombin. In other words, the fact that the heparin-binding ability is eliminated by the substitution of the active center amino acids means that the substitution of the active center amino acids results in a structural abnormality over the entire thrombin molecule to thereby destruct the structure of the heparin-binding region which exists at a position distant from the active center. The structural abnormality over the entire molecule means a decrease in the binding ability to a thrombin substrate.

In the present invention, the substitutions of the active center amino acids are preferably substitutions of 2 or more amino acids selected from the group consisting of serine at position 205, glycine at position 203, aspartic acid at position 99, and histidine at position 43 in the thrombin B chain. The substitutions of the active center amino acids more preferably include substitution of at least the serine at position 205, and particularly preferably include substitutions of at least the serine at position 205 and the histidine at position 43.

The glycine at position 203 in the B chain is preferably substituted by any one of alanine, serine, and threonine. The aspartic acid at position 99 is preferably substituted by asparagine. The histidine at position 43 is preferably substituted by alanine, serine or asparagine.

The serine at position 205 in the B chain is preferably substituted by alanine, threonine, or glycine. Of those, the substitution by alanine is particularly preferable because the structure of the exosite I is hardly impaired.

In a case where the substitutions of the active center amino acids are the substitutions of the serine at position 205 and the histidine at position 43, a derivative in which the serine at position 205 is substituted by alanine, glycine, or threonine and the histidine at position 43 is substituted by alanine or serine is preferable, and a derivative in which both the serine at position 205 and the histidine at position 43 are substituted by alanine is particularly preferable.

Hereinafter, the substitutions of the active center amino acids according to the first aspect of the present invention will be described by referring to specific examples. When the substitutions of the active center amino acids are the substitutions of 2 or more amino acids selected from the group consisting of serine at position 205, glycine at position 203, aspartic acid at position 99, and histidine at position 43 in the thrombin B chain, the combination of the 2 or more amino acids to be substituted (substitution sites) and the kind of the amino acid serving as a counterpart of substitution may provide differences in the thrombin substrate-binding ability and the antithrombotic effect. For explanation, description will be given by taking the following thrombin derivatives 1 to 7 as examples.

**[Table 1]**

| | Substitution site (active center amino acid) | | | |
|---|---|---|---|---|
| | 205Ser | 203Gly | 99Asp | 43His |
| Thrombin derivative 1 | Ala | - | Asn | - |
| Thrombin derivative 2 | Ala | - | - | Ala |
| Thrombin derivative 3 | Gly | Ala | - | - |
| Thrombin derivative 4 | Ala | Ala | - | - |
| Thrombin derivative 5 | Ala | Ala | Asn | - |
| Thrombin derivative 6 | Gly | - | - | Ala |
| Thrombin derivative 7 | Ala | - | - | Ser |

Thrombin derivative 1: a thrombin derivative (sequence of amino acid numbers 44 to 351 in SEQ ID NO:22) which has substitution sites of the serine at position 205 and the aspartic acid at position 99 in the B chain, in which the serine is substituted by alanine and the aspartic acid is substituted by asparagine.
Thrombin derivative 2: a thrombin derivative (sequence of amino acid numbers 44 to 351 in SEQ ID NO:26) which has substitution sites of the serine at position 205 and the histidine at position 43 in the B chain, in which the serine is substituted by alanine and the histidine is substituted by alanine.
Thrombin derivative 3: a thrombin derivative (sequence of amino acid numbers 44 to 351 in SEQ ID NO:8) which has substitution sites of the serine at position 205 and the glycine at position 203 in the B chain, in which the serine is substituted by glycine and the glycine is substituted by alanine.
Thrombin derivative 4: a thrombin derivative (sequence of amino acid numbers 44 to 351 in SEQ ID NO:24) which has substitution sites of the serine at position 205 and the glycine at position 203 in the B chain, in which the serine and the glycine are each substituted by alanine.
Thrombin derivative 5: a thrombin derivative (sequence of amino acid numbers 44 to 351 in SEQ ID NO:14) which has substitution sites of the serine at position 205, the glycine at position 203, and the aspartic acid at position 99 in the B chain, in which the serine and the glycine are each substituted by alanine and the aspartic acid is substituted by asparagine.
Thrombin derivative 6: a thrombin derivative (sequence of amino acid numbers 44 to 351 in SEQ ID NO:34) which has substitution sites of the serine at position 205 and the histidine at position 43 in the B chain, in which the serine is substituted by glycine and the histidine is substituted by alanine.
Thrombin derivative 7: a thrombin derivative (sequence of amino acid numbers 44 to 351 in SEQ ID NO:36) which has substitution sites of the serine at position 205 and the histidine at position 43 in the B chain, in which the serine is substituted by alanine and the histidine is substituted by serine.

Each of the thrombin derivatives 1 to 7 has maintained binding ability to the hirudin C-terminal peptide-immobilized gel. However, the thrombin derivatives 4 and 5 each had a decreased binding ability to heparin gel as compared to that of human wild-type thrombin.

Each of the thrombin derivatives 1 to 7 has an antithrombotic effect mainly including an APTT-prolonging effect, and the order of their effects was as described below.
Thrombin derivative 2 > Thrombin derivatives 6 and 7 > Thrombin derivatives 1 and 3 >> Thrombin derivatives 4 and 5

The thrombin derivatives 2, 6, and 7 each showed an extremely high antithrombotic effect regardless of the presence or absence of the modification of a carboxyl group described below. In addition, the antithrombotic effect observed in the thrombin derivative 2 specifically acted on the coagulation cascade, and thus particularly prolonged APTT well. Further, a thrombin derivative obtained by modifying the carboxyl group of the thrombin derivative 2 had imparted or enhanced antiplatelet ability, and thus had effects of inhibiting the coagulation cascade, the platelet aggregation, and the platelet adhesion.

The thrombin derivatives 2 and 4 each had the serine at position 205 substituted by alanine, but they had a significant difference in their antithrombotic effects as described above. Similarly, the thrombin derivatives 3 and 4 each had the glycine at position 203 substituted by alanine and had a difference in their antithrombotic effects as described above.

The thrombin derivatives 1 and 3 each do not exhibit such a strong antithrombotic effect. However, modifications of their carboxyl groups as described below cause a change in their substrate specificity, or cause substitution of an amino acid except the active center amino acids, such as the lysine at position 77 in the B chain, to specifically decrease the binding ability to Fbgn and relatively improve the specificity to FVIII, to thereby cause the thrombin derivative to exhibit one of or both of a strong APTT-prolonging effect and a strong platelet aggregation-inhibiting effect. To be specific, the APTT-prolonging and antiplatelet effects (i.e., aggregation and adhesion) are obtained in a case of the modification of carboxyl groups, and a thrombin derivative having only the APTT-prolonging effect is obtained in a case of the substitution of the amino acid at position 77 in the B chain.

Meanwhile, the thrombin derivatives 4 and 5 each had a weak antithrombotic effect even in a case where the modification of carboxyl groups was performed or in a case where the substitution of the amino acid at position 77 in the B chain was performed. The thrombin derivatives 6 and 7 each exhibited an antithrombotic effect similar to that of the thrombin derivative 2.

Both in the present invention and in the known arts, thrombin derivatives each obtained by substituting any of the serine at position 205, the glycine at position 203, the aspartic acid at position 99, and the histidine at position 43 in the thrombin B chain are classified into the following groups:
A. a thrombin derivative having an antithrombotic effect mainly including a strong APTT-prolonging effect like a thrombin derivative obtained by substituting the serine at position 205 and the histidine at position 43, even without modification of carboxyl groups and/or without substitution of an amino acid except the active center amino acids for decreasing the Fbgn-binding ability;
B. a thrombin derivative which does not exhibit such a strong antithrombotic effect like the thrombin derivative obtained by substituting one selected from the group consisting of the glycine at position 203 and the aspartic acid at position 99 and the serine at position 205, but exhibits a high antithrombotic effect by the carboxyl group modification and/or the substitution of the amino acid except the active center amino acids for decreasing the Fbgn-binding ability;
C. a thrombin derivative which has lost the thrombin substrate-cleaving activity but does not exhibit a high antithrombotic effect even by the carboxyl group modification and/or the substitution of the amino acid for decreasing the Fbgn-binding ability; and
D. a thrombin derivative which exhibits the antithrombotic effect but cannot be used as an antithrombotic agent because of the remaining activity.

The A group was confirmed for the binding abilities to Fbgn and FVIII, and it was found that every member of the group had an improved FVIII-binding ability. Therefore, it is considered that the high APTT-prolonging effect of the group is resulted from the loss of the thrombin substrate-cleaving activity caused by a combination of the substitution of the active center amino acids, and at the same time, the relative and specific increase in the FVIII-binding ability (relative decrease in Fbgn-binding ability). Those thrombin derivatives have also been confirmed to have a weaker antiplatelet effect due to the decrease in the Fbgn-binding ability even without modification of the carboxyl groups. Further, the effect is the PAR1 activation-inhibiting effect without accompanying the ristocetin-induced platelet aggregation-inhibiting effect.

The degree of the relative and specific improvement of the FVIII-binding ability caused by the substitution of the active center amino acids is determined depending on the combination of the specific substitution as described above. Basically, the antithrombotic ability of the thrombin derivative of the present invention can be improved by decreasing the Fbgn-binding ability while maintaining the binding ability to a thrombin substrate which promotes the blood coagulation reaction. In addition, when VIIIA/FA, a ratio between the FVIII-binding ability (VIIIA) and the Fbgn-binding ability (FA) of the obtained thrombin derivative, is 1.1 folds or larger than VIIIa/Fa, a ratio between the FVIII-binding ability (VIIIa) and the Fbgn-binding ability (Fa) of AHT, a high antithrombotic effect is obtained, and when the ratio is 1.2 folds or larger, an extremely high antithrombotic effect is obtained.
The thrombin derivative having the active center amino acids substituted has an FVIII-binding ability maintained by preferably 10% or more, or more preferably 80% or more with respect to anhydrothrombin.
A preferable example of the thrombin derivative having the active center amino acids substituted includes a derivative having active partial thromboplastin time (APTT) 1.1 folds or more of that of anhydrothrombin (AHT).
Further, a combination of the substitution of the active center amino acids causes a difference in the binding ability to FVIII itself as well as the relative and specific changes in the Fbgn-binding ability, the FVIII-binding ability, and the like. The thrombin derivatives 2, 6, and 7 each exhibited a high FVIII-binding ability equivalent to that of AHT. On the other hand, for each of other thrombin derivatives, the FVIII-binding ability is itself decreased. That is, the group A has a high binding ability to FVIII and has an increased ratio of the FVIII-binding ability to Fbgn, and these two effects in combination provide a high antithrombotic effect.
A more preferable example of the thrombin derivative of the present invention has an Fbgn-binding ability decreased by 10% or more due to the amino acid substitutions of the active center amino acids to thus have an APTT-prolonging effect increased to be 1.1 folds or more.

In the tertiary structure, the active center site of thrombin is distant from the exosite I and exosite II which are binding sites of a hirudin C-terminal peptide and heparin, respectively. In consideration of such tertiary structure of thrombin, it is considered that the thrombin derivative having a decreased heparin-binding ability due to the combination of the substitutions of the active center amino acids, and the thrombin derivative having a decreased binding ability to hirudin C-terminal peptide-immobilized gel due to the substitutions of the active center amino acids exemplified above each suffer from folding abnormality and structural abnormality over the entire molecule, which are caused by unreasonable substitution (i.e., substitution which may provide burden on the structure) of the active center amino acid. Further, abnormalities may occur in the exosite I structure or the exosite II structure. Accordingly, it is considered that a decrease in the thrombin substrate-binding ability leads to a decrease in the antithrombotic effect.

### 3-2. <Substitution of amino acids except active center amino acids; hereinafter, also referred to as "second aspect of the present invention">

In order to improve the antithrombotic effect of the first aspect of the present invention, a thrombin derivative obtained by additionally substituting an amino acid except the active center amino acids, in particular, an amino acid which exists in an exosite I region and/or an exosite II region, will be described hereinbelow. The substitution of the amino acid except the active center amino acids may decrease the binding ability to hirudin C-terminal peptide-immobilized gel or heparin. However, the decrease is a result of direct substitution of the amino acid in those regions, and therefore, it is different from the decrease in the binding ability according to the structural abnormality over the entire protein caused by the substitution of the active center amino acids.

The thrombin derivative according to the second aspect of the present invention is a derivative having an additionally improved antithrombotic effect obtained by decreasing the binding ability to Fbgn which exists in blood in a large amount.
The decrease in the Fbgn-binding ability allows the thrombin derivative to efficiently bind to a target thrombin substrate which promotes blood coagulation, to thereby inhibit activation of the thrombin substrate.
Examples of the thrombin substrate which promotes blood coagulation include FV, FVIII, FXI, a thrombin receptor (PAR1), and PAR4. It is more preferable to specifically inhibit the thrombin receptor or FVIII in order to improve the antithrombotic ability more efficiently.
That is, the thrombin derivative of the present invention is a thrombin derivative which has a Fbgn-binding ability relatively lower than the binding ability to a thrombin receptor or FVIII, and which can provide a good antithrombotic effect even with a decreased dosage. In addition, according to the second aspect of the present invention, the binding ability to TM can be specifically decreased without significantly impairing the antithrombotic effect held by the thrombin derivative according to the first aspect of the present invention. Thus, in the present invention, by selectively decreasing the TM-binding ability, the thrombin derivative of the present invention does not bind to TM and thus does not inhibit the protein C activation by thrombin even when the thrombin derivative is administered into a living body.
Since TM is not a substrate, it does not interact with the active center of thrombin. Therefore, it is difficult to specifically decrease the TM-binding ability by the first aspect of the present invention. Meanwhile, the antithrombotic ability enhanced by the first aspect of the present invention is further applied to the second aspect of the present invention, thereby applying a characteristic such as an increased antithrombotic effect or substrate specificity.

The thrombin derivative according to the second aspect of the present invention preferably has a decreased Fbgn-binding ability. Further, the thrombin derivative preferably has a relatively low Fbgn-binding ability as compared to the binding ability to the thrombin receptor or FVIII. To be specific, VIIIA/FA, a ratio between the FVIII-binding ability (VIIIA) and the Fbgn-binding ability (FA), is preferably 1.1 folds or more, or more preferably 1.5 folds or more of VIIIa/Fa, a ratio between the FVIII-binding ability (VIIIa) and the Fbgn-binding ability (Fa) of the thrombin derivative before the substitution of the amino acid except the active center amino acids.

Further, the thrombin derivative according to the second aspect of the present invention preferably has a specifically decreased binding ability to TM. To be specific, VIIIA/TMA, a ratio between the FVIII-binding ability (VIIIA) and the TM-binding ability (TMA), is preferably 1.1 folds or more, or more preferably 1.5 folds or more ofVIIIa/TMa, a ratio between the FVIII-binding ability (VIIIa) and the TM-binding ability (TMa) of the thrombin derivative before the substitution of the amino acid except the active center amino acids.

Furthermore, according to the second aspect of the present invention, there can be obtained thrombin derivatives having various effects, such as a thrombin derivative which has a high APTT-prolonging effect but with a low antiplatelet effect, and a thrombin derivative which has a high APTT-prolonging effect and a high antiplatelet effect (only PAR1-inhibiting effect).

The amino acid substitution outside the active center is not particularly limited as long as the effects described above can be obtained. Specific examples of the substitution include substitution of an amino acid in the exosite I or exosite II region, and substitution in other region in a thrombin derivative, which interacts with fibrinogen or TM.
The exosite I region of thrombin is known as an amino acid region which is rich in basicity and which specifically interacts with the C-terminal region of hirudin, and has been known as an important region which interacts with Fbgn, a thrombin receptor, TM, and the like (E. Di Cera, Thrombin Interactions Chest, 2003; 124 (90030): 11-17). In the present invention, one or more selected from the basic amino acids is preferably substituted. It is more preferable that one or more selected from the group consisting of glutamine at position 24 (i.e., B chain 24; corresponding to the glutamine at position 73 in SEQ ID NO:2), lysine at position 65, and lysine at position 77 (i.e., B chain 77; corresponding to the lysine at position 126 in SEQ ID NO:2) in the B chain be substituted. The amino acid to be substituted for the lysine at position 77, the glutamine at position 24, or the lysine at position 65 is not particularly limited, but alanine, serine, threonine, glutamic acid, or glutamine is preferable.

The exosite II region is known as an amino acid region which is rich in basicity and which mainly interacts with heparin (E. Di Cera, Thrombin Interactions Chest, 2003; 124 (90030): 11-17). In the present invention, one or more selected from the basic amino acids is preferably substituted. It is more preferable that one or more selected from the group consisting of arginine at position 98 (i.e., arginine corresponding to the arginine at position 147 in SEQ ID NO:2), arginine at position 245 (i.e., arginine corresponding to the arginine at position 294 in SEQ ID NO:2), lysine at position 248 (i.e., lysine corresponding to the lysine at position 297 in SEQ ID NO:2), and lysine at position 252 (i.e., lysine corresponding to the lysine at position 301 in SEQ ID NO:2) in the thrombin B chain be substituted. Examples of the amino acids to be substituted for the arginine at position 98, the arginine at position 245, the lysine at position 248, and the lysine at position 252, respectively, include alanine and the like, but are not particularly limited as long as the derivative of interest can be obtained.

With respect to the decrease in the heparin-binding ability, when the decrease in the heparin-binding ability is caused by the substitution outside the heparin-binding region (e.g., exosite II region or the like) such as the substitution of the active center amino acid, it is considered that the substitution gave an adverse effect, such as distortion or the like, on the entire molecular structure of a thrombin derivative, and further, on the tertiary structure of the exosite II, thereby leading to the decrease in the heparin-binding ability.
Meanwhile, when the administration into a living body is actually performed, the active center amino acid can be substituted according to an optimal combination as disclosed in the present invention, and then an amino acid except the active center amino acids, that is, an amino acid which exists in the exosite II such as the arginine at position 98 in the B chain, the arginine at position 245 in the B chain, or the lysine at position 248 in the B chain can be positively substituted by other amino acid such as alanine depending on the purpose, to thereby obtain a thrombin derivative having a decreased heparin-binding ability. It is expected that the binding ability to heparan sulfate or the like on a vascular endothelial cell is decreased by using the derivative having a decreased heparin-binding ability, to thereby increase the amount circulating in blood.
Such thrombin derivative having been introduced with amino acid substitutions in the exosite II region in addition to the substitution of the active center amino acids preferably has a binding ability to heparin gel of 90% or less of that of thrombin before the amino acid substitution in the exosite II region.
Examples of such thrombin derivative include: a thrombin derivative (i.e., sequence of amino acid numbers 44 to 351 in SEQ ID NO:54) in which the serine at position 205, the histidine at position 43, and the lysine at position 65 are each substituted by alanine, and the arginine at position 245 is additionally substituted by alanine; and a thrombin derivative (i.e., sequence of amino acid numbers 44 to 351 in SEQ ID NO:56) in which the serine at position 205, the histidine at position 43, and the lysine at position 65 are each substituted by alanine, and the lysine at position 248 is additionally substituted by alanine. Those derivatives each have an APTT-prolonging effect and have a relatively decreased affinity to heparin as compared to that of the human wild-type thrombin, due to the substitution of the amino acid on the exosite II.

WO 2002/004008 and WO 2002/007748 describe that thrombin sends a multiplicative signal to various cells independent of hydrolysis into PAR, through a region of Arg-Gly-Asp-Ala (i.e., from position 197 to position 200 in the thrombin B chain: region corresponding to position 246 to position 249 in SEQ ID NO:2), and that the sequence Arg-Gly-Asp-Ala as a whole is essential for the signal pathway. With the derivatives obtained in the present invention, the cell multiplicative signal is not necessary, and therefore any amino acid of the sequence Arg-Gly-Asp-Ala may be substituted as other amino acid.
For instance, a derivative (i.e., sequence of amino acid numbers 44 to 351 in SEQ ID NO:48) in which the arginine at position 197 (i.e., arginine corresponding to the arginine at position 246 in SEQ ID NO:2), the serine at position 205, and the histidine at position 43 in the B chain as described in Experimental Example 25 were each substituted by alanine had a relatively high APTT-prolonging ability as compared to a derivative in which the serine at position 205 and the histidine at position 43 were each substituted by alanine. This derivative is considered to lose the cell signaling ability due to the sequence Arg-Gly-Asp-Ala.
Meanwhile, the phenylalanine at position 19 (i.e., phenylalanine corresponding to the phenylalanine at position 68 in SEQ ID NO:2) in the B chain may be substituted. An example of such thrombin derivative includes a thrombin derivative (i.e., sequence of amino acid numbers 44 to 351 in SEQ ID NO:58) in which the serine at position 205, the histidine at position 43, and the phenylalanine at position 19 are each substituted by alanine.

The thrombin derivative according to the second aspect of the present invention more preferably has activated partial thromboplastin time of 1.5 fold or more and a thrombomodulin-binding ability decreased to 50% or less, due to the substitution of the amino acid except the active center amino acid.
The thrombin derivative according to the second aspect of the present invention more preferably has at least one or more of improved antithrombotic effect selected from the group consisting of the APTT-prolonging effect, the modified thrombin-induced platelet aggregation-inhibiting effect, and the ristocetin-induced platelet aggregation-inhibiting effect.

Hereinafter, the substitution of the amino acid except the active center amino acids in the second aspect of the present invention will be explained by way of specific examples.
Specific example 1: the APTT-prolonging effect of the thrombin derivative 3 (thrombin derivative in which the glycine at position 203 and the serine at position 205 were substituted by alanine and glycine, respectively) exemplified in the specific description of the first aspect of the present invention was not so high. On the other hand, the thrombin derivative (sequence of amino acid numbers 44 to 351 in SEQ ID NO:28) in which the lysine at position 77 was substituted by glutamic acid in addition to the substitution of the active center amino acids performed for the thrombin derivative 3 had a decreased Fbgn-binding ability and a relatively increased FVIII-binding ability as compared to the Fbgn-binding ability, thus leading to a high APTT-prolonging effect.

Specific example 2: the thrombin derivative 2 (thrombin derivative in which the serine at position 205 and the histidine at position 43 were each substituted by alanine) exemplified in the specific description of the first aspect of the present invention had a high APTT-prolonging effect. The thrombin derivative (sequence of amino acid numbers 44 to 351 in SEQ ID NO:38, 44, or 50) in which the lysine at position 77 was substituted by glutamic acid, alanine, or serine in addition to the substitution of the active center amino acids performed for the thrombin derivative 2 exhibited an even stronger APTT-prolonging effect. That is, it is considered that the change in the binding specificity to a thrombin substrate caused by the substitution of the active center amino acids and the change in the binding specificity to a thrombin substrate caused by the substitution of the amino acid except the active center amino acids gives a synergetic effect on the intensity of drug efficacy.

Specific example 3: the thrombin derivative (sequence of amino acid numbers 44 to 351 in SEQ ID NO:40) in which the glutamine at position 24 was substituted by glutamic acid in addition to the substitution of the active center amino acids performed for the thrombin derivative 2 had an APTT-prolonging effect and a significantly decreased TM-binding ability.

Specific example 4: the derivative (sequence of amino acid numbers 44 to 351 in SEQ ID NO:46) in which the lysine at position 65 was substituted by alanine in addition to the substitution of the active center amino acids performed for the thrombin derivative 2 showed a double-barreled effect of an increased APTT-prolonging effect and a decreased TM-binding effect. The substitution of the lysine at position 65 in the exosite I by alanine revealed a slight decrease in the binding ability to hirudin gel. However, the FVIII-binding ability was maintained and the FVIII specificity with respect to Fbgn increased.

Specific example 5: the derivative (sequence of amino acid numbers 44 to 351 in SEQ ID NO:52) in which the lysine at position 65 and the lysine at position 77 were each substituted by alanine in addition to the substitution of the active center amino acids performed for the thrombin derivative 2 showed an extremely high synergetic effect on an APTT-prolonging effect due to the substitutions of the amino acids at two positions.

Specific example 6: the derivative (sequence of amino acid numbers 44 to 351 in SEQ ID NO:54) in which the lysine at position 65 and the arginine at position 245 were each substituted by alanine in addition to the substitution of the active center amino acids performed for the thrombin derivative 2 had an antithrombotic ability and showed decreased heparin-binding ability.
As described above, thrombin derivatives having various characteristics can be obtained by, in addition to the substitutions of the active center amino acids, the substitution of other amino acid, in particular, the amino acid in the exosite I region or exosite II region.

### 3-3. Modification of carboxyl group <Third aspect of the present invention>

The carboxyl group of the thrombin derivative according to the first and the second aspects of the present application is modified according to the method described in Patent Document 2 (WO 02/077031) to decrease the binding ability to Fbgn and increase the selectivity to thrombin substrates such as a thrombin receptor, to thereby attain the antithrombotic effect even in a small amount.
In addition, not only the APTT is prolonged, but the antiplatelet effect, particularly inhibiting effect on the PAR1 activation and the ristocetin-induced platelet aggregation (due to the interaction between GPIbα and vWF) can be added and controlled depending on the substitution of the amino acid. To be specific, there can be obtained a thrombin derivative which has an extremely strong APTT-prolonging effect and a strong antiplatelet effect, a derivative having a moderate APTT-prolonging effect and a strong antiplatelet effect, and the like. In other words, the carboxyl group of the thrombin derivative of the present invention can be modified, to thereby obtain a thrombin derivative having an anti-blood coagulation effect and an antiplatelet effect which can be applied to various thrombosis.

When the carboxyl group of the thrombin derivative of the present invention is modified according to the method described in Patent Document 2 (WO 02/077031), the modification of the carboxyl group may preferably performed by using carbodiimide.

The carboxyl group of the thrombin derivative of the present invention can be modified with a compound having an amino group. The compound having an amino group is not particularly limited, but preferable examples thereof include esters of amino acid and polyethylene glycol having an amino acid in its side chain. An example of the esters of amino acid includes glycine ethyl ester. The modification by using the compound having an amino group can also be performed according to the method described in WO 02/077031. On the other hand, the thrombin derivative of the present invention may be reacted with polyethylene glycol, to thereby modify the carboxyl group. When polyethylene glycol or polyethylene glycol having an amino group is used, the molecular weight of the polyethylene glycol moiety is preferably 1,000 or less.

It should be noted that when the modification of the carboxyl group is performed, the effect thereof is affected by the number of modification. To obtain the effects described in the description of the following modified products 1 to 5, it is preferable that carboxyl groups at three or more positions be modified. When the number of the carboxyl group to be modified is three or more, the effect attained by the modification of the carboxyl group can be clearly obtained. Meanwhile, the number of the carboxyl groups to be modified is desirably 25 or less. When the number of the carboxyl groups to be modified is 25 or less, the effect attained by the modification of the carboxyl group can be clearly obtained in a similar manner, and at the same time, a decrease in yield due to the modification hardly occurs. A thrombin derivative obtained by introducing or deleting a carboxyl group by amino acid substitution on the surface of a thrombin derivative does not include the increase or decrease in the number of the carboxyl group due to the substitution.
Further, derivatives having various effects are obtained depending on the number of modification. For instance, when the modification sites in the modified product 1 described below is decreased from 8 or 5, a derivative having an extremely high APTT-prolonging effect and a moderate to decreased antiplatelet effect can be obtained.

When the carboxyl group is modified in the present invention, at least the carboxyl group of the glutamic acid at position 25 in the B chain is preferably modified. The thrombin derivative of the present invention in which the carboxyl group is modified is preferably a thrombin derivative which has an improved ristocetin aggregation-inhibiting ability and/or has a GPIbα-antagonizing ability in platelet.

The effect obtained by the substitution of the amino acid except the active center amino acids to increase the antithrombotic effect, and the effect obtained by the modification of a carboxyl group widely vary depending on the combination of the amino acid to be substituted or the like. Depending on the disease and clinical condition, or depending on the purpose, there can be used a thrombin derivative into which only the amino acid substitution is introduced, or a thrombin derivative in which the modification of the carboxyl group is combined with the amino acid substitution.
In other words, for thrombosis in a vein such as deep vein thrombosis, a drug which inhibits the blood coagulation is used in general. Therefore, it is desirable to use a thrombin derivative having a high APTT-prolonging effect selected from the respective thrombin derivatives and respective carboxyl group-modified products thereof.
Meanwhile, a drug which inhibits the platelet aggregation is used in general for thrombosis in an arterial system such as cardiac infarction and instable angina pectoris, so thrombin derivatives each having a high platelet aggregation inhibiting effect are desirable. Among those, the thrombin derivatives having different APTT-prolonging effects need to be selected depending on the circumstances of side effects such as bleeding.
In addition, the inhibition of coagulation system as well as that of platelets has been reported to be important for thrombosis accompanied with the opening of a plaque at a site of atherosclerosis ("Thrombosis", Nankodo Co., Ltd.), and therefore the inhibition of both the platelet and the coagulation is effective. No such drug which acts on both the coagulation and platelet is commercially available at present, and thus the present invention provides a radically new therapeutic agent.

Hereinafter, the relationship between the substitution of the active center amino acids and that of the amino acid except the active center amino acids and the carboxyl group modification will be described by way of specific examples. The following specific examples show a derivative in which about 15 carboxyl groups are modified, which is obtained by using water-soluble carbodiimide as a condensation agent and by modifying with glycine ethyl ester.
Modified product 1: the thrombin derivative in which the serine at position 205 and the histidine at position 43 are each substituted by alanine had an extremely high APTT-prolonging effect without the modification of a carboxyl group. The thrombin derivative is subjected to the carboxyl group modification, to thereby obtain a thrombin derivative which has an APTT-prolonging effect increased to a certain degree and a high ristocetin-induced platelet aggregation-inhibiting effect and M-thrombin-induced platelet aggregation-inhibiting effect in platelet, thus resulting in an extremely high effects of the anti-blood coagulation and antiplatelet capacities.

Modified product 2: the derivative in which the serine at position 205 is substituted by glycine and the glycine at position 203 is substituted by alanine did not have a high APTT-prolonging effect and antiplatelet effect before the modification of the carboxyl group. However, the modification of the carboxyl group increased the APTT-prolonging effect and the antithrombotic effect, producing a thrombin derivative having a moderate APTT-inhibiting effect and a high antiplatelet effect.

Modified product 3: the thrombin derivative in which the serine at position 205 and the glycine at position 203 are each substituted by alanine did not have a high APTT-prolonging effect. Even when the carboxyl group of the thrombin derivative was modified, the increase in the APTT-prolonging effect was not significantly observed.

Modified product 4: the thrombin derivative in which the serine at position 205 is substituted by glycine, the glycine at position 203 is substituted by alanine, and the lysine at position 77 is substituted by glutamic acid has an APTT-prolonging effect but with an extremely weak antiplatelet effect. When the thrombin derivative is subjected to the carboxyl group modification, an additional increase in the APTT-prolonging effect was hardly observed, however, the antiplatelet effect was significantly amplified. However, the modification of the carboxyl group significantly decreased the yield thereof to 50% or less.

Modified product 5: the derivative in which the serine at position 205 is substituted by alanine had a relatively weak APTT-prolonging effect and an extremely weak ante-platelet effect. When the carboxyl group modification of the thrombin derivative was performed, no significant increase in the APTT-prolonging effect was observed while the antiplatelet effect is significantly amplified with respect to the pathways of both the PAR1 and ristocetin.

Modified product 6: the derivative in which the serine at position 205, the histidine at position 43, and the lysine at position 65 are each substituted by alanine has a strong APTT-prolonging effect and a strong platelet aggregation-inhibiting effect of the PAR1 pathway. On the other hand, the derivative did not have the ristocetin-induced platelet aggregation-inhibiting effect. When the thrombin derivative was subjected to the carboxyl group modification, the APTT-prolonging effect slightly increased, the M-thrombin derivative-induced platelet aggregation effect slightly increased, and the ristocetin-induced platelet aggregation-inhibiting effect was newly imparted. In addition, the significant decrease in yield observed in the modified product 4 did not occur.

It should be noted that in a case of a derivative which inhibit only GPIbα, there can be used a thrombin derivative obtained by modifying PPACK-thrombin (thrombin obtained by subjecting the sequence Phe-Pro-Arg to chloromethylketone modification) which has decreased thrombin substrate-cleaving activity and thrombin substrate-binding ability due to obstruction of the active center. However, also in this case, more efficient effect is exerted by applying the substitution of the amino acid described herein, which decreases the binding ability to TM.

The various thrombin derivatives of the present invention can be selectively used depending on the purpose. For instance, the thrombin derivative 2 as described above is expected to be effective for a fibrin thrombus (red thrombus) in a vein, and the carboxyl group-modified product of the thrombin derivative 2 is expected to be effective also for a platelet thrombus (white thrombus) in an artery.
Alternatively, it is considered that a thrombin derivative obtained by subjecting the serine at position 205, the histidine at position 43, and the lysine at position 77 of the thrombin derivative with no carboxyl group modification to substitution by alanine, alanine, and glutamic acid, respectively, is also effective for the intravenous thrombus.
When the bleeding causes a problem in a disease such as a platelet thrombus in an artery, a carboxyl group-modified product of the thrombin derivative 3 which has a low APTT-prolonging effect is desirable rather than the carboxyl group-modified product of the thrombin derivative 2. Selection can be made in consideration of the risk of the disease for patients and the risk of bleeding.

### 4. Application and the like of the present invention

According to the present invention, there is also provided a DNA which encodes any of the above-mentioned thrombin derivatives. Examples of such DNA include DNAs each encoding a thrombin derivative precursor including a nucleotide sequence of numbers 130 to 1,056 in SEQ ID NO:7, 13, 21, 23, 25, 27, 33, 35, 37, or 39. Those DNAs may each have a change in codon without changing the encoded amino acid sequence. Further, as long as the particular amino acid as described above is substituted and a thrombin derivative having the activity of interest is encoded, the examples of the DNA may include one which hybridizes under stringent conditions with a DNA comprising a nucleotide sequence of nucleotide numbers 130 to 1,056 in SEQ ID NO:7, 13, 21, 23, 25, 27, 33, 35, 37, or 39. Here, an example of the stringent condition includes a condition where washing is performed once, or more preferably twice to three times, at a salt concentration corresponding to 60°C, 1 x SSC, 0.1 % SDS which is a condition for washing in a normal Southern hybridization, preferably 60°C, 0.1 x SSC, 0.1% SDS, and particularly preferably 65°C, 0.1 x SSC, 0.1% SDS.
It should be noted that the DNA of the present invention is not limited to a DNA encoding the thrombin derivative precursor, and DNAs each encoding the A chain and the B chain are also included in the DNA of the present invention.

The thrombin derivative of the present invention has an antithrombotic effect. This action can be confirmed from, for example, the APTT determination, the determination of the whole blood coagulation time, or platelet aggregation ability inhibiting effect as described in Examples. In the present invention, the confirmation of the antiplatelet effect is performed by using the M-thrombin-induced platelet aggregation-inhibiting effect for evaluation.
It is considered that M-thrombin activates a platelet through a thrombin receptor (PAR1) because the M-thrombin induces the platelet aggregation in PRP (platelet rich plasma) and the induction of the platelet aggregation is completely inhibited by an anti-PAR1 antibody (ATAP2, Cosmo Bio Co., Ltd.). Therefore, it is considered that the inhibiting effect of the derivative obtained in the present invention on the M-thrombin-induced platelet aggregation is based on the thrombin receptor activation-inhibiting effect.

The thrombin derivative of the present invention may be a thrombin derivative in which a sodium-binding site is substituted in addition to the above-mentioned substitution. The term "sodium-binding site" refers to a site disclosed in Biochemistry, 1992, Vol.31, p11721-11730. Here, the aspartic acid at position 232 or position 234 in the B chain (aspartic acid corresponding to the aspartic acid at position 281 or 283 in SEQ ID NO:2) is preferably substituted. Both of the aspartic acids may be substituted. The aspartic acids may each be substituted by alanine or asparagine.

The thrombin derivative of the present invention can be combined with a pharmaceutically acceptable carrier to be used as a pharmaceutical composition. Here, preferable examples of the pharmaceutical composition include an antithrombotic drug, an anti-inflammatory agent, a platelet aggregation-inhibiting agent, a platelet adhesion-inhibiting agent, a platelet GPIb antagonizing agent, and a thrombin receptor activation-inhibiting agent. The pharmaceutical acceptable carrier is not particularly limited as long as it is pharmaceutically acceptable, and examples thereof which can be used include a solvent for injection, a stabilizer, a diluent, and a surfactant which are widely used in a common drug. The form of dosage unit for the pharmaceutical composition of the present invention is not particularly limited and may appropriately be selected depending on the purpose of a treatment. An example thereof includes an injection. The dosage of the pharmaceutical composition of the present invention is appropriately selected depending on the disease condition and the like.

### EXAMPLES

Hereinafter, the present invention will be described in more detail by way of examples. However, the present invention is not limited to the examples as long as it falls within the gist of the present invention.

### <1> Assay of thrombin substrate-cleaving activity

Method A: the assay was carried out on the basis of an increase in absorption at a wavelength of 405 nm at 37°C by using a synthetic substrate S2238 (Sigma-Aldrich) as a substrate in 50 mM Tris-HCl (pH 8).
200 µl each of 50 mM Tris-HCl/0.1 M NaCl solution (pH 7.4) containing a test sample (i.e., human wild-type thrombin or thrombin derivative) (concentration of 1 µg/ml in the case of the human wild-type thrombin, and concentration of 200 µg/ml in the case of the thrombin derivative) and 50 mM Tris-HCl/0.1 M NaCl solution (pH 7.4) containing a synthetic substrate S2238 were added to an Eppendorf tube, and incubated at 37°C for 12 hours. The reaction was stopped by adding 200 µl of 50% acetic acid.
A control was prepared by adding 200 µl each of 50 mM Tris-HCl/0.1 M NaCl solution (pH 7.4) containing a synthetic substrate S2238 and 50 mM Tris-HCl/0.1 M NaCl (pH 7.4) to an Eppendorf tube, and then incubating at 37°C for 12 hours.
After completion of the 12-hour incubation, the absorbance of the control increased by 0.005 compared to the absorbance before incubation. When an increase in the absorbance of the test sample after completion of the 12-hour incubation was 0.05 or less compared to the absorption before incubation, the increase was evaluated to be less than measuring limit.
A thrombin whose activity decreased to such a level that no activity was observed in the method A was further subjected to activity measurement by any one of the methods B, C, and D.

Method B: Fibrogammin P (Aventis Pharma) was used as FXIII. With respect to 50 µl of solution in an Eppendorf tube obtained by subjecting 3 ml of Fibrogammin P 250 units to dialysis against 50 mM EDTA/0.1 M NaCl (pH 7.4), 100 µl of PBS solution (pH 7.4) containing 0.1 mg/ml of a test sample (i.e., thrombin derivative) were added, and the whole was incubated at 37°C for 3 hours. After that, the presence or absence of the activation of the FXIII was confirmed by SDS-PAGE. When a cleaved product was visually observed, SDS-PAGE was carried out under a "-SH" condition, to thereby compare and analyze the band densities of an A chain and an activated A chain of the FXIII by using a light capture (Atto Corporation).

Method C: 200 µl of solution of 50 mM Tris-HCl/0.1 M NaCl (pH 7.4) containing 4 mg/ml Fbgn dissolved therein was added to 100 µl of a test sample (i.e., thrombin derivative) adjusted to be 0.2 mg/ml. The whole was well mixed and incubated at 37°C for 3 hours. Three hours later, the presence or absence of clot formation was visually confirmed.

Method D: 200 µl of 50 mM Tris-HCl/0.1 M NaCl (pH 7.4) containing 0.1 mg/ml of a thrombin receptor extracellular domain peptide (SEQ ID NO:4) dissolved therein was added to 100 µl of a test sample (i.e., thrombin derivative) adjusted to be 0.3 mg/ml. The whole was well mixed and incubated at 37°C for 3 hours. The mixture was added with 200 µl of 50% acetic acid to stop the reaction. After that, the presence or absence of the activation of the thrombin receptor was confirmed by SDS-PAGE. When a cleaved product was visually observed, SDS-PAGE was carried out under a "-SH" condition, to thereby compare and analyze the band densities of the cleaved products of the thrombin receptor by using a light capture (Atto Corporation).

### <2> Method of measuring substrate binding ability

Method E: Confirmation of a binding ability to a hirudin C-terminal peptide (SEQ ID NO:3)
(1) Preparation of hirudin C-terminal peptide gel
   10 mg of a hirudin C-terminal peptide was dissolved in 0.1 M NaHCO₃ buffer solution. The solution was added and mixed with 10 ml of an NHS-activated Cellulofine (Chisso Corporation) which had been equilibrated by the buffer solution, and the whole was stirred at 25°C for 30 minutes.
   The mixture was added with 20 ml of 1 M Tris-HCl (pH 8, 25°C) and the whole was stirred for additional 30 minutes, to thereby obtain hirudin C-terminal peptide-immobilized gel. The gel was equilibrated with 50 mM Tris-HCl/0.15 M NaCl (pH 7.4) at 25°C. After that, the resultant was added with each of the thrombin derivatives, which had been subjected to dialysis against 0.1 M NaHCO₃ buffer solution, and washed with 30 ml of the 0.1 M HaHCO₃ buffer solution.
(2) Confirmation of binding ability to hirudin C-terminal peptide (SEQ ID NO:3)
   2 ml of a fraction containing a human wild-type thrombin or a thrombin derivative was added to 10 ml of a hirudin C-terminal peptide column which had been equilibrated with 50 mM Tris-HCl/0.15 M NaCl (pH 8) at 4°C. The resultant was washed with 30 ml of 50 mM Tris-HCl buffer solution and was then subjected to elution with 50 mM Tris-HCl/1 M NaCl/3 M urea (pH 8). Western blotting was carried out by using an anti-human thrombin antibody to confirm the thrombin in a flow-flow-through fraction or an eluted fraction. In the present invention, a thrombin derivative having a binding ratio to the hirudin C-terminal peptide-immobilized gel of 50% or less than that of the test sample which has been added to the column was evaluated to have lost the substrate binding ability.
<3> Method of confirming heparin binding ability
Method F: 5 ml of a human wild-type thrombin or a thrombin derivative was added to an HI-TRAP HEPARIN column (Amersham Pharmacia) which had been equilibrated with 50 mM NaHCO₃/50 mM NaCl solution, and the whole was washed with 15 ml of the 50 mM NaHCO₃/50 mM NaCl solution. After that, by using buffer A: 50 mM NaHCO₃/50 mM NaCl and buffer B: 50 mM NaHCO₃/l M NaCl, the resultant was subjected to gradient elution from B = 0% to B = 100% at a flow rate of 0.5 ml/min for 100 minutes.
The human wild-type thrombin had a peak of elution at A = 50% (0.5 M NaCl). A thrombin derivative, which was eluted at a salt concentration of A = 40% or lower as compared to that of the human wild-type thrombin under such a condition, was evaluated to have a decreased heparin binding ability by 80%.

### Method F: Measurement of thrombin receptor binding ability of thrombin derivative by using biosensor (IAsys, Nissei Sangyo Co., Ltd.)

### (1) Preparation of a thrombin derivative-immobilized cuvette

A test sample in 10 mM phosphate buffer (pH 7.7) (i.e., thrombin derivative) was stirred in an NHS-activated CM dextran cuvette (Nissan Sangyo Co., Ltd.) at 25°C for 10 minutes to immobilize the test sample (i.e., thrombin derivative) on the NHS-activated CM dextran cuvette, to thereby obtain a thrombin derivative-immobilized cuvette. Subsequently, 0.2 ml of 1 M ethanol amine (pH 8) was added thereto to perform a blocking treatment. (2) 0.1 ml of 50 mM phosphate buffer (pH 7.4)/0.15 M NaCl solution each adjusted to contain 1,000, 500, 200, 100, 50, 25, or 10 nM of a thrombin receptor extracellular domain peptide (SEQ ID NO:4) was added to the thrombin derivative-immobilized cuvette obtained in the above-mentioned section (1), to thereby analyze binding curves.
The analysis was performed by using FAST fit (Nissei Sangyo Co., Ltd.) according to a manual available from the manufacturer.

### <3> Method of measuring APTT

The determination of APTT was carried out by the method as described below as long as the method was not particularly specified in the examples of the present invention.
Standard plasma (Sysmex International Reagents Co., Ltd.) and a sample were mixed. The mixture was added with an APTT reagent (Sysmex International Reagents Co., Ltd.) (25% with respect to the total amount) and the whole was incubated at 37°C for 5 minutes. Five minutes later, the mixture was added with 0.1 M CaCl₂ to make a concentration of 8 µM and a time period from the addition of calcium to coagulation was determined.

### <4> Synthesis of AHT

AHT to be used in the examples of the present invention is obtained by the following experimental processes.
4-1: Synthesis of AHT
60 mg of a human wild-type thrombin was dissolved in 50 ml of 100 µM PIPES (pH 6.5) and added with 10 mg of APMSF (WAKO), and the whole was stirred for 10 minutes. Subsequently, the dissolved sample was cooled with ice water and added with 6 ml of 1 N NaOH, and the whole was allowed to react for 10 minutes (reaction was carried out in ice water). After that, the resultant was added with 14 ml of 5 M NaCl and 70 ml of glycerol, and the whole was stirred and adjusted to pH 8.0 with 1 M PIPES (pH 6.5). Further, the adjusted sample was diluted by being dropped to 560 ml of 0.1 M NaHCO₃-0.5 M NaCl, followed by dialysis against 0.1 M NaHCO₃-0.5 M NaCl. Subsequently, the dialyzed sample was subjected to centrifugation at 8,000 rpm for 5 min to remove a precipitate, and then the sample was concentrated to a final volume of 100 ml.
1 ml of anti-thrombin (AT) 50 units/ml was added with 10 mg of heparin, and the resultant was added to the concentrated sample, followed by dialysis against 50 mM NaHCO₃-0.3 M NaCl. After that, the resultant was subjected to centrifugation at 8,000 rpm for 5 min to remove a precipitate.

### 4-2: Purification of AHT

50 ml of benzamidine-sepharose which had been equilibrated with 50 mM NaHCO₃ was added with 50 ml of the sample obtained in the method A (at a flow rate of 2 ml/min). The resultant was washed with 50 mM NaHCO₃-0.1 M NaCl and then subjected to elution with 0.1 M benzamidine-50 mM NaHCO₃-0.1 M NaCl. The confirmation of a protein was carried out by means of BCA, and a fraction containing an AHT protein was added to heparin-sepharose which had been equilibrated with 50 mM NaHCO₃ (at a flow rate of 2 ml/min). After that, the resultant was washed with 1 mM PIPES-0.1 M NaCl (pH 6.5) to remove benzamidine, and then AHT was eluted therefrom with 1 mM PIPES ? 1 M NaCl (pH 6.5). The eluted fraction was added with 10 mg of APMSF and the whole was subjected to dialysis against 1mM PIPES ? 0.1 M NaCl (pH 6.5).

### 4-3: Second purification of benzamidine

Next, the sample was added with 1 mg of APMSF just before the sample was again added to a column, and then the mixture was added to a benzamidine-sepharose column which had been equilibrated with 1 mM PIPES (pH6.5). The resultant was washed with 1 mM PIPES-0.1 M NaCl (pH 6.5) and then subjected to elution with 0.1 M benzamidine-1 mM PIPES ? 0.1 M NaCl (pH 6.5). An AHT fraction was added to heparine-sepharose which had been equilibrated with 1 mM PIPES (pH 6.5) and washed with 1 mM PIPES-0.1 M NaCl (pH 6.5), followed by elution with 1 mM PIPES-1 M NaCl (pH 6.5)

### 4-4: third purification of benzamidine

The obtained sample was added with 10 mg of APMSF and the whole was subjected to dialysis with 1 mM PIPES-0.1 M NaCl (pH 6.5). After that, AHT was purified by operations similar to those used for the second purification of benzamidine. Heparin-eluted AHT was again added with 1 mg of APMSF and about 30 mg of AHT was collected.

### 4-5: Method of measuring APTT

100 µl of PBS solution (PBS; 137 mM NaCl, 2.68 mM KCI, 8.1 mM Na₂HPO₄, 1.47 mM KH₂PO₄) (pH 7.4) containing AHT or a thrombin derivative adjusted to have a concentration of 50 µg/ml and 25 µg/ml, respectively, was mixed with 100 µl of standard plasma (Sysmex International Reagents Co., Ltd.), respectively. The resultants were added with 50 µl of an APTT reagent (Sysmex International Reagents Co., Ltd.) and the whole was incubated at 37°C for 5 minutes. After that, the resultants were added with 22 µl of 0.1 M CaCl₂, and time periods from the addition of calcium to coagulation (precipitation of fibrin clot) were determined, respectively. Standard plasma which had been added with only PBS in a similar method was used as a control, and APTT was determined.
In the examples described hereinbelow, similar methods were employed for the incubation, the APTT reagent, the addition of the calcium solution, and the determination of APTT.
The APTT-prolonging ability of each thrombin derivative was judged to have increased when the APTT has increased with respect to a control such as AHT, in at least one of the concentrations of 50 µg/ml and 25 µg/ml. For instance, when the APTT is increased 1.1 folds or more with respect to a control in at least one of the concentrations of 50 µg/ml and 25 µg/ml, it was judged that the APTT-prolonging effect increased 1.1 folds or more.

### 4-6: Determination of APTT of AHT

100 µl of PBS solution (PBS; 137 mM NaCl, 2.68 mM KCI, 8.1 mM Na₂HPO₄, 1.47 mM KH₂PO₄) (pH 7.4) containing AhT or a thrombin derivative adjusted to have concentrations of 50 µg/ml and 25 µg/ml, respectively, was mixed with 100 µl of standard plasma (Sysmex International Reagents Co., Ltd.), respectively. The resultant was added with 50 µl of an APTT reagent (Sysmex International Reagents Co., Ltd.) and incubated at 37°C for 5 minutes. After that, the resultants were each added with 22 µl of 0.1 M CaCl₂, and time periods from the addition of calcium to coagulation (i.e., precipitation of fibrin clot) were determined, respectively. Standard plasma which had been added with only PBS instead of the AhT solution in a similar manner was used as a control. The results were as described below.
Control: 46.5 seconds
AHT 50 µg/ml: 63.5 seconds (1.36 folds)
AHT 25 µg/ml: 58.5 seconds (1.25 folds)

### [Experimental Example 1]

### (1) Expression of a human wild-type thrombin

A DNA (SEQ ID NO:5) containing an A chain and a B chain of human wild-type thrombin was inserted into a vector to transfect a CHO cell, to thereby obtain a prethrombin producing cell.
The sequence of the human wild-type prethrombin shown in SEQ ID NO:6 includes a signal sequence of amino acid numbers 1 to 43, an A chain of amino acid numbers 44 to 92, and a B chain of amino acid numbers 93 to 351.
The prethrombin producing cell was cultured in 2 liters of a CD-CHO medium for 10 days. 2 liters of the obtained culture solution of the prethrombin producing cell was subjected to dialysis against 20 liters of 10 mM PIPES buffer solution (pH 7) at 4°C twice for 6 hours each. Then, the resultant was added to 500 ml of CM cellulofine (Chisso Corporation) and washed with 1 liter of 10 mM PIPES buffer solution (pH 7). Next, the resultant was subjected to elution with a liner concentration gradient of 10 mM PIPES buffer solution (pH 7) from 0 to 1 M NaCl. The eluate was divided into fractions of 25 ml each, and each of the fractions was subjected to Western blotting by using an anti-human thrombin polyclonal antibody (Cosmo Bio Co., Ltd.), to thereby confirm that the human wild-type thrombin was eluted out at about 0.4 M.

### (2) Activation of human wild-type thrombin by ecarin, and confirmation of binding ability to hirudin C-terminal peptide of the activated human wild-type thrombin

About 100 ml of a fraction containing 5 mg of the obtained human wild-type thrombin was subjected to dialysis against 2 liters of 50 mM Tris-HCl buffer solution (pH 8) containing 0.15 M NaCl and 5 mM, CaCl₂. After that, the resultant was added with 100 units of ecarin (Sigma-Aldrich) and the whole was incubated at 37°C for 24 hours. The experiment on the binding to the hirudin C-terminal peptide as described in the method E was carried out by using part of the ecarin-treated thrombin, and it was confirmed that there was no thrombin in the flow-flow-through fraction and that there was a band of thrombin in the eluted fraction.

### (3) Purification of the human wild-type thrombin

Next, 98 ml of a solution containing the ecarin-activated thrombin, which is the rest of the thrombin used in the experiment of the binding to the hirudin C-terminal peptide, was added to 200 ml of a sulfated cellulofine column (Chisso Corporation) which had been equilibrated with 50 mM Tris-HCl buffer (pH 8) containing 0.1 M NaCl. The column was washed with 200 ml of the buffer, and then subjected to elution with 50 mM Tris-HCl buffer (pH 8) containing 1 M NaCl. The eluate was further subjected to dialysis against 50 mM Tris-HCl buffer (pH 8) containing 0.1 M NaCl. The resultant was added to 30 ml of a hirudin C-terminal peptide column (prepared according to the method described in the "method E: Confirmation of binding ability to hirudin C-terminal peptide (SEQ ID NO:3)" except that the amount of the hirudin C-terminal peptide was changed to 200 mg and that of the NHS-activated cellulofine (Chisso Corporation) was changed to 30 ml) which had been equilibrated with the buffer. The hirudin C-terminal peptide column was washed with 150 ml of 50 mM Tris-HCl buffer and then subjected to elution with 50 mM Tris-HCl buffer (pH 8) containing 1 M NaCl and 4 M guanidine hydrochloride, to thereby obtain about 5 mg of almost purified human wild-type thrombin having hirudin-binding ability on SDS-PAGE.

### (4) Synthesis of a carboxyl group-modified thrombin (M-thrombin)

1 mg of the human wild-type thrombin which had been dissolved in 5 ml of 50 mM phosphate buffer (pH 6.5) containing 0.5 M NaCl was subjected to dialysis against 0.25 M glycine ethyl ester/0.5 M NaCl (pH 6.5) at 4°C for 3 hours. After that, the resultant was added with 1-ethyl-3-(3-dimethylaminopropyl)-carbodiimide (Wako Pure Chemical Industries, Ltd.) to make the concentration thereof 20 mg/ml. The mixture was incubated at 25°C for 1 hour to modify carboxyl groups of the human wild-type thrombin. Glycine was added thereto so that the final concentration of the remaining carbodiimide became 0.5 M, thereby completing the reaction. As a result of mass analysis, the molecular weight thereof increased by about 1,400, which showed that about 15 carboxyl groups had been modified.

### (5) Evaluation by using PRP, about the induction of platelet aggregation and inhibition of anti-PAR1 antibody (ATAT2, Cosmo Bio Co., Ltd.) by the modified thrombin

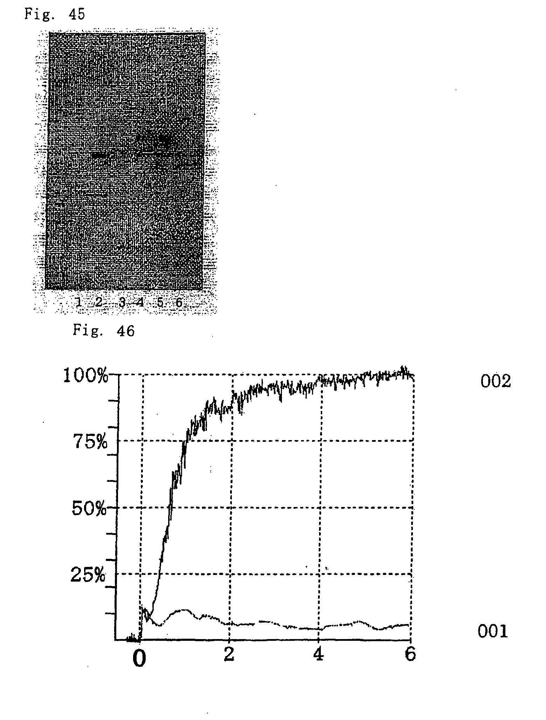

Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP. 100 µl of PBS was added to 140 µl of PRP, and the mixture was added with 35 µl of PBS solution containing 1 µg/ml of the modified thrombin, and the time course of the transmittance was recorded. The results are shown in "002" in Fig. 46.
Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP. 100 µl of PBS containing 50 µg/ml of PAR1 was added to 140 µl of PRP, the mixture was added with 35 µl of PBS containing 1 µg/ml of the modified thrombin, and the time course of the transmittance was recorded. The results are shown in "001" in Fig. 46.
The above-mentioned results showed that the modified thrombin induced the platelet aggregation, and that the platelet aggregation caused by the modified thrombin was completely inhibited by the PAR1 antibody. Accordingly, it was confirmed that the modified thrombin induces the platelet aggregation through activation of PAR1.

### [Experimental Example 2]

### (1) Expression of a thrombin derivative (hereinafter, referred to as "203A205G thrombin") in which glycine at position 230 in B chain is substituted by alanine, and serine at position 205 in B chain is substituted by glycine

Synthesis was carried out by PCR using a mutation-introduced primer corresponding to the DNA of 203A205G thrombin. SEQ ID NO:7 shows the nucleotide sequence of a gene encoding the 203A205G thrombin.
The 203A205G thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction, and a band equivalent to that of thrombin was observed at the elution peak. Subsequently, the purification was carried out by using the sulfated cellulofine column and the hirudin C-terminal peptide column according to the method of the above-mentioned section (3) in Experimental Example 1, to thereby obtain about 5 mg of almost purified 203A205G thrombin on SDS-PAGE. Next, the binding ability to heparin gel was measured according to the method F, and an eluate was obtained at the same elution position (A50%) as that of the wild-type human thrombin.

### (2) Measurement of substrate-cleaving activity of the 203A205G thrombin

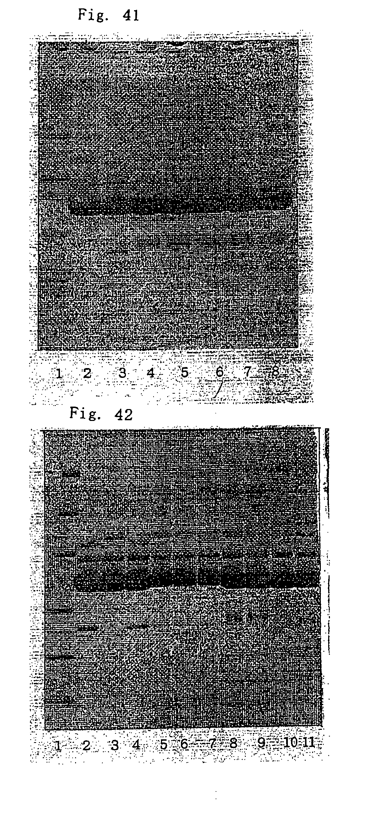

The thrombin substrate-cleaving activity of the 203A205G thrombin obtained in the above-mentioned section (1) was assayed according to the method A. As a result, no significant increase in absorbance after the incubation was observed.
Further, Fig. 41 shows the result of measurement of the thrombin substrate-cleaving activity of the 203A205G thrombin which was carried out according to the method B. No band of an A chain-activated product of FXEII was observed in the sample (lane 6) which was obtained after 3-hour incubation. Furthermore, the thrombin substrate-cleaving activity of the 203A205G thrombin was measured according to the method C, and as a result, no clot formation was observed.

### (3) Confirmation of binding ability of 203A205G thrombin to thrombin receptor

The binding ability of the 203A205G thrombin obtained in the above-mentioned section (1) to a thrombin receptor was measured according to the method F. The 203A205G thrombin had a dissociation constant of 3.2 µM to a thrombin receptor.

### (4) Determination of APTT of 203A205G thrombin

100 µl of 50 µg/ml 203A205G thrombin (PBS; 137 mM NaCl, 2.68 mM KCI, 8.1 mM Na₂HPO₄, 1.47 mM KH₂PO₄) (pH 7.4) was mixed with 100 µl of standard plasma (Sysmex International Reagents Co., Ltd.) and APTT was determined. Standard plasma which had been added with only PBS was used as a control for the determination. As a result, in the control it took 44 seconds, and in the 203A205G thrombin it took 48 seconds, which was prolonged by 1.09 folds.

### (5) Evaluation of antiplatelet effect of 203A205G thrombin by using PRP (platelet rich plasma)

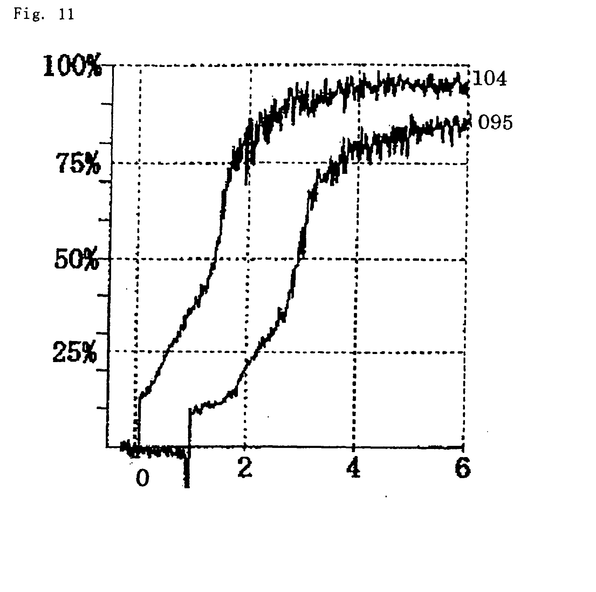

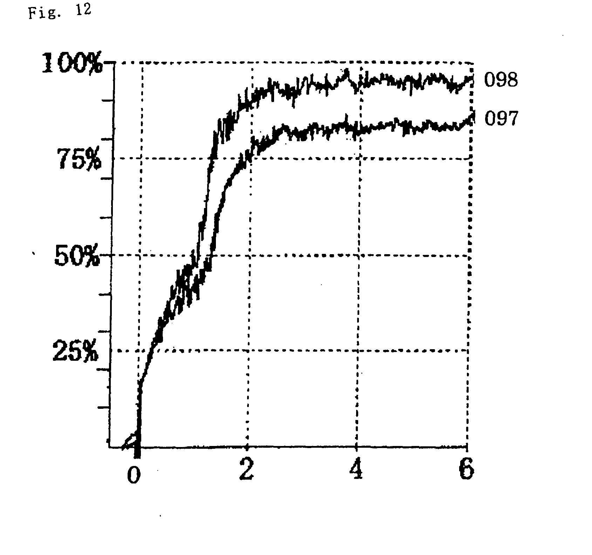

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,500 rpm for 10 minutes, to thereby obtain PPP (platelet poor plasma). 100 µl of 5 mM phosphate buffer (pH 7.4)/0.15 M NaCl containing the 203A205G thrombin, which had been adjusted so that the final concentration of the 203A205G thrombin was 80 µg/ml when 100 µl of the solution was added, was added to 130 µl of PRP. The mixture was added with 35 µl of 5 mM phosphate buffer (pH 7.4)/0.15 M NaCl containing 5 mg/ml ristocetin. As a control, 130 µl of PRP was added with 100 µl of 5 mM phosphate buffer (pH 7.4)/0.15 M NaCl, and the time course of the transmittance was recorded. The measurement of the transmittance (at wavelength of 700 nm) was carried out by using EASY TRACER ET-800 (Tokyo Koden). Fig. 11 shows the results. The transmittance represented by the vertical axis is a value which indicates a positive correlation with the platelet aggregating ability.
Evaluation 2: The experiment was carried out according to the method in Evaluation (1) except that 1 µg/ml M-thrombin (human wild-type)/5 mM phosphate buffer (pH 7.4)/0.15 M NaCl was used as a platelet aggregation-inducing substance. As a control, 130 µl of PRP was added with 100 µl of 5 mM phosphate buffer (pH 7.4)/0.15 M NaCl, and the time course of the transmittance was recorded. The measurement of the transmittance was carried out by using EASY TRACER ET-800 (Tokyo Koden). Fig. 12 shows the results.
From Fig. 11 and Fig. 12, results indicating that a significant inhibiting effect on the platelet aggregation was not attained even by adding 80 µg/ml of the 203A205G thrombin were obtained.

### (6) Experiment for comparing thrombin substrate-binding abilities by using IAsys

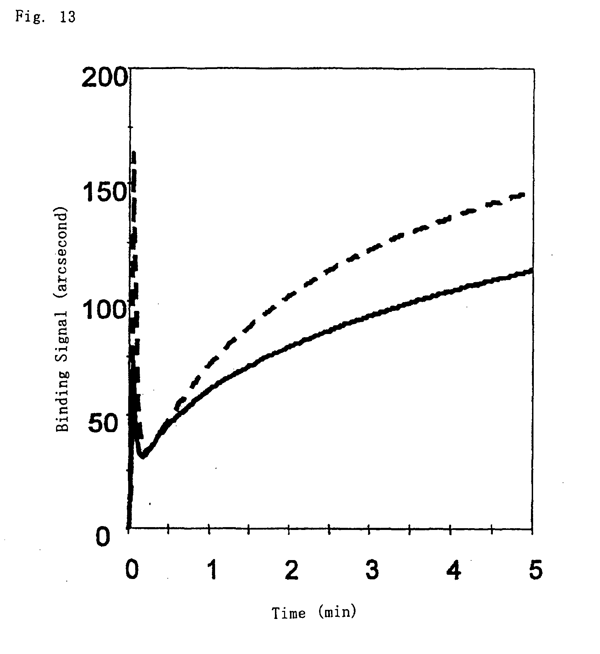

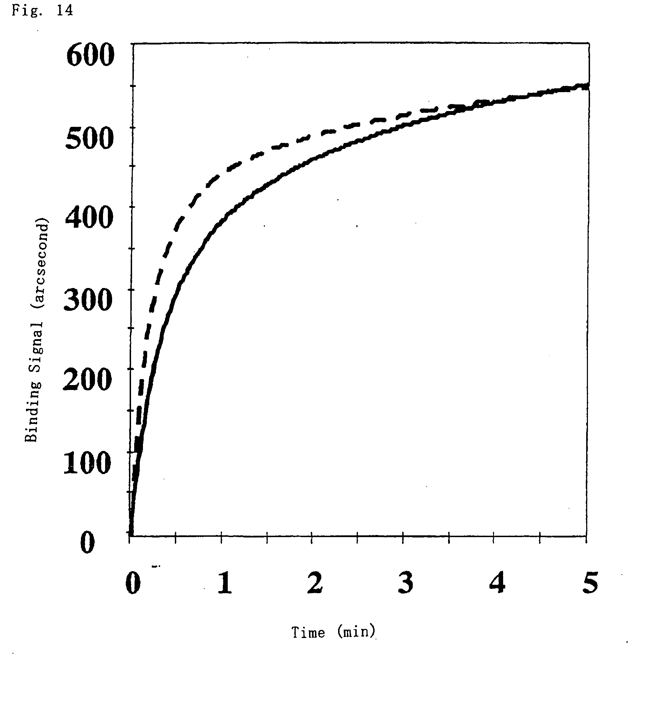

10 mM phosphate buffer (pH 7.4) containing 0.1 mg/ml 203A205G thrombin derivative or AHT was added to an NHS-activated CM dextran cuvette (Nissei Sangyo Co., Ltd.). Each of the solutions was stirred at 25°C for 10 minutes to immobilize a test sample (i.e., thrombin derivative) on the NHS-activated CM dextran cuvette, to thereby obtain 203A205G thrombin- and AHT-immobilized cuvettes (the cuvettes had 4,100 arc of the bound 203A205G thrombin and 2,400 arc of the bound AHT, respectively). Subsequently, 0.2 ml of 1 M ethanol amine (pH 8) was added thereto to perform a blocking treatment. The 203A205G cuvette and AHT cuvette were each added with 100 nM of Fbgn and FVIII, and respective binding curves were monitored. Fig. 13 and Fig. 14 each show the results.
Fig. 14 showed that the AHT cuvette had almost the same amounts of Fbgn and FVIII bound thereto while Fig. 13 showed that the 203A205G cuvette had a smaller amount of FVIII bound thereto than that of Fbgn. It was revealed that the 203A205G thrombin had a lower FVIII-binding ability compared to that of AHT which was obtained by simply converting serine at position 205 into dehydroalanine so as to minimize the structural change. Meanwhile, the 203A205G thrombin had a ratio of the FVIII binding per the solidified amount decreased to about 11% as compared to the AHT. Accordingly, it was considered that the 203A205G thrombin had a low APTT-prolonging effect.

### (7) Determination of APTT of carboxyl group-modified 203A205G thrombin and prothrombin time (PT)

1 mg of 203A205G thrombin which had been dissolved in 5 ml of 50 mM phosphate buffer (pH 6.5)/0.5 M NaCl was subjected to dialysis against 0.25 M glycine ethyl ester/0.5 M NaCl (pH 6.5) at 4°C for 3 hours. After that, the resultant was added with 1-ethyl-(3-dimethylaminopropyl)-carbodiimide (Wako Pure Chemical Industries, Ltd.) to make the concentration thereof 20 mg/ml. The mixture was incubated at 25°C for 1 hour to modify carboxyl groups f the 203A205G thrombin. Glycine was added thereto so that the final concentration of the remaining carbodiimide became 0.5 M, thereby completing the reaction. As a result of mass analysis, the molecular weight thereof increased about 1,400, which showed that about 15 carboxyl groups were modified.

### Evaluation 1: APTT determination 1

500 µg of the modified 203A205G thrombin was dissolved in 1 ml of 5 mM PIPES buffer (pH 7.4)/0.1 M NaCl. 50 µl of the mixture was added to standard plasma (Sysmex International Reagents Co., Ltd.) so as to have a volume ratio of 1:10, and APTT was determined. As a control, 5 mM PIPES buffer (pH 7.4)/0.15 M NaCl was added to standard plasma (Sysmex International Reagents Co., Ltd.) so as to have a volume ratio of 1:10, and APTT was determined. The APTT reagent available from Sysmex International Reagents Co., Ltd. was used. As a result, the APTT of the control was 38 seconds while the APTT of the carboxyl group-modified 203A205G thrombin was 65 seconds.
Evaluation 2: APTT determination 2
100 µl of a mixture obtained by dissolving 50 µg of the modified 203A205G thrombin in 1 ml of PBS was added to 100 µl of standard plasma (Sysmex International Reagents Co., Ltd.), and APTT was determined. A solution obtained by adding PBS to standard plasma (Sysmex International Reagents Co., Ltd.) in a similar manner was used as a control, and APTT was determined. The APTT reagent available from Sysmex International Reagents Co., Ltd. was used. As a result, the APTT of the control was 44 seconds while the APTT of the carboxyl group-modified 203A205G thrombin was 85 seconds.

### Evaluation 3: Determination of PT

A mixture obtained by dissolving 500 µg of the modified 203A205G thrombin in 1 ml of 5 mM PIPES buffer (pH 7.4)/0.15 M NaCl was added to standard plasma (Sysmex International Reagents Co., Ltd.) so as to have a volume ratio of 1:10, and PT was determined. As a control, 5 mM PIPES buffer (pH 7.4)/0.15 M NaCl was added to standard plasma (Sysmex International Reagents Co., Ltd.) so as to have a volume ratio of 1:10, and PT was determined. The PT reagent available from Sigma-Aldrich as "thromboplastin with calcium" was used. As a result, the PT of the control was 20 seconds while the PT of the carboxyl group-modified 203A205G thrombin was 21 seconds.

### (8) Evaluation of antiplatelet effect of carboxyl group-modified 203A205G thrombin by using PRP (platelet rich plasma)

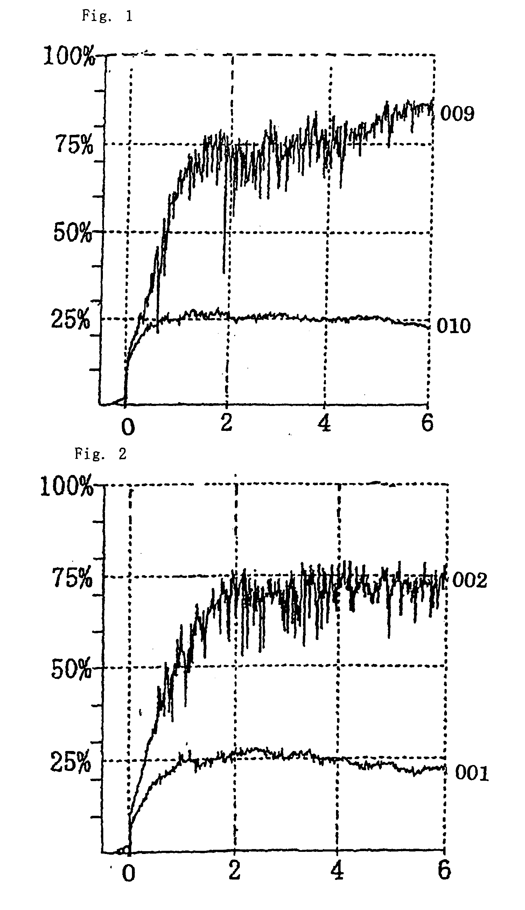

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP (platelet poor plasma). 100 µl of 5 mM phosphate buffer (pH 7.4)/0.15 M NaCl containing the carboxyl group-modified 203A205G thrombin, which had been adjusted so that a final concentration of the carboxyl group-modified 203A205G thrombin was 37 µg/ml when 100 µl of the solution was added, was added to 130 µl of PRP. The mixture was added with 35 µl of 5 mg/ml ristocetin/5 mM phosphate buffer (pH 7.4)/0.15 M NaCl as a platelet aggregation-inducing substance. As a control, 130 µl of PRP was added with 100 µl of 5 mM phosphate buffer (pH 7.4)/0.15 M NaCl, and the time course of the transmittance was recorded. The measurement of the transmittance (at wavelength of 700 nm) was carried out by using EASY TRACER ET-800 (Tokyo Koden). Fig. 1 shows the results. The transmittance represented by the vertical axis is a value which indicates a positive correlation with the platelet aggregating ability.
Evaluation 2: The experiment was carried out according to the method in Evaluation (1) except that 1 µg/ml M-thrombin (human wild-type)/5 mM phosphate buffer (pH 7.4)/0.15 M NaCl was used as a platelet aggregation-inducing substance. As a control, 130 µl of PRP was added with 100 µl of 5 mM phosphate buffer (pH 7.4)/0.15 M NaCl, and the time course of the transmittance was recorded. The measurement of the transmittance was carried out by using EASY TRACER ET-800 (Tokyo Koden). Fig. 2 shows the results.
Fig. 1 and Fig. 2 showed that the platelet aggregation was suppressed by the use of the carboxyl group-modified 203A205G thrombin, so it was revealed that the thrombin derivative exhibits an antiplatelet effect in the ristocetin-induced platelet aggregation and modified thrombin-induced platelet aggregation.

The above-mentioned results revealed that the 203A205G thrombin has an activity which was decreased to a level less than the detection limit while maintaining the binding ability to a substrate. Further, the results of APTT and the results of the experiment of platelet aggregation inhibition revealed that the 203A205G thrombin did not have sufficient antithrombotic effect and antiplatelet effect at a concentration of 80 µg/ml, while the carboxyl group-modified 203A205G thrombin, in which about 15 carboxyl groups were modified, had sufficient antithrombotic effect and antiplatelet effect even at a low concentration.
In addition, from the experiment for comparing the substrate binding capacities by using IAsys, the 203A205G thrombin had a low FVIII-binding ability and a ratio of binding per immobilized proteins decreased to about 11 % as compared to that of AHT. Thus, the 203A205G thrombin was considered to have no APTT-prolonging effect. The 203A205G thrombin having modified carboxyl groups had an APTT-prolonging effect equivalent to that of the 205A43A thrombin described in Experimental Example 12 while the 203A205G thrombin having modified carboxyl groups also exhibited a platelet aggregation-inhibiting effect equivalent to that of the 205A43A thrombin. As compared to that of the 205A43A thrombin having modified carboxyl groups, the 203A205G thrombin having modified carboxyl groups had a weaker APTT-prolonging effect but exhibited a strong platelet aggregation-inhibiting effect at an equivalent level.

### (9) Evaluation of relationship between number of carboxyl groups modified in 203A205G thrombin and antithrombotic effect

### (9)-1. Preparation of a carboxyl group-modified 203A205G derivative having 1 to 10 modification(s)

1 mg of 203A205G thrombin which had been dissolved in 5 ml of 50 mM phosphate buffer (pH 6.5)/0.5 M NaCl was subjected to dialysis against 0.25 M glycine ethyl ester/0.5 M NaCl (pH 6.5) at 4°C for 3 hours. After that, the resultant was added with 1-ethyl-3-(3-dimethylaminopropyl)-carbodiimide (Wako Pure Chemical Industries, Ltd.) to make the concentration thereof 2 to 20 mg/ml. The mixture was incubated at 25°C for 60 minutes to 120 minutes, to thereby obtain carboxyl group-modified thrombin derivatives 1 to 5 each having 2 to 28 modified carboxyl groups of the 203A205G thrombin.

### (9)-2. APTT-prolonging effects of the respective carboxyl group-modified thrombin derivatives 1 to 5 which had been subjected to dialysis against PBS

100 µl of a mixture obtained by dissolving 50 µg of the modified 203A205G thrombin in 1 ml of PBS was added to 100 µl of standard plasma (Sysmex International Reagents Co., Ltd.), and APTT was determined. A solution obtained by adding PBS to standard plasma (Sysmex International Reagents Co., Ltd.) in a similar manner was used as a control, and APTT was determined. The APTT reagent available from Sysmex International Reagents Co., Ltd. was used.
The results were as shown below.
203A205G thrombin
About 0 modification APTT 47 seconds
Carboxyl group-modified thrombin derivative 1
About 1 modification APTT 48 seconds
Carboxyl group-modified thrombin derivative 2
About 3 modifications APTT 56 seconds
Carboxyl group-modified thrombin derivative 3
About 5 modifications APTT 61 seconds
Carboxyl group-modified thrombin derivative 4
About 10 modifications APTT 77 seconds
Carboxyl group-modified thrombin derivative 5
About 26 modifications APTT 60 seconds
As described above, it was confirmed that the 203A205G thrombin utilizing EDC had a significant APTT-prolonging effect by three or more carboxyl group modifications. The yields of the carboxyl group-modified thrombin derivatives 1 to 4 before and after the modification were 75% or more. The derivative having 26 modifications showed an APTT-prolonging effect, however, it was revealed that an aggregation occurred and the yield was as low as 40%, leading to a decrease in the yield due to excess carboxyl group modification.

### [Experimental Example 3]

### (1) Expression of thrombin (hereinafter, referred to as "205A thrombin") in which serine at position 205 in B chain is substituted by alanine

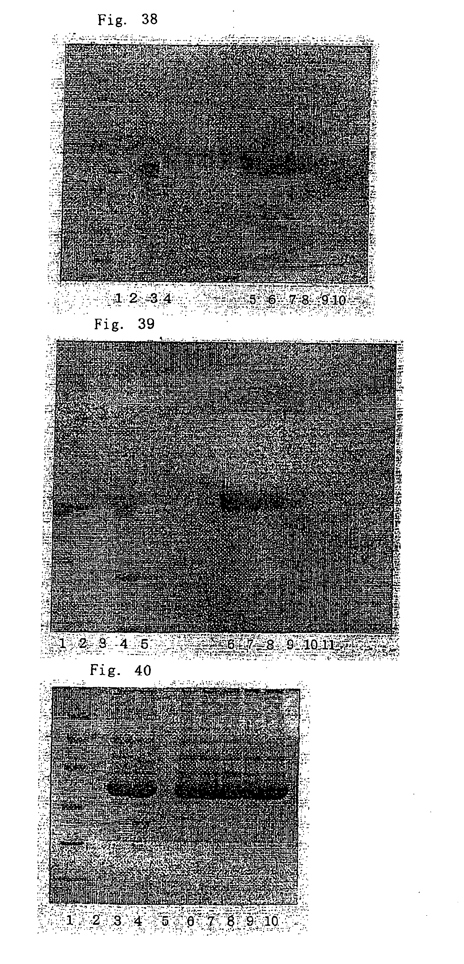

Synthesis was carried out by PCR using a mutation-introduced primer corresponding to the DNA of 205A thrombin. SEQ ID NO:9 shows the nucleotide sequence of a gene encoding the 205A thrombin.
The 205A thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 6 mg of almost purified 205A thrombin on SDS-PAGE. Fig. 39 shows the results of electrophoresis of the purification of the 205A thrombin by means of the hirudin C-terminal-immobilized peptide. No band of the 205A thrombin was observed in the flow-through fraction in the lane 5 while a band of purified 205A thrombin was observed in the eluted fraction.
The above-mentioned results showed that the 205A thrombin had the binding ability to the hirudin C-terminal peptide-immobilized gel.
Next, the binding ability to heparin gel was measured according to the method F, and an eluate was obtained at the same elution position (A50%) as that of the wild-type human thrombin.

### (2) Measurement of substrate-cleaving activity of 205A thrombin

Fig. 40 shows the results of measurement of the thrombin substrate-cleaving activity of the 205A thrombin obtained in the above-mentioned section (1), which was carried out according to the method B. Even 3 hours later, no A chain of activated FXIII was observed as shown in lane 8.

### (3) Determination of APTT of 205A thrombin

100 µl of solution obtained by dissolving 500 µg of 205A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 55 seconds while the APTT of the 205A thrombin was 95 seconds.
100 µl of a solution obtained by dissolving 50 µg of 205A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 41 seconds while the APTT of the 205A thrombin was 62 seconds.

Further, 1 mg/5 ml of 205A thrombin/50 mM phosphate buffer (pH 6.5)/0.5 M NaCl was subjected to dialysis against 0.25 M glycine ethyl ester/0.5 M NaCl (pH 6.5) at 4°C for 3 hours. After that, the resultant was added with 1-ethyl-3-(3-dimethylaminopropyl)-carbodiimide (Wako Pure Chemical Industries, Ltd.) to make the concentration thereof 20 mg/ml. The mixture was incubated at 25°C for 1 hour to modify carboxyl groups of the 205A thrombin. About 80% of the modified derivatives were obtained in a solubilized state.
100 µl of a solution obtained by dissolving 50 µg of carboxyl group-modified 205A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 42 seconds while the APTT of the carboxyl group-modified 205A thrombin was 79 seconds.

### (4) Determination of prothrombin time (PT)

Solutions obtained by respectively dissolving 50 µg of 205A thrombin and carboxyl group-modified 205A thrombin in 1 ml of PBS were each added to standard plasma (Sysmex International Reagents Co., Ltd.) in a volume ratio of 1:1, and PT was determined. A solution obtained by adding PBS to standard plasma (Sysmex International Reagents Co., Ltd.) in a volume ratio of 1:1 was used as a control, and PT was determined. The PT reagent available from Sigma-Aldrich as "thromboplastin with calcium" was used. As a result, the PT of the control was 21 seconds while the PT of the 205A thrombin was 21 seconds and that of the carboxyl group-modified 205A thrombin was 20 seconds. Thus, both the 205A thrombin and the carboxyl group-modified 205A thrombin exhibited no prolonging effects.

### (5) Evaluation of antiplatelet action of 205A thrombin by using PRP (platelet rich plasma)

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP (platelet poor plasma). 100 µl of 5 mM phosphate buffer (pH 7.4)/0.15 M NaCl of the 205A thrombin, which had been adjusted so that a final concentration of the 205A thrombin was 70 µg/ml when 100 µl of the solution was added, was added to 130 µl of PRP. The mixture was then added with 35 µl of 5 mg/ml ristocetin/5 mM phosphate buffer (pH 7.4)/0.15 M NaCl as a platelet aggregation-inducing substance. For a control, 130 µl of PRP was added with 100 µl of 5 mM phosphate buffer (pH 7.4)/0.15 M NaCl, and the time course of the transmittance was recorded. The measurement of the transmittance (at wavelength of 700 nm) was carried out by using EASY TRACER ET-800 (Tokyo Koden). However, the platelet aggregation-inhibiting effect was not observed at a concentration of 70 µg/ml in this experiment.
Evaluation 2: The experiment was carried out according to the method in Evaluation (1) except that 1 µg/ml M-thrombin (human wild-type)/5 mM phosphate buffer (pH 7.4)/0.15 M NaCl was used as a platelet aggregation-inducing substance. As a control, 130 µl of PRP was added with 100 µl of 5 mM phosphate buffer (pH 7.4)/0.15 M NaCl, and the time course of the transmittance was recorded. The measurement of the transmittance was carried out by using EASY TRACER ET-800 (Tokyo Koden). However, the platelet aggregation-inhibiting effect was not observed at a concentration of 70 µg/ml in this experiment.
As described above, it was confirmed that the 205A thrombin had a weak APTT-prolonging effect. However, no antiplatelet effect was observed. It was indicated that the 205A thrombin exhibits a thrombin receptor activation-inhibiting ability under an environment where the medium does not contain plasma as shown in Patent Document 4, but the 205A thrombin does not have the thrombin receptor-activating ability of a level at which the 205A thrombin can be practically used as an antiplatelet agent in plasma.

### (6) Evaluation of antiplatelet effect of carboxyl group-modified 205A thrombin by using PRP

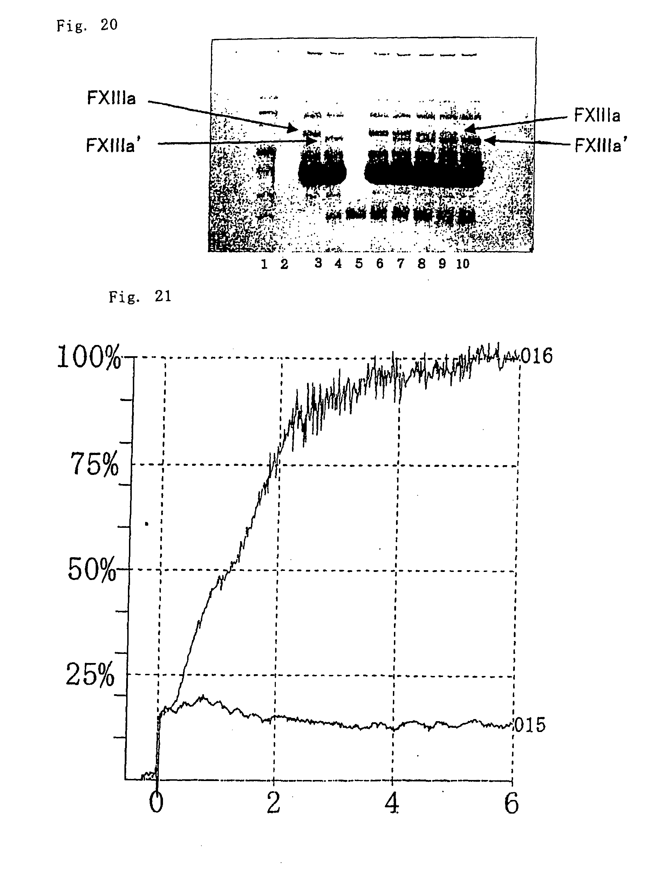

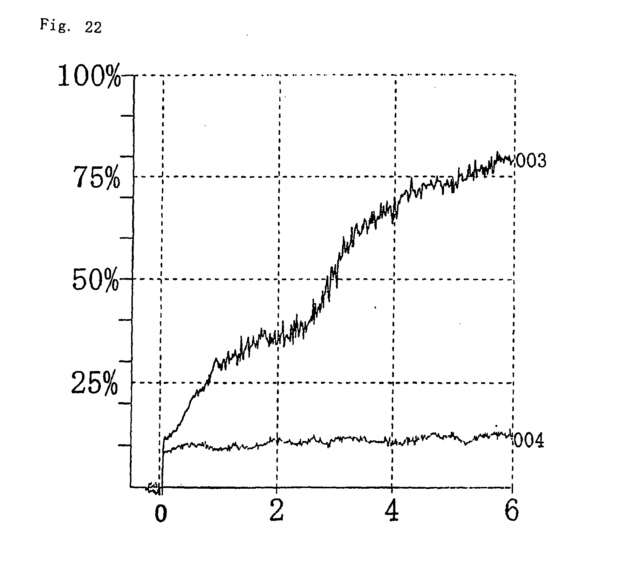

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP. 100 µl of PBS solution of the carboxyl group-modified 205A thrombin, which had been adjusted so that a final concentration of the carboxyl group-modified 205A thrombin was 100 µg/ml when 100 µl of the solution was added, was added to 130 µl of PRP. The mixture was added with (i) 1 µg/ml M-thrombin/PBS solution or (ii) 5 mg/ml ristocetin/PBS solution, each serving as an inducing substance. As a control, 130 µl of PRP was added with 100 µl of 5 mM phosphate buffer (pH 7.4)/0.15 M NaCl, and the experiment on platelet aggregation inhibition was carried out. Fig. 21 and Fig. 22 each show the results of the above-mentioned items (i) and (ii).

### (7) Comparison of binding ability of 205A thrombin to Fbgn and FVIII

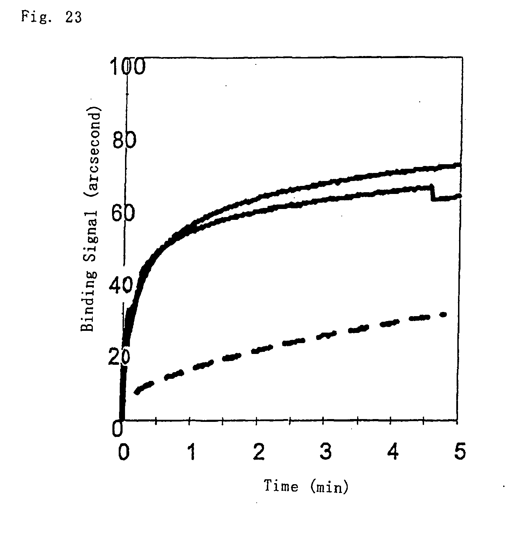

10 mM phosphate buffer (pH 7.7) containing 205A thrombin at a concentration of about 0.1 mg/ml was added to NHS-activated CM dextran cuvette (Nissan Sangyo Co., Ltd.). The mixtures was stirred at 25°C for 10 minutes to immobilize the test sample (i.e., thrombin derivative) on the NHS-activated CM dextran cuvette, to thereby obtain a 205A thrombin-immobilized cuvette (About 600 arc of immobilization). Subsequently, 0.2 ml of 1 M ethanol amine (pH 8) was added thereto to perform a blocking treatment.
The 205A thrombin cuvette was added respectively with 100 nM of Fbgn and FVIII, to thereby monitor respective binding curves. Fig. 23 shows the results. Fig. 23 showed that the 205A thrombin had an enhanced FVIII specificity while a FVIII binding signal itself relatively decreased to about 40% as compared to that of AHT (Fig. 14). Therefore, it was considered that 195A thrombin did not exhibit a strong antithrombotic ability. In addition, the 195A thrombin attained an increased APTT-prolonging effect by the modification of the carboxyl groups, and exhibited inhibiting effects on both M-thrombin-induced platelet aggregation and ristocetin-induced platelet aggregation.

### [Experimental Example 4]

### (1) Expression of thrombin (hereinafter, referred to as "205T thrombin") in which serine at position 205 in B chain is substituted by threonine

Synthesis was carried out by PCR using a mutation-introduced primer corresponding to the DNA of 205T thrombin. SEQ ID NO:11 shows the nucleotide sequence of a gene encoding the 205T thrombin.
The 205T thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 5 mg of almost purified 205T thrombin on SDS-PAGE.

### (2) Measurement of substrate-cleaving activity of 205T thrombin

A thrombin substrate-cleaving activity of the 205T thrombin obtained in the above-mentioned section (1) was assayed according to the method A. As a result, the substrate-cleaving activity of the 205T thrombin was about 2.5 x 10⁻⁴ folds as compared with that of the human wild-type thrombin.
Further, the thrombin substrate-cleaving activity of the thrombin was measured according to the method B. As a result, A chains of almost all FXIH were activated in a similar manner as that of the wild-type thrombin, and bands of cleaved FXIII were observed. As described above, it was revealed that the activity of the 205T thrombin was still left therein.

### [Experimental Example 5]

### (1) Expression of thrombin (i.e., 203A205A99N thrombin) in which glycine at position 203 in B chain is substituted by alanine, serine at position 205 in B chain is substituted by alanine, and aspartic acid at position 99 in B chain is substituted by asparagine

Synthesis was carried out by PCR using a mutation-introduced primer corresponding to the DNA of 203A205A99N thrombin. SEQ ID NO:13 shows the nucleotide sequence of a gene encoding the 203A205A99N thrombin.
The 203A205A99N thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 5 mg of almost purified 203A205A99N thrombin on SDS-PAGE.
Next, the binding ability to heparin gel was measured according to the method F, and an eluate was obtained at A40%, whereby a decrease in affinity to the heparin gel was confirmed.

### (2) Measurement of substrate-cleaving activity of 203A205A99N thrombin

The thrombin substrate-cleaving activity of the 203A205A99N thrombin obtained in the above-mentioned section (1) was assayed according to the method A. As a result, no increase in absorption after incubation was observed.
Further, the thrombin substrate-cleaving activity of the 203A205A99N thrombin was measured according to the method B. As a result, no band of an A chain-activated product of FXIII was observed.

### (3) Determination of APTT of blood added with 203A205A99N thrombin

A solution obtained by dissolving 100 µg of 203A205A99N thrombin in 1 ml of 5 mM PIPES buffer (pH 7.4)/0.15 M NaCl was added to standard plasma (Sysmex International Reagents Co., Ltd.) in a volume ratio of 1:1, and APTT was determined. A solution obtained by adding 5 mM PIPES buffer (pH 7.4)/0.15 M NaCl to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 55 seconds while the APTT of the 203A205A99N thrombin was 60 seconds. Accordingly, it was revealed that the 203A205A99N thrombin had a binding ability to hirudin gel, and that the 203A205A99N thrombin had a sufficiently decreased activity while not having a sufficient antithrombotic effect.

### [Experimental Example 6]

### (1) Expression of thrombin (hereinafter, referred to as "205V thrombin") in which serine at position 205 in B chain is substituted by valine

Synthesis was carried out by PCR using a mutation-introduced primer corresponding to the DNA of 205V thrombin. SEQ ID NO:15 shows the nucleotide sequence of a gene encoding the 205V thrombin.
The 205V thrombin was expressed by the method of the above-mentioned section (1) in Experimental Example 1. The binding ability to a hirudin C-terminal peptide was confirmed according to the method of the above-mentioned section (2) in Experimental Example 1. Fig. 44 shows the results of Western blot under reduction of S-S bond. Almost all bands of thrombin were observed in a flow-through fraction and no thrombin was observed at the elution peak. Accordingly, it was confirmed that the amino acid substitution in the active center decreased the substrate binding ability of the 205V thrombin. The band which had appeared upstream of the molecular weight of the standard thrombin in a pre-applied sample represented thrombin which has not been activated or cleaved by ecarin into A and B chains, and the thrombin was confirmed to have a significantly decreased sensitivity to ecarin.

### [Experimental Example 7]

### (1) Expression of thrombin (hereinafter, referred to as "205D thrombin") in which serine at position 205 in B chain is substituted by aspartic acid

Synthesis was carried out by PCR using a mutation-introduced primer corresponding to the DNA of 205D thrombin. SEQ ID NO:17 shows the nucleotide sequence of the 205D thrombin.
The 205D thrombin was expressed by the method of the above-mentioned section (1) in Experimental Example 1. The binding ability to a hirudin C-terminal peptide was confirmed according to the method of the above-mentioned section (2) in Experimental Example 1. Fig. 45 shows the results of Western blot under reduction of S-S bond. All bands of thrombin were observed in a flow-through fraction and no thrombin was observed at the elution peak. Accordingly, it was confirmed that the amino acid substitution in the active center decreased the substrate binding ability of the 205D thrombin. The band which had appeared upstream of the molecular weight of the standard thrombin in a pre-applied sample represented thrombin which has not been activated or cleaved by ecarin into A and B chains, and the thrombin was confirmed to have a significantly decreased sensitivity to ecarin.

### [Experimental Example 8]

### (1) Expression of thrombin (hereinafter, referred to as "205N thrombin") in which serine at position 205 in B chain is substituted by asparagine

Synthesis was carried out by PCR using a mutation-introduced primer corresponding to the DNA of 205N thrombin. SEQ ID NO:19 shows the nucleotide sequence of a gene encoding the 205N thrombin.
The 205N thrombin was expressed by the method of the above-mentioned section (1) in Experimental Example 1. The binding ability to a hirudin C-terminal peptide was confirmed according to the method of the above-mentioned section (2) in Experimental Example 1, and almost all bands of thrombin were observed in a flow-through fraction and no thrombin was observed at the elution peak. Accordingly, it was confirmed that the amino acid substitution in the active center decreased the substrate binding ability of the 205N thrombin.

### [Experimental Example 9]

The measurement of the thrombin receptor-binding ability of an AHT was carried out by the method D, and the dissociation constant between the AHT and a thrombin receptor was 1.2 µM.

### [Experimental Example 10]

### (1) Expression of thrombin (hereinafter, referred to as "205A99N thrombin") in which serine at position 205 in B chain is substituted by alanine and aspartic acid at position 99 in B chain is substituted by asparagine

Synthesis was carried out by PCR using a mutation-introduced primer corresponding to the DNA of 205A99N thrombin.
SEQ ID NO:21 shows the nucleotide sequence of a gene encoding the 205A99N thrombin.
The 205A99N thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 5.5 mg of almost purified 205A99N thrombin on SDS-PAGE.

### (2) Measurement of substrate-cleaving activity of 205A99N thrombin

The thrombin substrate-cleaving activity of the 205A99N thrombin obtained in the above-mentioned section (1) was assayed according to the method A. As a result, no significant increase in absorption after the incubation was observed.
Further, the thrombin substrate-cleaving activity of the 205A99N thrombin was determined according to the method B. As a result, no band of an A chain-activated product of FXIII was observed.

### (3) Determination of APTT of 205A99N thrombin

50 µg of 205A99N thrombin was dissolved in 1 ml of PBS. 100 µl of the solution was mixed with 100 µl of standard plasma (Sysmex International Reagents Co., Ltd.), and APTT was determined. As a control, PBS was added to standard plasma (Sysmex International Reagents Co., Ltd.) in a volume ratio of 1:1, and APTT was determined. The APTT reagent available from Sysmex International Reagents Co., Ltd. was used. As a result, the APTT of the control was 55 seconds while the APTT of the 205A99N thrombin was 58 seconds.

### [Experimental Example 11]

### (1) Expression of thrombin (hereinafter, referred to as "203A205A thrombin") in which glycine at position 203 in B chain is substituted by alanine and serine at position 205 in B chain is substituted by alanine

Synthesis was carried out by PCR using a mutation-introduced primer corresponding to the DNA of 203A205A thrombin. SEQ ID NO:23 shows the nucleotide sequence of a gene encoding the 203A205A thrombin.
The 203A205A thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 4 mg of almost purified 203A205G thrombin on SDS-PAGE. Next, the binding ability to heparin gel was measured according to the method F, and an eluate was obtained at A40%, whereby a decrease in affinity to the heparin gel to 40% as compared with human wild-type thrombin was confirmed.

### (2) Measurement of substrate-cleaving activity of 203A205A thrombin

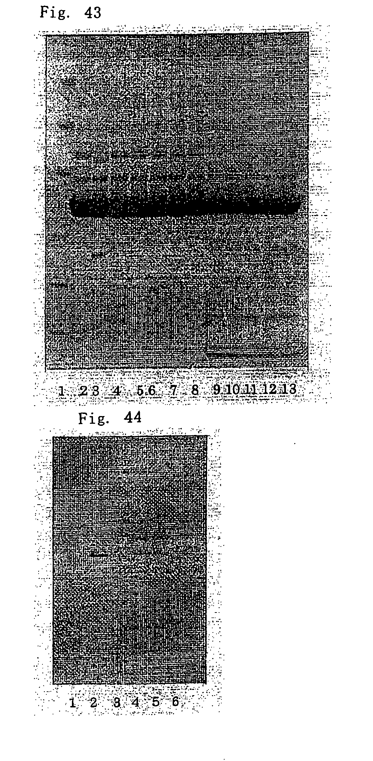

The thrombin substrate-cleaving activity of the 203A205A thrombin obtained in the above-mentioned section (1) was assayed according to the method A. As a result, no increase in absorption after incubation was observed.
Further, the thrombin substrate-cleaving activity of the 203A205A thrombin was measured according to the method B. Fig. 43 shows the results. No band of A chain-activated product of FXIII was observed even in lane 6 which was obtained after 3 hour-incubation. It was revealed that the 203A205A thrombin had a hirudin C-terminal peptide binding ability and that the 203A205A thrombin had sufficiently lost its activity.
A solution obtained by dissolving 100 µg of 203A205A thrombin in 1 ml of 5 mM PIPES buffer (pH 7.4)/0.15 M NaCl was added to standard plasma (Sysmex International Reagents Co., Ltd.) in a volume ratio of 1:1, and APTT was determined. A solution obtained by adding 5 mM PIPES buffer (pH 7.4)/0.15 M NaCl to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 55 seconds while the APTT of the 203A205A thrombin was 59 seconds.

### (3) APTT of carboxyl group-modified product

Further, 1 mg/5 ml of 203A205A thrombin/0.5 M NaCl (pH 6.5) was subjected to dialysis against 0.25 M glycine ethyl ester/0.5 M NaCl (pH 6.5) at 4°C for 3 hours. After that, the resultant was added with 1-ethyl-3-(3-dimethylaminopropyl)-carbodiimide (Wako Pure Chemical Industries, Ltd.) to make the concentration thereof 20 mg/ml. The mixture was incubated at 25°C for 1 hour to modify carboxyl groups of the 203A205A thrombin. About 80% of carboxyl group-modified product was obtained in a solubilized state.
100 µl of a solution obtained by dissolving 50 µg of carboxyl group-modified 203A205A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 45 seconds while the APTT of the carboxyl group-modified 203A205A thrombin was 46 seconds.

### (4) Determination of prothrombin time (PT)

Solutions obtained by respectively dissolving 50 µg of 203A205A thrombin and carboxyl group-modified 203A205A thrombin in 1 ml of PBS were each added to standard plasma (Sysmex International Reagents Co., Ltd.) in a volume ratio of 1:1, and PT was determined. A solution obtained by adding PBS to standard plasma (Sysmex International Reagents Co., Ltd.) in a volume ratio of 1:1 was used as a control, and PT was determined. The PT reagent available from Sigma-Aldrich as "thromboplastin with calcium" was used. As a result, the PT of the control was 20 seconds while the PT of the 203A205A thrombin was 21 seconds and that of the carboxyl group-modified 203A205A thrombin was 20 seconds. Thus, both the 203A205A thrombin and the carboxyl group-modified 203A205A thrombin exhibited no prolonging effects.

### (5) Evaluation of antiplatelet effect of 203A205A thrombin by using PRP

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP. 100 µl of PBS solution of the 203A205A thrombin, which had been adjusted so that a final concentration of the 203A205A thrombin was 100 µg/ml when 100 µl of the solution was added, was added to 130 µl of PRP. The mixture was respectively added with 35 µl of (i) 1 µg/ml M-thrombin/PBS solution and (ii) 5 mg/ml ristocetin/PBS solution, each serving as an inducing substance. As a control, 130 µl of PRP was added with 100 µl of 5 mM phosphate buffer (pH 7.4)/0.15 M NaCl, and the experiment on platelet aggregation inhibition was carried out. However, there was no difference between the control and the 203A205A thrombin with respect to the inducing substances of the above-mentioned items (i) and (ii).

### (6) Comparison of binding specificity of 203A205A thrombin to Fbgn and FVIII

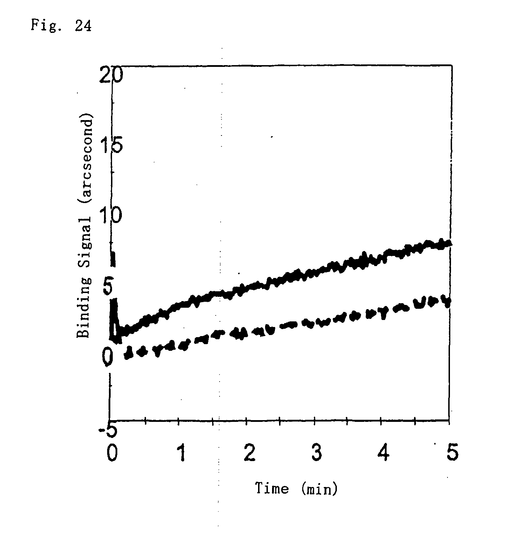

10 mM phosphate buffer (pH 7.7) containing 203A205A thrombin at a concentration of about 0.1 mg/ml was added to NHS-activated CM dextran cuvette (Nissan Sangyo Co., Ltd.). It was stirred at 25°C for 10 minutes to immobilize the test sample (i.e., thrombin derivative) on the NHS-activated CM dextran cuvette, to thereby obtain a 203A205A thrombin-immobilized cuvette. (About 600 arc of solidification) Subsequently, 0.2 ml of 1 M ethanol amine (pH 8) was added thereto to perform a blocking treatment. The 203A205A thrombin cuvette was respectively added with 100 nM of Fbgn and FVIII, to thereby monitor respective binding curves. Fig. 24 shows the results. Fig. 24 showed that the 203A205A thrombin showed about 5% of signal of the FVIII per the solidified protein amount as compared to that of the AHT. It was considered that the substrate binding ability itself of the derivative having the sequence decreased, and thus it had no anticoagulation ability.

### [Experimental Example 12]

### (1) Expression of thrombin (hereinafter, referred to as "205A43A thrombin") in which serine at position 205 in B chain is substituted by alanine and histidine at position 43 in B chain is substituted by alanine

Synthesis was carried out by PCR using a mutation-introduced primer corresponding to the DNA of 205A43A thrombin. SEQ ID NO:25 shows the nucleotide sequence of a gene encoding the 205A43A thrombin.
The 205A43A thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Therefore, it was confirmed that 95% or more of the 205A43A thrombin were bound.
Subsequently, the purification was carried out by using sulfated cellulofine column and hirudin C-terminal peptide column according to the method of the above-mentioned section (3) in Experimental Example 1, to thereby obtain about 3 mg of almost purified 205A43A thrombin on SDS-PAGE.
Fig. 38 shows the purification using the hirudin C-terminal peptide column. In the step of purifying the hirudin C-terminal peptide, no elution of thrombin was observed in a flow-through fraction (i.e., lane 4) and the purified 205A43A thrombin was collected and observed in a elution fraction.
Next, the binding ability to heparin gel was measured according to the method F and an eluate was obtained at A50%.

### (2) Measurement of substrate-cleaving activity of 205A43A thrombin

The thrombin substrate-cleaving activity of the 205A43A thrombin obtained in the above-mentioned section (1) was assayed according to the method A. As a result, no increase in absorption after incubation was observed.
A solution obtained by dissolving 50 µg of the 205A43A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 46 seconds while the APTT of the 205A43A thrombin was 78 seconds, which prolonged the APTT to 1.7 folds. The prolonging effect (i.e., APTT-prolonging effect) was 1.25 folds as compared with that of the anhydrothrombin under the same condition.

(3) Determination of PT: A solution obtained by dissolving 50 µg of the 205A43A thrombin in 1 ml of PBS was added to standard plasma (Sysmex International Reagents Co., Ltd.) in a volume ratio of 1:1, and PT was determined. A solution obtained by adding PBS to standard plasma (Sysmex International Reagents Co., Ltd.) in a volume ratio of 1:1 was used as a control, and PT was determined. The PT reagent available from Sigma-Aldrich as "thromboplastin with calcium" was used. As a result, the PT of the control was 24 seconds while the PT of the 205A43A thrombin was 25 seconds.

### (4) Determination of APTT of carboxyl group-modified 205A43A thrombin

1 mg/5 ml of 205A43G thrombin/50 mM phosphate buffer (pH 6.5)/0.5 M NaCl was subjected to dialysis against 0.25 M glycine ethyl ester/0.5 M NaCl (pH 6.5) at 4°C for 3 hours. After that, the resultant was added with 1-ethyl-3-(3-dimethylaminopropyl)-carbodiimide (Wako Pure Chemical Industries, Ltd.) to make the concentration thereof 20 mg/ml. The mixture was incubated at 25°C for 1 hour to modify carboxyl groups of the 205A43A thrombin. As a result of mass analysis of the modified product, about 16 carboxyl groups were modified.

A solution obtained by dissolving 50 µg of the modified 205A43A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 46 seconds while the APTT of the carboxyl group-modified 205A43A thrombin was 190 seconds.

### (5) Determination of prothrombin time (PT)

A solution obtained by dissolving 50 µg of the carboxyl group-modified 205A43A thrombin in 1 ml of PBS was added to standard plasma (Sysmex International Reagents Co., Ltd.) in a volume ratio of 1:1, and PT was determined. A solution obtained by adding PBS to standard plasma (Sysmex International Reagents Co., Ltd.) in a volume ratio of 1:1 was used as a control, and PT was determined. The PT reagent available from Sigma-Aldrich as "thromboplastin with calcium" was used. As a result, the PT of the control was 22 seconds while the PT of the carboxyl group-modified 205A43A thrombin was 23 seconds. Thus, even the modified product of the 205A43A thrombin exhibited no prolonging effects on PT.

### (6) Evaluation of antiplatelet effect of 205A43A thrombin by using PRP

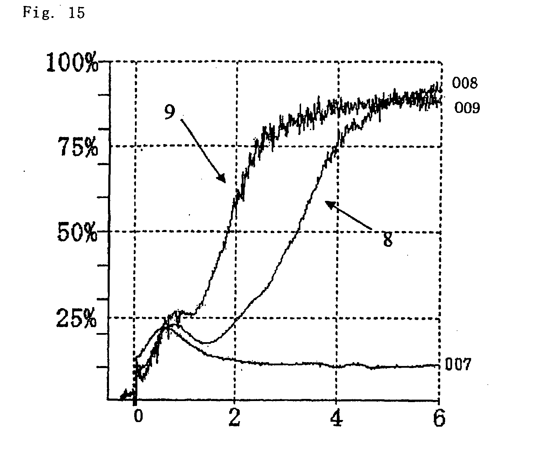

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP. 100 µl of PBS solution of the 205A43A thrombin, which had been adjusted so that a final concentration of the 205A43A thrombin was 30 µg/ml when 100 µl of the solution was added, was added to 130 µl of PRP. The mixture was added with 35 µl of 5 mg/ml ristocetin/PBS solution as an inducing substance. As a control, 130 µl of PRP was added with 100 µl of 5 mM phosphate buffer (pH 7.4)/0.15 M NaCl, and the time course of the transmittance was recorded.

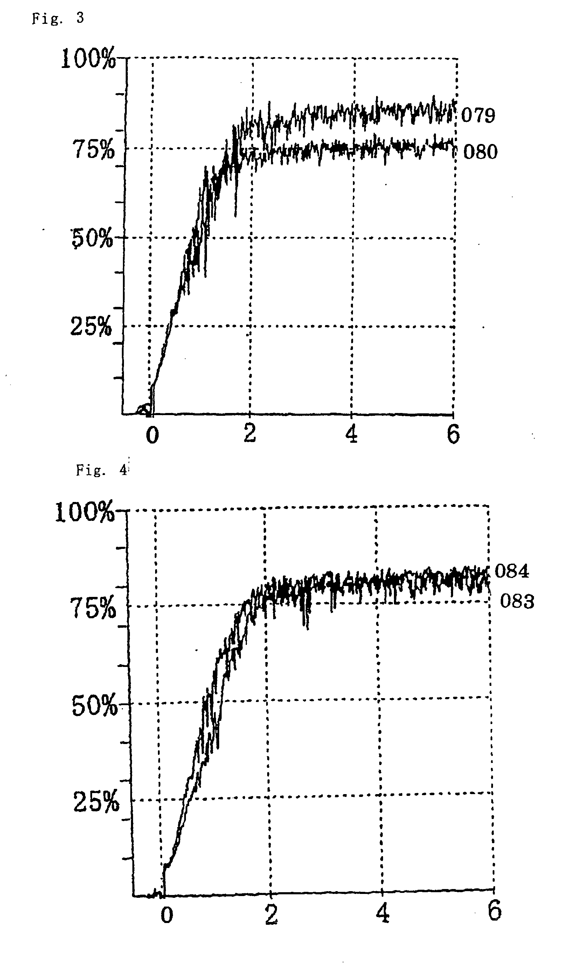

Fig. 3 shows the results.
Evaluation 2: The experiment was carried out according to the method in Evaluation (1) except that 1 µg/ml M-thrombin/PBS was used as an inducing substance. As a control, 130 µl of PRP was added with 100 µl of PBS and the time course of the transmittance was recorded. Fig. 4 shows the results.
Evaluation 3: A 1 µg/ml M-thrombin/PBS solution was used as an inducing substance, and solutions of the 205A43A thrombin, which had been adjusted so that final concentration of the 205A43A thrombin was 120 µg/ml and 30 µg/ml, respectively, when 100µl each of solutions was added, were added, respectively. The experiment was carried out according to the method in the Evaluation 1. Fig. 15 shows the results.
Evaluation 4: 100 µl of PBS solution of the 205A43A thrombin, which had been adjusted so that a final concentration of the 205A43A thrombin was 150 µg/ml, was added to 130 µl of PRP. The mixture was added with 35 µl of 5 mg/ml ristocetin/PBS serving as an inducing substance. As a control, 130 µl of PRP was added with 100 µl of PBS, and the time course of the transmittance was recorded. Even at a high concentration of the 205A43A thrombin, the inhibiting effect on ristocetin-induced platelet aggregation was not observed.

### (7) Evaluation of antiplatelet effect of carboxyl group-modified 205A43A thrombin by using PRP

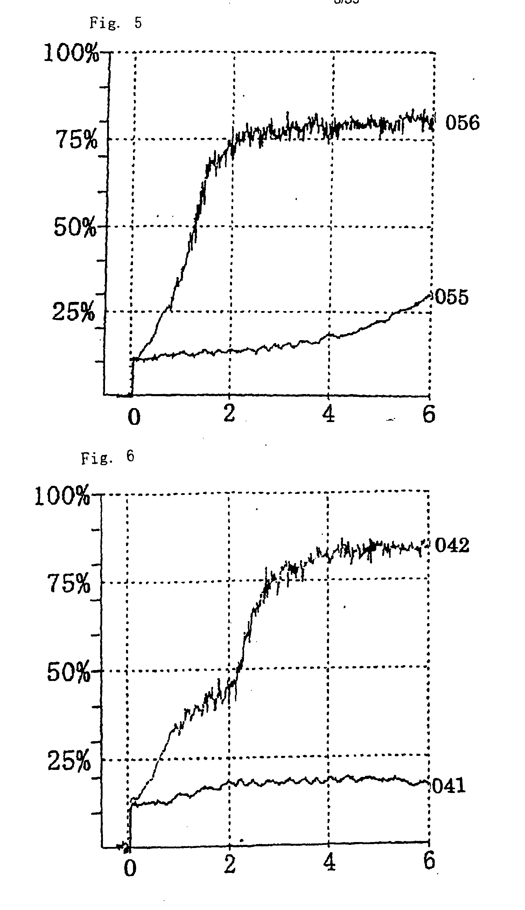

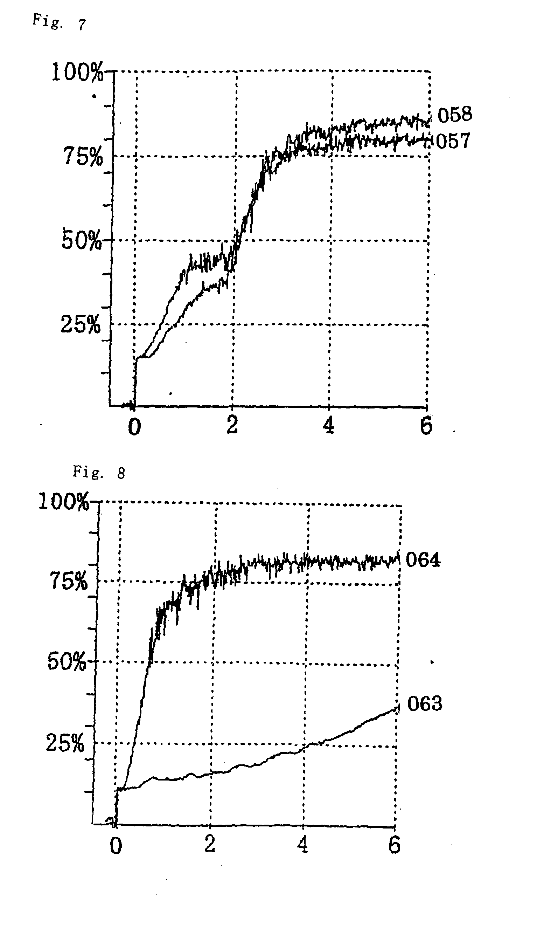

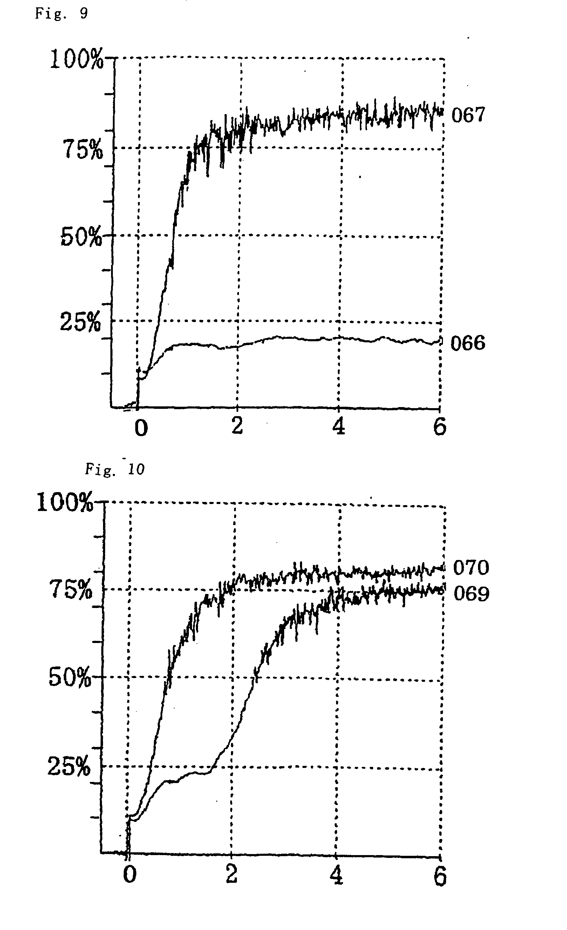

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,500 rpm for 10 minutes, to thereby obtain PPP. 100 µl of 5 mM phosphate buffer (pH 7.4)/0.15 M NaCl containing the carboxyl group-modified 205A43A thrombin, which had been adjusted so that final concentration of the carboxyl group-modified 205A43A thrombin was 30 µg/ml, 15 µg/ml, and 7.5 µg/ml, respectively, when 100 µl of each solution was added, were added to 130 µl of PRP. The mixture was each added with 35 µl of 5 mg/ml ristocetin/5 mM phosphate buffer (pH 7.4)/0.15 M NaCl serving as an inducing substance. As a control, 130 µl of PRP was added with 100 µl of 5 mM phosphate buffer (pH 7.4)/0.15 M NaCl, and the time course of the transmittance was recorded. The measurement of the transmittance was carried out by using EASY TRACER ET-800 (Tokyo Koden). Fig. 5 to Fig. 7 each show the results.
Evaluation 2: The experiment was carried out according to the method in Evaluation (1) except that 1 µg/ml M-thrombin/5 mM phosphate buffer (pH 7.4)/0.15 M NaCl was used as an inducing substance. As a control, 130 µl of PRP was added with 100 µl of 5 mM phosphate buffer (pH 7.4)/0.15 M NaCl, and the time course of the transmittance was recorded. The measurement of the transmittance was carried out by using EASY TRACER ET-800 (Tokyo Koden). Fig. 8 to Fig. 10 each show the results.

### (8) Experiment for comparing thrombin substrate-binding abilities by using IAsys

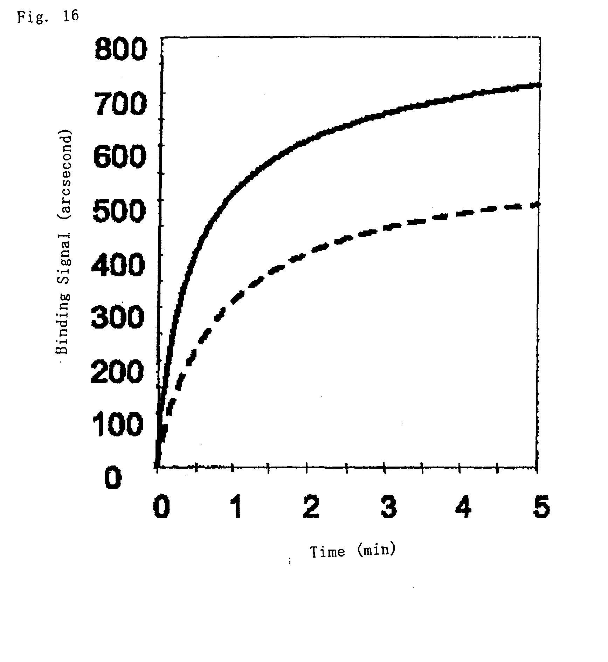

10 mM phosphate buffer (pH 7.4) containing 205A43A thrombin derivative or AHT each having a concentration of 0.1 mg/ml was added to an NHS-activated CM dextran cuvette (Nissei Sangyo Co., Ltd.), respectively. Each of the solutions was stirred at 25°C for 10 minutes to immobilize a test sample (i.e., thrombin derivative) on the NHS-activated CM dextran cuvette, to thereby obtain 205A43A thrombin- and AHT-immobilized cuvettes. 1,307 arc of the 205A43A thrombin was immobilized on the cuvette. Subsequently, 0.2 ml of 1 M ethanol amine (pH 8) was added thereto to perform a blocking treatment. The 205A43A cuvette and AHT cuvette were each added with 100 nM Fbgn and FVIII and respective binding curves were monitored. Fig. 16 and Fig. 14 each show the results. Fig. 14 showed that the AHT cuvette had almost the same amounts of Fbgn and FVIII bound thereto while Fig. 16 showed that the 205A43A cuvette had a lager amount of FVIII bound thereto than Fbgn.
It was confirmed that the ratio between the binding abilities to FVIII and Fbgn of the 205A43A thrombin derivative increased about 1.4 folds as compared with that of the AHT.
In addition, it was considered that the 205A43A thrombin derivative had an FVW-binding ability per immobilized proteins equivalent to that of the AHT.

### (9) Experiment on number of modifications and APTT-prolonging effect of 205A43A thrombin

1 mg/5 ml of 205A43A thrombin/50 mM phosphate buffer (pH 6.5)/0.5 M NaCl was subjected to dialysis against 0.25 M glycine ethyl ester/0.5 M NaCl (pH 6.5) at 4°C for 3 hours. After that, the resultant was added with 1-ethyl-3-(3-dimethylaminopropyl)-carbodiimide (Wako Pure Chemical Industries, Ltd.) to make the concentration thereof 20 mg/ml. The mixture was incubated at 25°C for 10 minutes, 60 minutes, and 120 minutes respectively to modify carboxyl groups. Glycine was added thereto at each reaction time so that the final concentration of the remaining carbodiimide became 0.5 M, thereby completing the reaction.
Carboxyl group modification reactions were confirmed that, with respect to unreacted 205A43A thrombin, the molecular weight of the sample which had been reacted for 10 minutes increased by about 480 Da (corresponding to 5.6 modifications), the molecular weight of the sample which had been reacted for 60 minutes increased by about 1,240 Da (corresponding to 14.6 modifications), and the molecular weight of the sample which had been reacted for 120 minutes increased by about 1,900 Da (corresponding to 22.4 modifications).

### APTT-prolonging effects of respective thrombin derivatives

The modified products were each adjusted to have concentrations of 50, 25, and 12.5 µg/ml, respectively, and each of the modified products was subjected to dialysis against PBS. Each sample was mixed with standard plasma at a volume ratio of 1:1, and APTT was determined. The following Table 2 shows the results of the determination. The APTT was determined twice for each sample to obtain an average value.

**[Table 2]**

| | APTT | | Average (second) | Ratio |
|---|---|---|---|---|
| | First time | Second time | | |
| Dialysate as control | 42 | 42 | 42 | 1 |
| 0 minute, sample concentration of 12.5µg/ml | 61 | 62 | 61.5 | 1.46 |
| 0 minute, sample concentration of 25µg/ml | 70 | 71 | 70.5 | 1.68 |
| 0 minute, sample concentration of 50µg/ml | 83 | 80 | 81.5 | 1.94 |
| 10 minutes, sample concentration of 12.5µg/ml | 87 | 89 | 88 | 2.1 |
| 10 minutes, sample concentration of 25µg/ml | 100 | 103 | 101.5 | 2.42 |
| 10 minutes, sample concentration of 50µg/ml | 118 | 114 | 116 | 2.76 |
| 60 minutes, sample concentration of 12.5µg/ml | 88 | 90 | 89 | 2.12 |
| 60 minutes, sample concentration of 25µg/ml | 110 | 112 | 111 | 2.64 |
| 60 minutes, sample concentration of 50µg/ml | 140 | 143 | 141.5 | 3.37 |
| 120 minutes, sample concentration of 12.5µg/ml | 88 | 90 | 89 | 2.12 |
| 120 minutes, sample concentration of 25µg/ml | 137 | 125 | 131 | 3.12 |
| 120 minutes, sample concentration of 50µg/ml | 240 | 250 | 245 | 5.83 |

From the above-mentioned results, it was confirmed that the APTT-prolonging effect of the 205A43A thrombin was enhanced even with 5.6 modifications as compared to the unmodified 205A43A thrombin. With increase in the number of modification from 14.6 to 22.4, the APTT-prolonging effect increased, but the yield thereof with 22.4 modifications decreased to about 50%. It was confirmed that from the perspective of the yield and APTT-prolonging effect, 5 or more modifications were effective for the APTT-prolonging effect due to modification, while 22 or more modifications decreased the yield.
It was revealed that the 205A43A thrombin had a higher FVIII specificity as compared to that of AHT which was obtained by simply converting the active center serine into dehydroalanine to minimize the structural change.

From the above-mentioned results, the 205A43A thrombin derivative had a strong APTT-prolonging effect regardless of the presence or absence of carboxyl group modification. Further, the APTT-prolonging effect was increased by modification of carboxyl groups. The platelet aggregation-inhibiting effect which depends on the M-thrombin-induced platelet aggregation inhibition (i.e., PAR1 activation inhibition) was present regardless of the presence or absence of the modification of carboxyl groups, but the effect was stronger after the modification of carboxyl groups as before. Thus, it was revealed that the 205A43A thrombin strongly affects on mainly the blood coagulation in which the APTT plays a central role and had a mild platelet aggregation-inhibiting effect, while the carboxyl group-modified 205A43A thrombin had a strong antiplatelet ability as well as the APTT. The modified 205A43A thrombin had a ristocetin-induced platelet aggregation-inhibiting effect in addition to the M-thrombin-induced platelet aggregation inhibition (i.e., PAR1 activation inhibition), but the unmodified 205A43A thrombin did not have the ristocetin-induced platelet aggregation even at a high concentration. Accordingly, a thrombin derivative which has the following characteristics and an antithrombotic effect was obtained. Carboxyl group-modified 205A43A thrombin:
APTT-prolonging effect (large), PAR1 inhibition (large), ristocetin-induced platelet aggregation (large) 205A43A thrombin:
APTT-prolonging effect (large), PAR1 inhibition (small), ristocetin-induced platelet aggregation (absent)
In addition, it was considered that the 205A43A thrombin had an extremely high APTT-prolonging effect in the experiment of comparing the thrombin substrate-binding abilities by using IAsys because the thrombin derivative, in which the serine at position 205 and the histidine at position 43 were concomitantly mutated, completely lost the substrate-cleaving activity and had an FVIII-binding ability higher than that of the AHT (that is, the binding ability to Fbgn relatively decreased). As described in Experimental Examples 16 and 17 shown hereinbelow, the thrombin derivative, in which the serine at position 205 and the histidine at position 43 were concomitantly mutated, had a high antithrombotic effect including as a main effect of the APTT extension even in a case of a combination of other amino acids.

### [Experimental Example 13]

### (1) Expression of thrombin (hereinafter, referred to as "77E203A205G thrombin") in which glycine at position 230 in B chain is substituted by alanine, serine at position 205 in B chain is substituted by glycine, and lysine at position 77 in B chain is substituted by glutamic acid

Synthesis was carried out by PCR using a mutation-introduced primer corresponding to the DNA of 77E203A205G thrombin. SEQ ID NO:27 shows the nucleotide sequence of the 77E203A205G thrombin.
The 77E203A205G thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 3 mg of almost purified 77E203A205G thrombin on SDS-PAGE.

### (2) Determination of APTT of blood added with 77E203A205G thrombin

A solution obtained by dissolving 50 µg of 77E203A205G thrombin in PBS was added to standard plasma (Sysmex International Reagents Co., Ltd.) in a volume ratio of 1:1, and APTT was determined. Similarly, a solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 44 seconds while the APTT of the 77E203A205G thrombin was 74 seconds, which showed that APTT was prolonged by 1.68 folds.
In Experimental Example 2, the extension of APTT of the 203A205G thrombin under the same condition was 1.09 folds. Thus, the substitution of glutamine for the lysine at position 77 increased the APTT-prolonging effect by 1.5 folds.

### (3) Evaluation of antiplatelet effect of 77E203A205G thrombin by using PRP

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP. 100 µl of PBS solution of the 77E203A205G thrombin, which had been adjusted so that a final concentration of the 77E203A205G thrombin was 70 µg/ml when 100 µl of the solution was added, was added to 130 µl of PRP. The mixture was added with 35 µl of 5 mg/ml ristocetin/PBS serving as an inducing substance. As a control, 130 µl of PRP was added with 100 µl of 5 mM phosphate buffer (pH 7.4)/0.15 M NaCl, and the time course of the transmittance was recorded. However, no ristocetin-induced platelet aggregation-inhibiting effect was observed.
Evaluation 2: The experiment was carried out according to the method in Evaluation (1) except that 1 µg/ml M-thrombin/PBS was used as an inducing substance. As a control, 130 µl of PRP was added with 100 µl of PBS and the time course of the transmittance was recorded. However, no M-thrombin-induced platelet aggregation-inhibiting effect was observed.
As described above, the 203A205G thrombin did not have a sufficient antithrombotic ability, but the 77E203A205G thrombin caused a significant increase in APTT-prolonging effect by decreasing the Fbgn-binding ability owing to the substitution of lysine at position 77. In addition, the 77E203A205G thrombin did not exhibit inhibiting effects on M-thrombin-and ristocetin-induced platelet aggregation.

### (4) APTT test of carboxyl group-modified product

Further, 1 mg/5 ml of 77E203A205G thrombin/0.5 M NaCl (pH 6.5) was subjected to dialysis against 0.25 M glycine ethyl ester/0.5 M NaCl (pH 6.5) at 4°C for 3 hours. After that, the resultant was added with 1-ethyl-3-(3-dimethylaminopropyl)-carbodiimide (Wako Pure Chemical Industries, Ltd.) to make the concentration thereof 20 mg/ml. The mixture was incubated at 25°C for 1 hour to modify carboxyl groups of the 77E203A205G thrombin. About 35% of carboxyl group-modified product was obtained in a solubilized state.
100 µl of solution obtained by dissolving 50 µg of carboxyl group-modified 77E203A205G thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 45 seconds while the APTT of the carboxyl group-modified 77E203A205G thrombin was 77.5 seconds.

### (5) Determination of prothrombin time (PT)

Solutions obtained by respectively dissolving 50 µg of 77E203A205G thrombin and carboxyl group-modified 77E203A205G thrombin in 1 ml of PBS were each added to standard plasma (Sysmex International Reagents Co., Ltd.) in a volume ratio of 1:1, and PT was determined. A solution obtained by adding PBS to standard plasma (Sysmex International Reagents Co., Ltd.) in a volume ratio of 1:1 was used as a control, and PT was determined. The PT reagent available from Sigma-Aldrich as "thromboplastin with calcium" was used. As a result, the PT of the control was 20 seconds while the PT of the 77E203A205G thrombin was 22 seconds and that of the carboxyl group-modified 77E203A205G thrombin was 23 seconds. Thus, both the 77E203A205G thrombin and the carboxyl group-modified 77E203A205G thrombin exhibited no prolonging effects.

### (6) Evaluation of antiplatelet effect of carboxyl group-modified 77E203A205G thrombin by using PRP

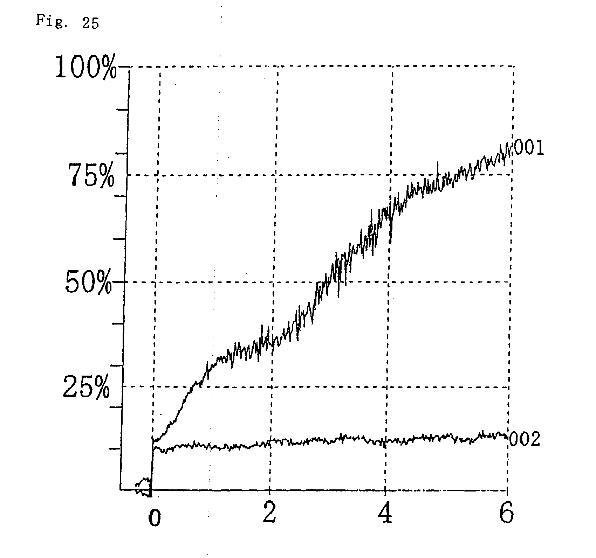

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP. 100 µl of PBS solution of the modified 77E203A205G thrombin, which had been adjusted so that a final concentration of the modified 77E203A205G thrombin was 50 µg/ml when 100 µl of the solution was added, was added to 130 µl of PRP. The mixture was added with 35 µl of 5 mg/ml ristocetin/PBS serving as an inducing substance. As a control, 130 µl of PRP was added with 100 µl of 5 mM phosphate buffer (pH 7.4)/0.15 M NaCl, and the time course of the transmittance was recorded. Fig. 25 shows the results.

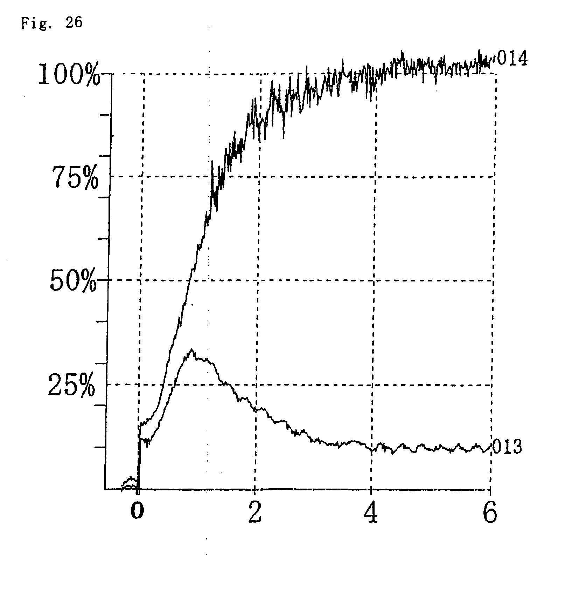

Evaluation 2: The experiment was carried out according to the method in Evaluation (1) except that 1 µg/ml M-thrombin/PBS was used as an inducing substance. As a control, 130 µl of PRP was added with 100 µl of PBS and the time course of the transmittance was recorded. Fig. 26 shows the results.
The above-mentioned results confirmed that the carboxyl group-modified 77E203A205G thrombin did not have an enhanced APTT-prolonging effect as compared to that before the modification of carboxyl groups, but exhibited inhibiting effects on M-thrombin-induced platelet aggregation and ristocetin-induced platelet aggregation. In addition, a significant decrease in yield due to the substitution of glutamic acid 77 as compared to that before the substitution was confirmed.

### (7) Confirmation of binding specificity of 77E203A205G thrombin to Fbgn and TM

0.1 mg/ml of 203A205 G thrombin/10 mM phosphate buffer (pH 7.7) and 0.1 mg/ml of 77E203A205G thrombin/10 mM phosphate buffer (pH 7.7) were added to an NHS-activated CM dextran cuvette (Nissei Sangyo Co., Ltd.), respectively. Each of the solutions was stirred at 25°C for 10 minutes to immobilize a test sample (i.e., thrombin derivative) on the NHS-activated CM dextran cuvette, to thereby obtain a 203A205G thrombin-immobilized cuvette and a 77E203A205G thrombin-immobilized cuvette. About 4,100 arc of the protein was immobilized on the 203A205G cuvette and about 2,000 arc of the protein was immobilized on the 77E203A205G cuvette. Subsequently, 0.2 ml of 1 M ethanol amine (pH 8) was added thereto to perform a blocking treatment.
Further, both cuvettes were washed with 50 mM phosphate buffer (pH 7.4) containing 2 M NaCl and 30 mM benzamidine, and regenerated. After that, both cuvettes were added with 100 µl of 500 nM Fbgn solution obtained by dissolving Fbgn in 50 mM phosphate buffer (pH 7.4)/0.15 M NaCl. Three minutes later, about 300 arc sec of Fbgn was adsorbed on the 203A205G thrombin-immobilized cuvette and about 80 arc sec of Fbgn was adsorbed on the 77E203A205G thrombin-immobilized cuvette.
Then, both cuvettes were washed in the same manner as described above and regenerated. After that, both cuvettes were added with 100 µl of 500 nM FVIII solution obtained by dissolving FVIII in 50 mM Tris-HCl (pH 7.4) cntaining 0.15 M NaCl and 10 mM CaCl₂. Three minutes later, about 300 arc sec of FVIII was adsorbed on the 203A205G thrombin-immobilized cuvette and about 300 arc sec of FVIII was adsorbed on the 77E203A205G thrombin-immobilized cuvette.
Then, both cuvettes were washed with 50 mM phosphate buffer (pH 7.4) containing 2 M NaCl and 30 mM benzamidine, and regenerated. After that, the 203A205G thrombin-immobilized cuvette and 77E203A205G thrombin-immobilized cuvette were each added with 100 µl of 50 nM soluble TM solution (Cosmo Bio Co., Ltd.) obtained by dissolving soluble TM in 50 mM phosphate buffer (pH 7.4)/0.15 M NaCl. Three minutes later, about 100 arc sec of TM was adsorbed on the 203A205G thrombin-immobilized cuvette and about 20 arc sec of TM was adsorbed on the 77E203A205G thrombin-immobilized cuvette.
The above-mentioned results revealed that the additional substitution of glutamic acid for the lysine at position 77 in the B chain of the 203A205G thrombin derivative causes the amount of adsorbed Fbgn to decrease to about one-third, and the amount of adsorbed TM to decrease to about one-half of the adsorption of blood coagulation factor 8 on a cuvette.
In addition, the results of the analysis by using IAsys FAST FIT PROGRAM (Nissei Sangyo Co., Ltd.) showed that the Fbgn binding constant was 10.8 nM and the FVIII binding ability was 5.07 nM with respect to 203A205G, and the Fbgn binding ability was 190 nM and the FVIII binding ability was 22.5 nM with respect to 77E203A205G.
It was considered that the 77E203A205G thrombin described in Experimental Example 13 has an APTT-prolonging ability higher than that of the 203A205G thrombin, owing to the improvement of the FVIII specificity to Fbgn caused by the substitution of lysine at position 77 in the B chain.

### [Experimental Example 14]

### (1) Expression of thrombin (hereinafter, referred to as "77E203A205A99N thrombin") in which glycine at position 203 in B chain is substituted by alanine, serine at position 205 in B chain is substituted by alanine, aspartic acid at position 99 in B chain is substituted by asparagine, and lysine at position 77 in B chain is substituted by glutamic acid

Synthesis was carried out by PCR using a mutation-introduced primer corresponding to the DNA of 77E203A205A99N thrombin. SEQ ID NO:29 shows the nucleotide sequence of the 77E203A205A99N thrombin.
The 77E203A205A99N thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 3 mg of almost purified 77E203A205A99N thrombin on SDS-PAGE.

### (2) Determination of APTT of blood added with 77E203A205A99N thrombin

A solution obtained by dissolving 50 µg of 77E203A205A99N thrombin was added to standard plasma (Sysmex International Reagents Co., Ltd.) in a volume ratio of 1:1, and APTT was determined. Similarly, a solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 41 seconds while the APTT of the 77E203A205A99N thrombin was 39 seconds. As described above, it was revealed that the 77E203A205A99N thrombin had a binding ability to hirudin gel but did not have a sufficient anti-APTT effect though it was provided with a mutation which sufficiently decreases the activity and the Fbgn-binding ability.

### [Experimental Example 15]

### Expression of thrombin (hereinafter, referred to as "203A thrombin") in which glycine at position 203 in B chain is substituted by alanine

Synthesis was carried out by PCR using a mutation-introduced primer corresponding to the DNA of 203A thrombin. SEQ ID NO:31 shows the nucleotide sequence of the 203A thrombin.
The 203A thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C terminal peptide column according to the method of the above-mentioned section (3) in Experimental Example 1, to thereby obtain about 2 mg of almost purified 203A thrombin on SDS-PAGE.
The thrombin substrate-cleaving activity of the 203A thrombin was measured according to the method B. Fig. 20 shows the results. The A chain of FXIII was observed to provide a cleaved product 30 minutes later, and more than half of the A chain was cleaved after three hours.
Further, the thrombin substrate-cleaving activity of the 203A thrombin was measured according to the method C. As a result, a clot was formed after three hours.
The above-mentioned results confirmed that the 203A thrombin, although being reported as a derivative having completely lost its activity in Non-patent Document 5, had an apparent thrombin substrate-cleaving activity when the 203A thrombin was used as an antithrombotic agent in the method of measuring the activity by using the thrombin substrate of the present invention, and thus it was difficult to use the 203A thrombin as an antithrombotic agent.

### [Experimental Example 16]

### (1) Expression of thrombin (hereinafter, referred to as "205G43A thrombin") in which serine at position 205 is substituted by glycine and histidine at position 43 is substituted by alanine

Synthesis was carried out by PCR using a mutation-introduced primer corresponding to the DNA of 205G43A thrombin. SEQ ID NO:33 shows the nucleotide sequence of a gene encoding the 205G43A thrombin.
The 205G43A thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C terminal peptide column according to the method of the above-mentioned section (3) in Experimental Example 1, to thereby obtain about 0.5 mg of almost purified 205G43A thrombin on SDS-PAGE.

### (2) Measurement of substrate-cleaving activity of 205G43A thrombin

The thrombin substrate-cleaving activity of the 205G43A thrombin obtained in the above-mentioned section (1) was measured according to the method A. As a result, no increase in absorption after incubation was observed.
Further, the thrombin substrate-cleaving activity of the 205G43A thrombin was measured according to the method B. As a result, no band of an A chain-activated product of FXIII was observed. The 205G43A thrombin was found to have a hirudin C-terminal peptide-binding ability and have sufficiently lost its activity. Solutions obtained by dissolving the 205G43A thrombin in an amount of 24 µg and 12 µg, respectively, to 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 43 seconds while the APTT of the 205G43A thrombin was 62 seconds and 55 seconds, which showed that APTT was prolonged by 1.44 folds and by 1.28 folds, respectively.
The APTT-prolonging effect of the sample added with 25 µg/ml AhT was 1.25 folds larger, and the APTT-prolonging effect of the sample added with 24 µg/ml 205G43A thrombin was 1.44 folds larger, so the 205G43A thrombin exhibited an APTT-prolonging effect 1.15 folds or more larger than that of AhT.
The above-mentioned results revealed that the 205G32A thrombin in which the serine and histidine in the active center were concomitantly substituted exhibited a high APTT-prolonging effect without modification of carboxyl groups even at a low concentration.

### [Experimental Example 17]

### (1) Expression of thrombin (hereinafter, referred to as "205A43S thrombin") in which serine at position 205 is substituted by alanine and histidine at position 43 is substituted by serine

Synthesis was carried out by PCR using a mutation-introduced primer corresponding to the DNA of 205A43S thrombin. SEQ ID NO:35 shows the nucleotide sequence of a gene encoding the 205A43S thrombin.
The 205A43S thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 3 mg of almost purified 205A43S thrombin on SDS-PAGE.

### (2) Assay of substrate-cleaving activity of 205A43S thrombin

The substrate-cleaving activity of the 205A43S thrombin obtained in the above-mentioned section (1) was assayed according to the method B. As a result, no band of A chain-activated product of FXIII was observed. It was revealed that the 205A43S thrombin had a hirudin C-terminal peptide-binding ability and had sufficiently lost its activity. Next, a solution obtained by dissolving 40 µg of the 205A43S thrombin in 1 ml of PBS was added to standard plasma (Sysmex International Reagents Co., Ltd.) in a volume ratio of 1:1, and APTT was determined. A solution obtained by adding PBS to standard plasma in a volume ratio of 1:1 was used as a control, and APTT was determined. The APTT reagent available from Sysmex International Reagents Co., Ltd. was used. As a result, the APTT of the control was 42 seconds while the APTT of the 203A43S thrombin was 72 seconds, which showed that APTT was prolonged by 1.71 folds. 50 µg/ml AhT showed an APTT-prolonging effect of 1.36 folds, so it showed that the 205A43S thrombin had an APTT-prolonging effect of at least 1.26 folds or more as compared to that of AhT. The above-mentioned results revealed that the 205A43S thrombin, in which the active center serine and histidine were concomitantly substituted, exhibited a high APTT-prolonging effect without modification of carboxyl groups.

### (3) Comparison of binding specificity of 205A43S thrombin to Fbgn and FVIII

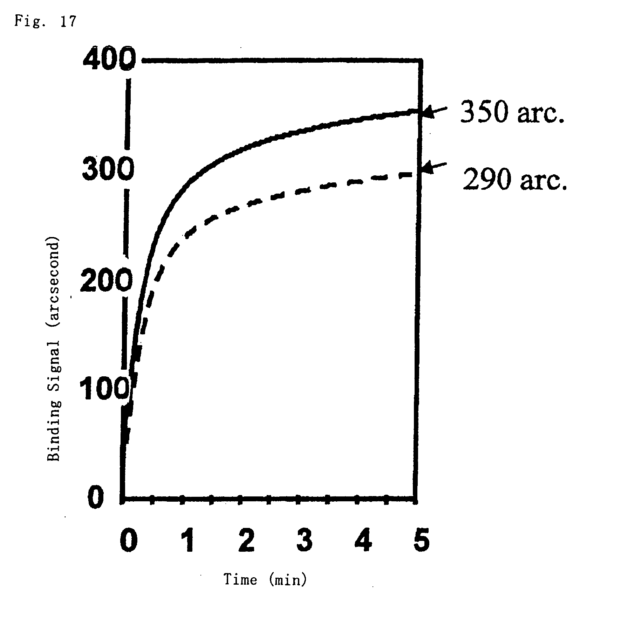

10 mM phosphate buffer (pH 7.4) containing 205A43S thrombin derivative and AHT each at a concentration of about 0.1 mg/ml were respectively added to NHS-activated CM dextran cuvette (Nissan Sangyo Co., Ltd.). Each of the mixtures was stirred at 25°C for 10 minutes to immobilize the test sample (i.e., thrombin derivative) on the NHS-activated CM dextran cuvette, to thereby obtain a 205A43S thrombin and an AHT-immobilized cuvette. About 1,200 arc of the 205A43S thrombin was immobilized on the cuvette. Subsequently, 0.2 ml of 1 M ethanol amine (pH 8) was added thereto to perform a blocking treatment.
The 205A43S cuvette and AHT cuvette were each added with 100 nM Fbgn and FVIII, to thereby monitor respective binding curves. Fig. 17 shows the results. Fig. 14 showed that about the same amounts of Fbgn and FVIII were bound in the AHT cuvette while Fig. 17 showed that more FVIII were bound in the 205A43S thrombin cuvette than Fbgn.
Further, the ratio of binding signals confirmed that the binding ratio between FVIII and Fbgn increased by about 1.2 folds as compared with that of AhT.
In addition, it was shown that at least the 205A43S thrombin had an FVIII-binding ability equivalent to that of AHT as compared to the immobilized protein. It was revealed that the 205A43S thrombin had a higher FVIII specificity as compared to that of AHT which was obtained by simply converting the active center serine into dehydroalanine to minimize the structural change.

### [Experimental Example 18]

### (1) Expression of thrombin (hereinafter, referred to as "77E205A43A thrombin") in which lysine at position 77 in B chain is substituted by glutamic acid, serine at position 205 is substituted by alanine, and histidine at position 43 is substituted by alanine

Synthesis was carried out by PCR using a mutation-introduced primer corresponding to the DNA of 77E205A43A thrombin. SEQ ID NO:37 shows the nucleotide sequence of a gene encoding the 77E205A43A thrombin.
The 77E205A43A thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine and hirudin C-terminal peptide columns according to the method of the above-mentioned section (3) in Experimental Example 1, to thereby obtain about 3 mg of almost purified 77E205A43A thrombin on SDS-PAGE.

(2) Solutions each obtained by dissolving 50 µg or 25 µg of 77E205A43A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 45 seconds while the APTT of the 77E205A43A thrombin were 92 seconds (50 µg) and 85 seconds (25 µg), respectively, which showed that APTT was extension by 2.04 folds and 1.89 folds. 50 µg/ml 205A43A thrombin in Example 12 showed an APTT-prolonging effect of 1.7 folds, so the APTT-prolonging effect increased by 1.2 folds as compared to that before the substitution, owing to the substitution of glutamic acid for lysine at position 77 in the B chain.

### (3) Evaluation of antiplatelet effect of 77E205A43A thrombin by using PRP

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP. 100 µl of PBS solution of the 77E205A43A thrombin, which had been adjusted so that a final concentration of the 77E205A43A thrombin was 100 µg/ml when 100 µl of the solution was added, was added to 130 µl of PRP. The mixture was added with 35 µl of 5 mg/ml ristocetin/PBS as an inducing substance. As a control, 130 µl of PRP was added with 100 µl of 5 mM phosphate buffer (pH 7.4)/0.15 M NaCl, and the time course of the transmittance was recorded. However, it showed an activation curve same as that of the control and thus showed no inhibiting effect.

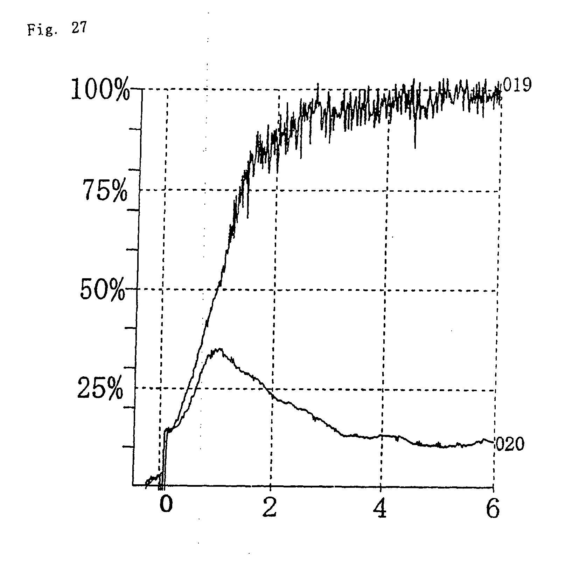

Evaluation 2: The experiment was carried out according to the method in Evaluation (1) except that 1 µg/ml M-thrombin/PBS solution was used as an inducing substance. As a control, 130 µl of PRP was added with 100 µl of PBS and the time course of the transmittance was recorded. Fig. 27 shows the results.
The above-mentioned results confirmed that the 77E205A43A thrombin had a specifically decreased Fbgn-binding ability due to the substitution of glutamic acid for lysine at position 77, and exhibited an APTT-inhibiting effect more stronger than that of the 205A43A thrombin which inherently had a strong APTT-inhibiting effect due to the enhancement of the FVIII-binding ability caused by the concomitant change of the serine at position 205 and histidine at position 43. Meanwhile, the 77E205A43A thrombin did not exhibit the ristocetin-induced platelet aggregation-inhibiting ability, but exhibited the M-thrombin-induced platelet aggregation-inhibiting effect.

### (4) APTT test of carboxyl group-modified product

Further, 1 mg/5 ml of 77E205A43A thrombin/0.5 M NaCl (pH 6.5) was subjected to dialysis against 0.25 M glycine ethyl ester/0.5 M NaCl (pH 6.5) at 4°C for 3 hours. After that, the resultant was added with 1-ethyl-3-(3-dimethylaminopropyl)-carbodiimide (Wako Pure Chemical Industries, Ltd.) to make the concentration thereof 20 mg/ml. The mixture was incubated at 25°C for 1 hour to modify carboxyl groups of the 77E205A43A thrombin. About 40% of modified product was obtained in a solubilized state.
100 µl of solutions each obtained by dissolving 50 µg or 25 µg of carboxyl group-modified 77E205A43A thrombin in 1 ml of PBS were added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 45 seconds while the APTT of the carboxyl group-modified 77E205A43A thrombin were 119 seconds (50 µg) and 89 seconds (25 µg), respectively.

### (5) Evaluation of antiplatelet effect of carboxyl group-modified 77E205A43A thrombin by using PRP

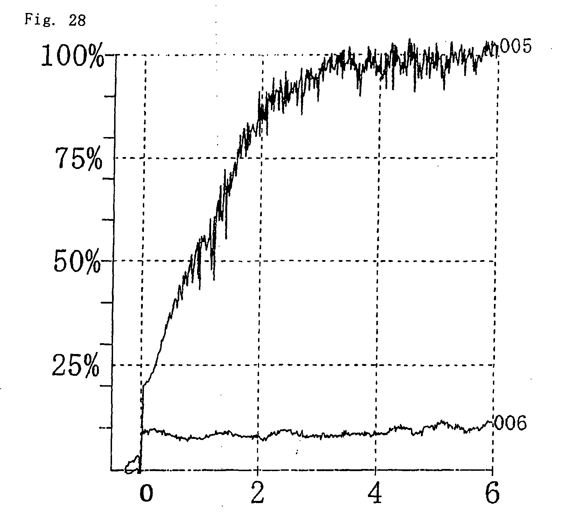

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP. 100 µl of PBS solution of the carboxyl group-modified 77E205A43A thrombin, which had been adjusted so that a final concentration of the modified 77E205A43A thrombin was 30 µg/ml when 100 µl of the solution was added, was added to 130 µl of PRP. The mixture was added with 35 µl of 5 mg/ml ristocetin/PBS solution as an inducing substance. As a control, 130 µl of PRP was added with 100 µl of 5 mM phosphate buffer (pH 7.4)/0.15 M NaCl, and the time course of the transmittance was recorded. Fig. 28 shows the results.

Evaluation 2: The experiment was carried out according to the method in Evaluation 1 except that 1 µg/ml M-thrombin/PBS was used as an inducing substance. As a control, 130 µl of PRP was added with 100 µl of PBS and the time course of the transmittance was recorded. As a result, the carboxyl group-modified 77E205A43A thrombin completely inhibited the M-thrombin-induced platelet aggregation at a final concentration of 30 µ/ml.
The above-mentioned results confirmed that the 77E205A43A thrombin had an increased APTT-prolonging effect due to the modification. Meanwhile, the 77E205A43A thrombin exhibited inhibiting effects on the M-thrombin-induced platelet aggregation and ristocetin-induced platelet aggregation. However, like the carboxyl group-modified product of the 77E203A205G thrombin, the yield of the 77E205A43A thrombin after the modification significantly decreased because of the substitution of glutamic acid at position 77 in the B chain, as compared to that of the modified product of the 205A43A thrombin.

### [Experimental Example 19]

### (1) Expression of thrombin (referred to as "24E205A43A thrombin") in which glutamine at position 24 in B chain is substituted by glutamic acid, serine at position 205 is substituted by alanine, and histidine at position 43 is substituted by alanine

Synthesis was carried out by PCR using a mutation-introduced primer corresponding to the DNA of 24E205A43A thrombin. SEQ ID NO:39 shows the nucleotide sequence of a gene encoding the 24E205A43A thrombin.
The 24E205A43A thrombin was expressed by the method of the above-mentioned section (1) in Experimental Example 1. The binding ability to a hirudin C-terminal peptide was confirmed according to the method of the above-mentioned section (2) in Experimental Example 1, and few bands were observed in a flow-through fraction and most of the bands of the thrombin were observed at the elution peak. Subsequently, the purification was carried out by using sulfated cellulofine column and hirudin C-terminal peptide column according to the method of the above-mentioned section (3) in Experimental Example 1. After that, the eluate containing a recombinant thrombin was subjected to dialysis against 50 mM NaHCO₃ (pH 8), and the resultant was applied to 5 ml of an HI TRAP benzamidine column (Pharmacia) which had been equilibrated with 50 mM NaHCO₃ (pH 8). The resultant was sufficiently washed with 50 mM NaHCO₃/0.3 M NaCl (pH 8) and subjected to elution with 50 mM NaHCO₃/0.3 M NaCl/0.1 M benzamidine (pH 8), to thereby obtain about 1 mg of almost purified 24E205A43A thrombin on SDS-PAGE.
A solution obtained by dissolving 50 µg of the 24E205A43A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 42 seconds while the APTT of the 24E205A43A thrombin was 62 seconds.

### (2) Comparison of binding specificity of 24E205A43A thrombin and AHT to Fbgn and FVIII

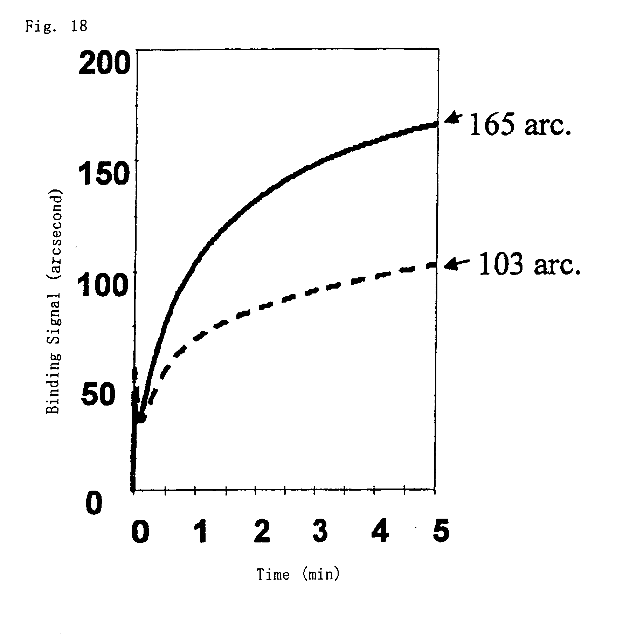

10 mM phosphate buffer (pH 7.4) containing the 24E205A43A thrombin or AHT at a concentration of about 0.1 mg/ml were added to NHS-activated CM dextran cuvette (Nissan Sangyo Co., Ltd.). Each of the cuvettes was stirred at 25°C for 10 minutes to immobilize the test sample (i.e., thrombin derivative) on the NHS-activated CM dextran cuvette, to thereby obtain a 24E205A43A thrombin-immobilized cuvette and an AHT-immobilized cuvette. (1,380 arc and 2,400 arc of them were immobilized, respectively.) Subsequently, 0.2 ml of 1 M ethanol amine (pH 8) was added thereto to perform a blocking treatment.
The 24E205A43A thrombin cuvette and AHT cuvette were each added with 100 nM of Fbgn and FVIII, to thereby monitor respective binding curves. Fig. 18 and Fig. 14 each show the results.
Fig. 18 confirmed that the 24E205A43A thrombin cuvette had more FVIII binding than Fbgn binding. It was confirmed that 24E205A43A had high FVIII specificity.

### (3) Comparison of TM-binding abilities of 205A43A thrombin and 24E205A43A thrombin

The 205A43A thrombin- and 24E205A43A thrombin-immobilized cuvettes were each added with 16.7 nM TM and then 50 nM TM.

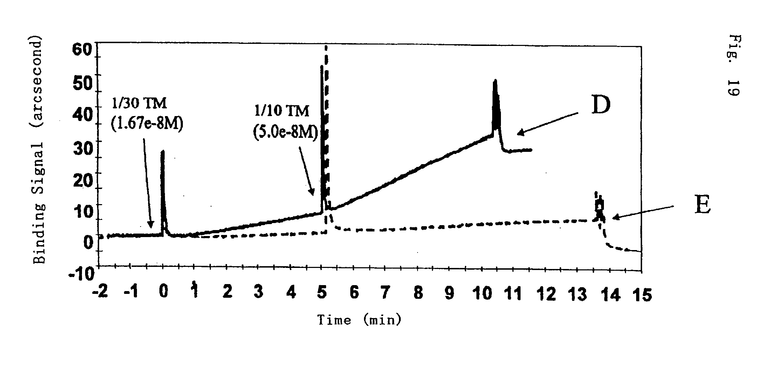

Fig. 19 shows the binding curves. About 980 arc of he 205A43A thrombin was immobilized and about 1,380 arc of the 24E205A43A thrombin was immobilized.
From the above-mentioned results, the 24E205A43A thrombin was a derivative having specificity to FVIII higher than that to Fbgn, and had an APTT-prolonging effect. Further, the 24E205A43A thrombin had a significantly decreased TM-binding ability. Therefore, it is considered that this thrombin mutant does not inhibit or block the activation of protein C caused by thrombin in a living body when the mutant has been administered to the living body.

### [Experimental Example 20]

### Expression of thrombin (referred to as "205A99A thrombin") in which serine at position 205 is substituted by alanine and aspartic acid at position 99 is substituted by alanine

Synthesis was carried out by PCR using a mutation-introduced primer corresponding to the DNA of 205A99A thrombin. SEQ ID NO:41 shows the nucleotide sequence of a gene encoding the 205A99A thrombin.
The 205A99A thrombin was expressed by the method of the above-mentioned section (1) in Experimental Example 1. Activation by ecarin was conducted by the method of the above-mentioned section (2) in Experimental Example 1, but no cleavage into A and B chains by ecarin occurred.
From the above-mentioned results, the 205A99A thrombin activated in a form of two chains was not able to be obtained in the experiment in which the 205A99A thrombin was expressed in a form of a prethrombin in a CHO cell.

### [Experimental Example 21]

### Comparison of APTT among carboxyl group-modified AHT, carboxyl group-modified 203A205G thrombin, and 205A43A thrombin

50 µg/ml each of the carboxyl group-modified AHT, carboxyl group-modified 203A205G thrombin, and 205A43A thrombin each dissolved in PBS were subjected to determination of APTT by the following two methods. PBS was added alone as a control and APTT was determined by the following methods "a" and "b". In order to prevent the influence of remaining activity as much as possible, the carboxyl group-modified AHT was added with 2 mg/ml of an APMSF reagent (Sigma-Aldrich) again after synthesis to inactivate the carboxyl group-modified AHT, and the resultant was then sufficiently subjected to dialysis against PBS to remove the remaining APMSF reagent.

Method "a": 100 µl of standard plasma was added with 50 µl of an APTT reagent, and the whole was incubated at 37°C for 5 minutes. The solution was added with 100 µl each of respective samples, and immediately followed by addition of 12 µl of 0.1 M CaCl₂. The respective time periods from the addition of calcium to coagulation were determined.
Method "b": 100 µl of standard plasma was added with 100 µl each of respective samples dissolved in PBS and 50 µl of an APTT reagent. The solutions were each incubated at 37°C for 5 minutes. After that, each of the solutions was added with 22 µl of 0.1 M CaCl₂, and the respective time periods from the addition of calcium to coagulation were determined.

The results were as shown below.

| | | |
|---|---|---|
| Control | Method "a" 43 seconds; | Method "b" 45 seconds |
| Carboxyl group-modified AHT | Method "a" 105 seconds; | Method "b" 66 seconds |
| Carboxyl group-modified 203A205G thrombin | Method "a" 75 seconds; | Method "b" 80 seconds |
| 205A43A thrombin | Method "a" 67 seconds; | Method "b" 77 seconds |
| Carboxyl group-modified 205A43A thrombin | Method "a" 145 seconds; | Method "b" 170 seconds |

As described above, the APTT of the carboxyl group-modified AHT was increased well by the method "a" while the APTT of the carboxyl group-modified 203A205G thrombin and 205A43A thrombin were increased well by the method "b". Meanwhile, the carboxyl group-modified 203A205G thrombin had an APTT-prolonging effect higher than that of the 205A43A thrombin under the same condition.
The method "a" involves no incubation time for the mixture of standard plasma and respective thrombin derivative, while the method "b" involves incubation of the mixture of standard plasma and respective thrombin derivative at 37°C for 5 minutes. The reason that the APTT-prolonging effect of the carboxyl group-modified AHT was decreased by the method "b" was because the presence of thrombin, which exists in an extremely small amount in the AHT which is chemically synthesized from thrombin, could not be ignored, and an extremely small amount of thrombin was incubated with standard plasma at 37°C, leading to activation of a small amount of a blood coagulation factor, especially FVIII, thus inhibiting the APTT-prolonging effect. Meanwhile, the carboxyl group-modified 203A205G thrombin and 205A43A thrombin were a thrombin derivative which had completely lost its activity by the gene recombination technique. Therefore, the activation of the blood coagulation factor did not occur even when the incubation was carried out with standard plasma. Rather, the APTT was significantly prolonged when the incubation was carried out because they were equilibrated and the substrate specificity was well reflected.
In view of the above discussion, the thrombin derivative and a carboxyl group-modified thrombin derivative thereof, which are used as antithrombotic agents, are completely inactivated and have uniform functions in terms of gene engineering as compared to the case of using the AHT which may have been contaminated with an extremely small amount of thrombin. Accordingly, the thrombin derivative and carboxyl group-modified thrombin derivative thereof of the present invention are considered to exhibit a stable and high drug efficacy.

### [Experimental Example 22]

### (1) Expression of thrombin (hereinafter, referred to as "77A205A43A thrombin") in which lysine at position 77 in B chain is substituted by alanine, serine at position 205 is substituted by alanine, and histidine at position 43 is substituted by alanine

Synthesis was carried out by PCR using a mutation-introduced primer corresponding to the DNA of 77A205A43A thrombin. SEQ ID NO:43 shows the nucleotide sequence of a gene encoding the 77A205A43A thrombin.
The 77A205A43A thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 3 mg of almost purified 77A205A43A thrombin on SDS-PAGE.
Next, the binding ability to heparin gel was measured according to the method F, and an eluate was obtained at the same elution position (i.e., A50%) as that of the wild-type human thrombin. It was confirmed that the binding ability to heparin was maintained even when the substitutions of amino acids at the active center and on the exosite I were performed.

(2) Solutions obtained by dissolving 50 µg and 25 µg of 77A205A43A thrombin in 1 ml of PBS, respectively, were each added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 45 seconds while the APTT of 77A205A43A thrombin were 90.5 seconds and 85 seconds, respectively, which showed that APTT were prolonged by 2.01 folds and 1.89 folds, respectively. The 205A43A thrombin at a concentration of 50 µg/ml in Experimental Example 12 had an APTT-prolonging effect of 1.7 folds, thus the substitution of alanine for lysine at position 77 in the B chain enhanced the APTT-prolonging effect to 1.2 folds.

### (3) APTT test of carboxyl group-modified product

Further, 1 mg/5 ml of 77A205A43A thrombin/0.5 M NaCl (pH 6.5) was subjected to dialysis against 0.25 M glycine ethyl ester/0.5 M NaCl (pH 6.5) at 4°C for 3 hours. After that, the resultant was added with 1-ethyl-3-(3-dimethylaminopropyl)-carbodiimide (Wako Pure Chemical Industries, Ltd.) to make the concentration thereof 20 mg/ml. The mixture was incubated at 25°C for 1 hour to modify carboxyl groups of the 77A205A43A thrombin. About 85% of modified products were obtained in a solubilized state.
100 µl of solutions each obtained by dissolving 50 µg or 25 µg of carboxyl group-modified 77A205A43A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 45 seconds while the APTT of the carboxyl group-modified 77A205A43A thrombin were 144 seconds and 109 seconds, respectively.

### (4) Determination of prothrombin time (PT)

Solutions obtained by dissolving 50 µg each of the 77A205A43A thrombin and the carboxyl group-modified 77A205A43A thrombin in 1 ml of PBS were each added to standard plasma (Sysmex International Reagents Co., Ltd.) in a volume ratio of 1:1, and PT was determined. A solution obtained by adding PBS to standard plasma (Sysmex International Reagents Co., Ltd.) was used as a control, and PT was determined. The PT reagent available from Sigma-Aldrich as "thromboplastin with calcium" was used. As a result, the PT of the control was 23 seconds while the PT of the 77A205A43A thrombin was 24 seconds and the PT of the carboxyl group-modified 77A205A43A thrombin was 25 seconds, both of which exhibiting no PT-prolonging effect. From the above results, the substitution of alanine for lysine at position 77 in the B chain increased the APTT-prolonging effect as compared to that of before the substitution. In addition, the substitution of alanine for lysine at position 77 enhanced the APTT-prolonging effect also in a modified product. Further, a significant decrease in yield was not observed in a case of modification as compared to a case where glutamic acid was substituted at position 77 in the B chain, so it was shown that alanine was more suitable for the modification at position 77 in the B chain.

### [Experimental Example 23]

### (1) Expression of thrombin (hereinafter, referred to as "65A205A43A thrombin") in which lysine at position 65 in B chain is substituted by alanine, serine at position 205 is substituted by alanine, and histidine at position 43 is substituted by alanine

Synthesis was carried out by PCR using a mutation-introduced primer corresponding to the DNA of 65A205A43A thrombin. SEQ ID NO:45 shows the nucleotide sequence of a gene encoding the 65A205A43A thrombin.
The 65A205A43A thrombin was expressed by the method of the above-mentioned section (1) in Experimental Example 1. Subsequently, the purification was carried out by using sulfated cellulofine column and hirudin C-terminal peptide column according to the method of the above-mentioned section (3) in Experimental Example 1, to thereby obtain about 3 mg of almost purified 65A205A43A thrombin on SDS-PAGE.

(2) Solutions obtained by dissolving 50 µg and 25 µg of 65A205A43A thrombin in 1 ml of PBS, respectively, were each added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 42 seconds while the APTT of 65A205A43A thrombin were 93 seconds and 86 seconds, which were prolonging effects of 2.21 folds and 2.05 folds, respectively. The 205A43A thrombin at a concentration of 50 µg/ml in Experimental Example 12 had an APTT-prolonging effect of 1.7 folds, thus the substitution of alanine for lysine at position 65 enhanced the APTT-prolonging effect to 1.3 folds.

### (3) APTT test of carboxyl group-modified product

Further, 1 mg/5 ml of 65A205A43A thrombin/0.5 M NaCl (pH 6.5) was subjected to dialysis against 0.25 M glycine ethyl ester/0.5 M NaCl (pH 6.5) at 4°C for 3 hours. After that, the resultant was added with 1-ethyl-3-(3-dimethylaminopropyl)-carbodiimide (Wako Pure Chemical Industries, Ltd.) to make the concentration thereof 20 mg/ml. The mixture was incubated at 25°C for 1 hour to modify carboxyl groups of the 65A205A43A thrombin.
100 µl of solution obtained by dissolving 50 µg or 25 µg of carboxyl group-modified 65A205A43A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 41 seconds while the APTT of the carboxyl group-modified 65A205A43A thrombin were 118 seconds and 88 seconds, respectively.

### (4) Determination of prothrombin time (PT)

Solutions each obtained by dissolving 50 µg of 65A205A43A thrombin or carboxyl group-modified 65A205A43A thrombin in 1 ml of PBS were each added to standard plasma (Sysmex International Reagents Co., Ltd.) in a volume ratio of 1:1, and PT was determined. A solution obtained by adding PBS to standard plasma (Sysmex International Reagents Co., Ltd.) in a volume ratio of 1:1 was used as a control, and PT was determined. The PT reagent available from Sigma-Aldrich as "thromboplastin with calcium" was used. As a result, the PT of the control was 23 seconds while the PT of the 65A205A43A thrombin was 22 seconds and that of the carboxyl group-modified 65A205A43A thrombin was 25 seconds, thus both of them exhibited almost no prolonging effects.

### (5) Evaluation of antiplatelet effect of 65A205A43A thrombin by using PRP

Evaluation 1: Immediately after blood collection, 10 ml of the whole blood added with citric acid was subjected to centrifugation at 800 rpm for 15 minutes, to thereby obtain 2 ml of PRP from a supernatant. The blood was further subjected to centrifugation at 2,800 rpm for 10 minutes, to thereby obtain PPP. 100 µl of PBS solution of the 65A205A43A thrombin, which had been adjusted so that a final concentration of the 65A205A43A thrombin was 100 µg/ml when 100 µl of the solution was added, was added to 130 µl of PRP. The mixture was added with 35 µl of 5 mg/ml ristocetin/PBS as an inducing substance. As a control, 130 µl of PRP was added with 100 µl of 5 mM phosphate buffer (pH 7.4)/0.15 M NaCl, and the experiment on platelet aggregation inhibition was carried out. There was no difference between the control and the 65A205A43A thrombin.

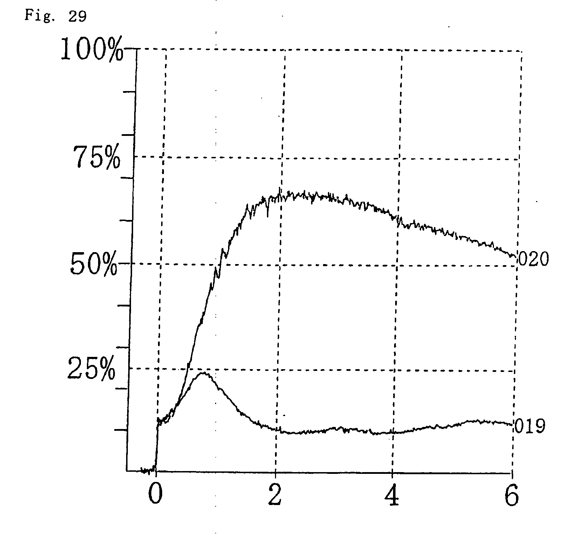

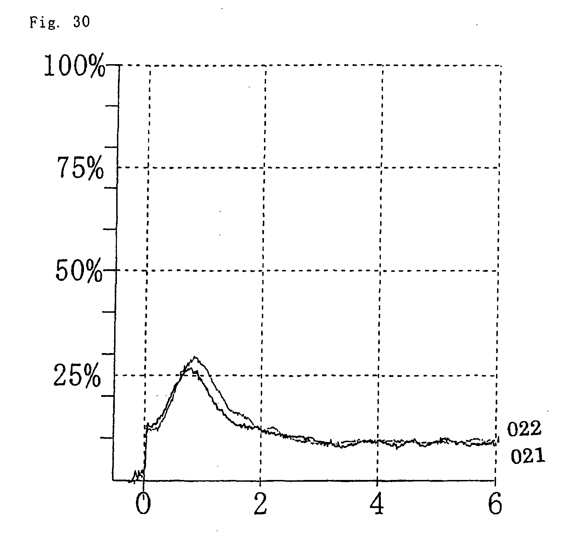

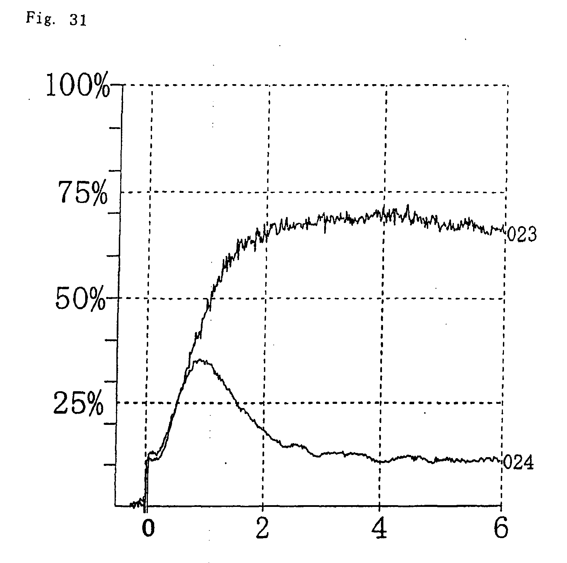

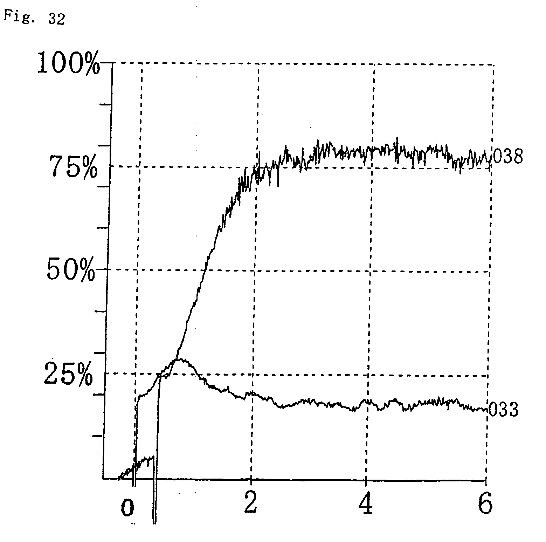

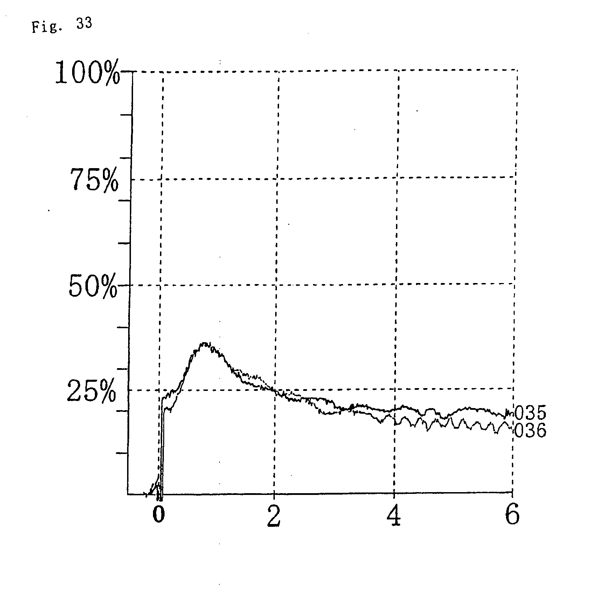

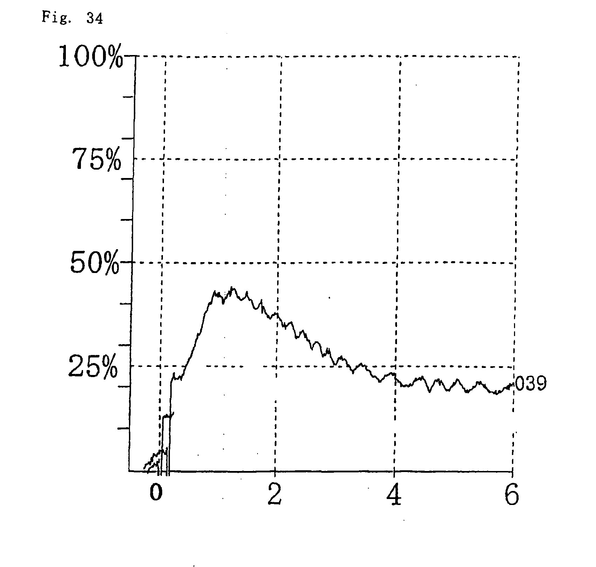

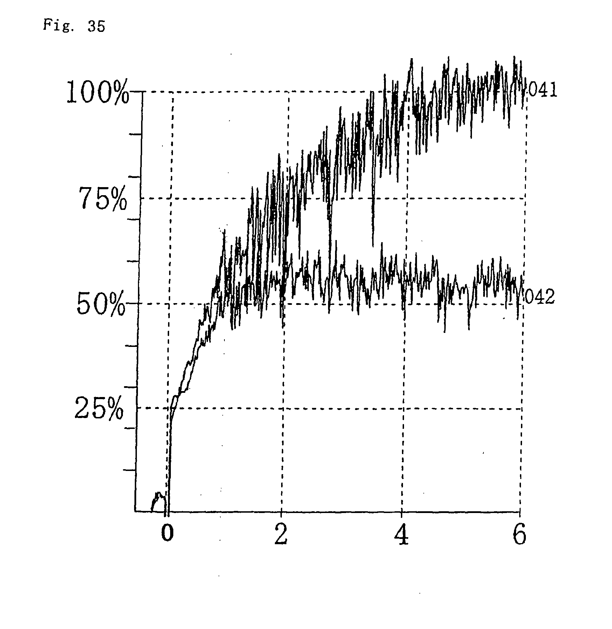

Evaluation 2: The experiment was carried out according to the method in Evaluation (1) except that 1 µg/ml M-thrombin/PBS was used as an inducing substance and the 65A205A43A thrombin was added so as to have final concentration of 100, 50, 25, and 10 µg/ml, respectively. As a control, 130 µl of PRP was added with 100 µl of PBS and the time course of the transmittance was recorded. Fig. 29 to Fig. 31 each show the results.
Evaluation 3: A 1 µg/ml M-thrombin/PBS as an inducing substance was added with respective PBS solutions of modified 65A205A43A thrombin, which had been adjusted so that final concentrations of the 65A205A43A thrombin were 36, 18, 9, and 4.5 µg/ml, respectively, when 100 µl of the solution was added. The experiment was carried out according to the method in Evaluation 1. Fig. 32, Fig. 33, and Fig. 34 each show the results.
Evaluation 4: 100 µl each of PBS solution of the modified 65A205A43A thrombin, which had been adjusted so that a final concentration of the modified 65A205A43A thrombin was 36 µg/ml, was added to 130 µl of PRP. The mixture was added with 35 µl of 5 mg/ml ristocetin/PBS as an inducing substance. As a control, 130 µl of PRP was added with 100 µl of PBS and the time course of the transmittance was recorded. Fig. 35 shows the results.

From the above-mentioned results, the 65A205A43A thrombin had a higher APTT than that of the 205A43A thrombin and exhibited a strong M-thrombin-induced platelet aggregation-inhibiting effect at a lower concentration as compared to the 205A43A thrombin. In addition, the modified 65A205A43A thrombin exhibited a ristocetin-induced platelet aggregation inhibition effect and at the same time, exhibited a much higher level of a thrombin receptor activity-inhibiting effect than that of the other derivatives.

### (6) Comparison of binding specificity of 65A205A43A thrombin and 205A43A thrombin to Fbgn and FVIII

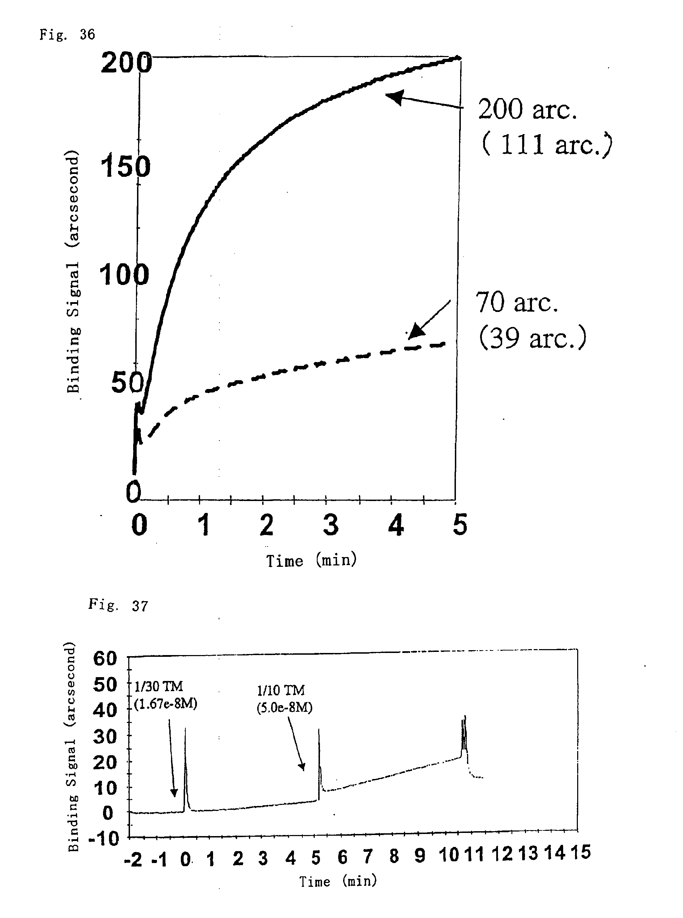

10 mM phosphate buffer (pH 7.7) containing the 65A205A43A thrombin or the 205A43A thrombin each at a concentration of about 0.1 mg/ml was added to an NHS-activated CM dextran cuvette (Nissei Sangyo Co., Ltd.), respectively. Each of the solutions was stirred at 25°C for 10 minutes to immobilize a test sample (thrombin derivative) on the NHS-activated CM dextran cuvette, to thereby obtain a 65A205A43A thrombin-immobilized cuvette (about 1,800 arc was bound). Subsequently, 0.2 ml of 1 M ethanol amine (pH 8) was added thereto to perform a blocking treatment. The 65A205A43A cuvette was added with 100 nM of Fbgn and FVIII and respective binding curves were monitored. Fig. 36 shows the results. Fig. 36 showed that in the 65A205A43A cuvette, the Fbgn-binding ability decreased and thus the specificity to FVIII increased as compared to those of the 205A43A thrombin.
It was confirmed that the ratio between FVIII-binding ability and Fbgn-binding ability increased 2.8 folds than those before the substitution of lysine at position 65 in the B chain.

### (7) Comparison of TM-binding abilities of 205A43A thrombin and 65A205A43A thrombin

The 65A205A43A thrombin-immobilized cuvette was added with 16.7 nM TM and then 50 nM TM.
Fig. 37 shows the binding curves. About 1,800 arc of the 65A205A43A thrombin was immobilized.
From the above-mentioned results, when compared to the TM-binding ability of the 205A43A thrombin described in Fig.19, the 65A205A43A thrombin had a TM-binding ability decreased to about 30% of that before the substitution of lysine at position 65 in the B chain in terms of a relative ratio of the amount of the immobilized protein. Because the TM-binding ability decreased, it was considered that the 65A205A43A thrombin mutant inhibits or blocks the activation of protein C caused by thrombin in a living body when the mutant has been administered to the living body.

### [Experimental Example 24]

### (1) Expression of thrombin (hereinafter, referred to as "205G thrombin") in which serine at position 205 in B chain is substituted by glycine

Synthesis was carried out by PCR using a mutation-introduced primer corresponding to the DNA of 205G thrombin.
The 205G thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C terminal peptide column according to the method of the above-mentioned section (3) in Experimental Example 1, to thereby obtain about 5 mg of almost purified 205T thrombin on SDS-PAGE.

### (2) Measurement of substrate-cleaving activity of 205G thrombin

The thrombin substrate-cleaving activity of thrombin was measured according to the above-mentioned method B, and the results are shown in Fig. 42. The lane 7 shows a sample obtained by a three-hour incubation. A band of FXIII which had been cleaved by about 50% was detected. As described above, the 205G thrombin still had an activity which causes problems when it is used as an antithrombotic agent.

### [Experimental Example 25]

### (1) Expression of thrombin (hereinafter, referred to as "197A205A43A thrombin") in which arginine at position 197 in B chain is substituted by alanine, serine at position 205 is substituted by alanine, and histidine at position 43 is substituted by alanine

Synthesis was carried out by PCR using a mutation-introduced primer corresponding to the DNA of 197A205A43A thrombin. SEQ ID NO:47 shows the nucleotide sequence of a gene encoding the 197A205A43A thrombin.
The 197A205A43A thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 3 mg of almost purified 77A205A43A thrombin on SDS-PAGE.

(2) Solutions each obtained by dissolving 50 µg or 25 µg of 197A205A43Athrombin in 1 ml of PBS were each added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 43.5 seconds while the APTT of the 197A205A43A thrombin were 77.5 seconds and 65 seconds, respectively.
From the above-mentioned results, the 197A205A43A thrombin exhibited a slightly stronger APTT-prolonging effect than that before the substitution of arginine at position 197. In addition, it is considered that the substitution of arginine at position 197 eliminates the cell growth activity due to the RGDA sequence.

### [Experimental Example 26]

### (1) Expression of thrombin (hereinafter, referred to as "77S205A43A thrombin") in which lysine at position 77 in B chain is substituted by serine, serine at position 205 is substituted by alanine, and histidine at position 43 is substituted by alanine

Synthesis was carried out by PCR using a mutation-introduced primer corresponding to the DNA of 77S205A43A thrombin. SEQ ID NO:49 shows the nucleotide sequence of a gene encoding the 77S205A43A thrombin.
The 77S205A43A thrombin was expressed by the method of the above-mentioned section (1) in Experimental Example 1. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain 2.5 mg of almost purified 77S205A43A thrombin on SDS-PAGE.

(2) A solution obtained by dissolving 50 µg of the 77S205A43A thrombin in 1 ml of PBS was added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 48 seconds while the APTT of the 77S205A43A thrombin was 86 seconds.

### [Experimental Example 27]

### (1) Expression of thrombin (65A77A205A43A thrombin) in which lysine at position 65 in B chain is substituted by alanine, lysine at position 77 in B chain is substituted by alanine, serine at position 205 is substituted by alanine, and histidine at position 43 is substituted by alanine

The 65A77A205A43A thrombin was expressed by the method of the above-mentioned section (1) in Experimental Example 1.
SEQ. ID. NO:51 shows the nucleotide sequence of the gene encoding the 65A77A205A43A thrombin. Subsequently, the purification was carried out by using sulfated cellulofine column and hirudin C-terminal peptide column according to the method of the above-mentioned section (3) in Experimental Example 1, to thereby obtain 3 mg of almost purified 65A77A205A43A thrombin on SDS-PAGE.

(2) Solutions each obtained by dissolving 50 µg, 25 µg, or 12.5 µg of the 65A77A205A43A thrombin in 1 ml of PBS were added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 49 seconds while the APTT of the 65A77A205A43A thrombin were 145.5 seconds, 112.5 seconds, and 86.5 seconds, respectively, which showed that APTT was prolonged by 2.3 folds and 1.77 folds, respectively. 50 µg/ml 205A43A thrombin in Example 12 showed an APTT-prolonging effect of 1.7 folds, so the APTT-prolonging effect increased to 1.35 folds owing to the substitution of alanine for lysine at position 65 and alanine for lysine at position 77 in the B chain.
It was confirmed that the 65A77A205A43A thrombin had an extremely high APTT-prolonging effect due to a synergistic effect of the substitutions of amino acids at positions 65 and 77 in the B chain.

### [Experimental Example 28]

### (1) Expression of thrombin (65A245A205A43A thrombin) in which lysine at position 65 in B chain is substituted by alanine, arginine at position 245 in B chain is substituted by alanine, serine at position 205 is substituted by alanine, and histidine at position 43 is substituted by alanine

The 65A245A205A43A thrombin was expressed by the method of the above-mentioned section (1) in Experimental Example 1.
SEQ. ID. NO:53 shows the nucleotide sequence of the gene encoding the 65A245A205A43A thrombin. Subsequently, the purification was carried out by using sulfated cellulofine column and hirudin C-terminal peptide column according to the method of the above-mentioned section (3) in Experimental Example 1, to thereby obtain 2 mg of almost purified 65A245A205A43A thrombin on SDS-PAGE.
Subsequently, the binding ability to heparin was measured according to the method F and an eluate was obtained at A35%. The affinity to heparin decreased to 35% of that of the human wild-type thrombin. It is considered that the decrease in the affinity to heparin is due to the substitution of an amino acid, i.e., arginine at position 245, which exists on the exosite II that is the heparin-binding region.

(2) Solutions each obtained by dissolving 50 µg or 25 µg of the 65A245A205A43A thrombin in 1 ml of PBS were each added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 41 seconds while the APTT of the 65A245A205A43A thrombin were 65 seconds and 60 seconds, respectively.
The above-mentioned results confirmed that the 65A245A205A43A thrombin had an APTT-prolonging effect and had decreased affinity to heparin as compared to that of the human wild-type thrombin due to the substitution of amino acids on the exosite II.

### [Experimental Example 29]

### (1) Expression of thrombin (65A248A205A43A thrombin) in which lysine at position 65 in B chain is substituted by alanine, lysine at position 248 in B chain is substituted by alanine, serine at position 205 is substituted by alanine, and histidine at position 43 is substituted by alanine

The 65A248A205A43A thrombin was expressed by the method of the above-mentioned section (1) in Experimental Example 1.
SEQ. ID. NO:55 shows the nucleotide sequence of the gene encoding the 65A248A205A43A thrombin. Subsequently, the purification was carried out by using sulfated cellulofine column and hirudin C-terminal peptide column according to the method of the above-mentioned section (3) in Experimental Example 1, to thereby obtain 2 mg of almost purified 65A248A205A43A thrombin on SDS-PAGE.
Subsequently, the binding ability to heparin was measured according to the method F and an eluate was obtained at A35%.
The affinity to heparin decreased to 35% of that of the human wild-type thrombin. It is considered that the decrease in the affinity to heparin is due to the substitution of an amino acid, i.e., lysine at position 248, which exists on the exosite II that is the heparin-binding region.

(2) Solutions each obtained by dissolving 50 µg or 25 µg of the 65A248A205A43A thrombin in 1 ml of PBS were each added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 45 seconds while the APTT of the 65A248A205A43A thrombin were 83 seconds and 71 seconds, respectively.
The above-mentioned results confirmed that, like the 65A245A205A43A thrombin, the 65A248A205A43A thrombin had an APTT-prolonging effect and had decreased affinity to heparin as compared to that of the human wild-type thrombin due to the substitution of amino acids on the exosite II.

### [Experimental Example 30]

Immediately after blood collection, 200 µl of the whole blood added with citric acid was added to a glass tube. Solutions were prepared by dissolving 205A43A thrombin, M-205A43A thrombin, and M-65A205A43A thrombin in PBS at a concentration of 100 µg/ml, respectively. 20 µl of each of the solutions and 20 µl of PBS were added to the glass tube. The blood added with each thrombin derivative was added with 30 µl of 0.1 M CaCl₂.
After the addition of calcium, fluidity was observed every 30 seconds to determine the time at which the fluidity was lost (i.e., coagulation time). As a result, the coagulation time was 4 minutes and 30 seconds for PBS, 7 minutes for the 205A43A thrombin, 8 minutes and 30 seconds for the M-205A43A thrombin, and 8 minutes and 30 seconds for the M-65A205A43A thrombin. The 205A43A thrombin, M-205A43A thrombin, and M-65A205A43A thrombin each exhibited a high anticoagulation ability even in whole blood.

### [Experimental Example 31]

### (1) Expression of thrombin (referred to as "19A205A43A thrombin") in which phenylalanine at position 19 in B chain is substituted by alanine, serine at position 205 is substituted by alanine, and histidine at position 43 is substituted by alanine

The 19A205A43A thrombin was expressed by the method of the above-mentioned section (1) in Experimental Example 1. SEQ ID NO:57 shows the nucleotide sequence of a gene encoding the 19A205A43A thrombin. The binding ability to a hirudin C-terminal peptide was confirmed according to the method of the above-mentioned section (2) in Experimental Example 1, and no band was observed in a flow-through fraction and a band equivalent to that of thrombin was observed at the elution peak. Subsequently, the purification was carried out by using sulfated cellulofine column and hirudin C-terminal peptide column according to the method of the above-mentioned section (3) in Experimental Example 1, to thereby obtain about 3 mg of almost purified 19A205A43A thrombin on SDS-PAGE.

(2) Solutions each obtained by dissolving 50 µg or 25 µg of the 19A205A43A thrombin in 1 ml of PBS were each added to standard plasma (Sysmex International Reagents Co., Ltd.) in a volume ratio of 1:1, and APTT was determined. A solution obtained by adding PBS to standard plasma (Sysmex International Reagents Co., Ltd.) in a volume ratio of 1:1 was used as a control, and APTT was determined. The APTT reagent available from Sysmex International Reagents Co., Ltd. was used. As a result, the APTT of the control was 46.5 seconds while the APTT of the 19A205A43A thrombin were 83.5 seconds and 79 seconds, respectively.
It was confirmed that the APTT-prolonging effect slightly increased than that before the substitution owing to the substitution of alanine for phenylalanine at position 19.

### (3) Comparison of binding specificity of 19A205A43A thrombin to Fbgn and FVIII

10 mM phosphate buffer (pH 7.7) of a 19A205A43A thrombin derivative at a concentration of about 0.1 mg/ml was added to an NHS-activated CM dextran cuvette (Nissan Sangyo Co., Ltd.). The solution was stirred at 25°C for 10 minutes to immobilize the test sample (i.e., thrombin derivative) on the NHS-activated CM dextran cuvette, to thereby obtain a 19A205A43A thrombin-immobilized cuvette. (About 1,000 arc was bound) Subsequently, 0.2 ml of 1 M ethanol amine (pH 8) was added thereto to perform a blocking treatment. The 19A205A43A cuvette was added with 100 nM of Fbgn and FVIII, to thereby monitor respective binding curves. Fig. 47 shows the results. It was confirmed that the Fbgn-binding ability decreased and the specificity to FVIII increased in the 19A205A43A cuvette as compared to that of the 205A43A thrombin.

### [Industrial Applicability]

The thrombin derivatives of the present invention can attain a stable antithrombotic effect even in a used amount smaller than that of a conventional thrombin derivative. The effect can be confirmed through the determination of APTT, the determination of the whole blood clotting time, or the platelet aggregation-inhibiting effect of plasma as described in the examples. In the present invention, the term "antithrombotic effect" means an antithrombotic effect including an antiplatelet effect and anti-blood coagulation which involve in the primary hemostasis and secondary hemostasis, as a whole. The thrombin derivatives of the present invention having the anti-platelet effect and anti-blood coagulation effect, for example: in the primary hemostasis, act on thrombin receptors mainly including GPIbα (i.e., platelet receptor glycoprotein Ibα) and PAR1 to inhibit stimulation to a platelet such as a carboxyl group-modified thrombin (hereinafter, also referred to as "M-thrombin"), ristocetin, or shearing stress, leading to inhibition of the aggregation and adhesion of the platelets; and in the secondary hemostasis, inhibit mainly the activation of FVIII to thereby specifically prolong APTT.

Meanwhile, the substitution of the active center amino acids and other amino acids provides thrombin derivatives having various aspects in the expression of the effects, such as a thrombin derivative which has a high APTT-prolonging effect but with a low antiplatelet effect, and a thrombin derivative which has high APTT-prolonging effect and high antiplatelet (only PAR1-inhibiting) effect. The thrombin derivatives of the present invention in which the active center amino acids and other amino acid are substituted inhibit the activation of protein C caused by thrombin on TM in a living body.
Furthermore, when the carboxyl group of a thrombin derivative of the present invention is modified, not only the APTT is prolonged but the antiplatelet effect, particularly inhibiting effect on the PAR1 activation and ristocetin-induced platelet aggregation can be imparted or controlled depending on the substitution of the amino acids. Thus, a thrombin derivative having an anti-blood coagulation effect and an antiplatelet effect which can be applied to various thrombosis can be obtained.

## Claims

1. A thrombin derivative comprising an A chain and a B chain, wherein the B chain has an amino acid sequence in which one or more kinds of active center amino acids selected from the group consisting of serine at position 205, glycine at position 203, aspartic acid at position 99, and histidine at position 43 in the amino acid sequence of a thrombin B chain are substituted, and wherein
(1) the thrombin derivative cleaves a thrombin substrate at the ratio of 10% or less when it is reacted with the thrombin substrate in 50 mM Tris-HCl (pH 7.4) containing 0.1 M NaCl at 37°C for 3 hours, and
(2) the thrombin derivative maintains a structure of exosite I.

2. A thrombin derivative comprising an A chain and a B chain, wherein the B chain has an amino acid sequence in which one or more kinds of active center amino acids selected from the group consisting of serine at position 205, glycine at position 203, aspartic acid at position 99, and histidine at position 43 in the amino acid sequence of a thrombin B chain are substituted, wherein
(1) the thrombin derivative cleaves a thrombin substrate at the ratio of 10% or less when it is reacted with the thrombin substrate in 50 mM Tris-HCl (pH 7.4) containing 0.1 M NaCl at 37°C for 3 hours, and
(2) the thrombin derivative maintains a binding ability to hirudin C-terminal peptide-immobilized gel.

3. The thrombin derivative according to claim 2, further maintaining a binding ability to heparin.

4. The thrombin derivative according to claim 1 or 2, wherein the thrombin substrate is a blood coagulation factor 13.

5. The thrombin derivative according to claim 1 or 2, wherein the thrombin substrate is fibrinogen.

6. The thrombin derivative according to any one of claims 1 to 5, wherein the substitutions of the active center amino acids are substitutions of two or more kinds of amino acids selected from the group consisting of serine at position 205, glycine at position 203, aspartic acid at position 99, and histidine at position 43 in a thrombin B chain.

7. The thrombin derivative according to any one of claims 1 to 6, wherein the substitutions of the active center amino acids comprises a substitution of serine at position 205.

8. The thrombin derivative according to any one of claims 1 to 7, wherein the substitution of the active center amino acids comprises substitutions of serine at position 205 and histidine at position 43.

9. The thrombin derivative according to claim 8, wherein the histidine at position 43 is substituted by alanine or serine.

10. The thrombin derivative according to any one of claims 6 to 9, wherein the serine at position 205 is substituted by alanine, threonine, or glycine.

11. The thrombin derivative according to any one of claims 6 to 9, wherein the serine at position 205 is substituted by alanine.

12. The thrombin derivative according to claim 8, wherein the serine at position 205 and the histidine at position 43 are substituted by alanine.

13. The thrombin derivative according to any one of claims 1 to 12, wherein its blood coagulation factor 8-binding ability is 10% or more of that of anhydrothrombin.

14. The thrombin derivative according to any one of claims 1 to 12, wherein its blood coagulation factor 8-binding ability is 80% or more of that of anhydrothrombin.

15. The thrombin derivative according to any one of claims 1 to 14, wherein VIIIA/FA, a ratio between a blood coagulation factor 8-binding ability (VIIIA) and a fibrinogen-binding ability (FA) of the thrombin derivative is 1.1 folds or more of VIIIa/Fa, a ratio between a blood coagulation factor 8-binding ability (VIIIa) and a fibrinogen-binding ability (Fa) of anhydrothrombin.

16. The thrombin derivative according to any one of claims 1 to 14, wherein VIIIA/FA, a ratio between a blood coagulation factor 8-binding ability (VIIIA) and a fibrinogen-binding ability (FA) of the thrombin derivative is 1.2 folds or more of VIIIa/Fa, a ratio between a blood coagulation factor 8-binding ability (VIIIa) and a fibrinogen-binding ability (Fa) of anhydrothrombin.

17. The thrombin derivative according to any one of claims 1 to 12, wherein an activated partial thromboplastin time of the thrombin derivative is 1.1 folds or more of that of anhydrothrombin.

18. The thrombin derivative according to any one of claims 1 to 12, wherein its fibrinogen-binding ability has been decreased by 10% or more and its activated partial thromboplastin time has been prolonged 1.1 folds or more owing to the amino acid substitutions of the active center amino acids.

19. A thrombin derivative comprising an A chain and a B chain, wherein the B chain has an amino acid sequence in which serine at position 205 and one or more kinds of amino acids selected from the group consisting of glycine at position 203, aspartic acid at position 99, and histidine at position 43, in the amino acid sequence of a thrombin B chain are substituted.

20. A thrombin derivative comprising an A chain and a B chain, wherein the B chain has an amino acid sequence in which serine at position 205 and histidine at position 43 in the amino acid sequence of a thrombin B chain are substituted.

21. The thrombin derivative according to any one of claims 1 to 20, wherein the B chain has an amino acid sequence in which amino acids except the active center amino acids are further substituted.

22. The thrombin derivative according to claim 21, wherein the amino acids except the active center amino acids are amino acids in an exosite I region of thrombin.

23. The thrombin derivative according to claim 22, wherein the amino acids in the exosite I region of thrombin are basic amino acids.

24. The thrombin derivative according to claim 22, wherein the amino acids in the exosite I region of thrombin are one or more amino acids selected from the group consisting of glutamine at position 24, lysine at position 65, and lysine at position 77 in a thrombin B chain.

25. The thrombin derivative according to any one of claims 21 to 24, wherein VIIIA/FA, a ratio between a blood coagulation factor 8-binding ability (VIIIA) and a fibrinogen-binding ability (FA) of the thrombin derivative is 1.1 folds or more of VIIIa/Fa, a ratio between a blood coagulation factor 8-binding ability (VIIIa) and a fibrinogen-binding ability (Fa) of a thrombin derivative before the amino acids except the active center amino acids are substituted.

26. The thrombin derivative according to any one of claims 21 to 24, wherein VIIIA/FA, a ratio between a blood coagulation factor 8-binding ability (VIIIA) and a fibrinogen-binding ability (FA) of the thrombin derivative is 1.5 folds or more of VIIIa/Fa, a ratio between a blood coagulation factor 8-binding ability (VIIIa) and a fibrinogen-binding ability (Fa) of a thrombin derivative before the amino acids except the active center amino acids are substituted.

27. The thrombin derivative according to any one of claims 21 to 24, said thrombin derivative having:
any one of the effects selected from the group consisting of an APTT-prolonging effect, a thrombin receptor activation-inhibiting effect, and a ristocetin-induced platelet aggregation-inhibiting effect; and having
a decreased thrombomodulin-binding ability as compared to that of a thrombin derivative before the amino acids except the active center amino acids are substituted.

28. The thrombin derivative according to any one of claims 21 to 24, said thrombin derivative having:
any one of the effects selected from the group consisting of an APTT-prolonging effect, a thrombin receptor activation-inhibiting effect, and a ristocetin-induced platelet aggregation-inhibiting effect; and having
a thrombomodulin-binding ability decreased by 10% or more as compared to that of a thrombin derivative before the amino acids except the active center amino acids are substituted.

29. The thrombin derivative according to any one of claims 21 to 24, wherein VIIIA/TMA, a ratio between a blood coagulation factor 8-binding ability (VIIIA) and a thrombomodulin-binding ability (TMA) of the thrombin derivative is 1.1 folds or more of VIIIa/TMa, a ratio between a blood coagulation factor 8-binding ability (VIIIa) and a thrombomodulin-binding ability (TMa) of a thrombin derivative before the amino acids except the active center amino acids are substituted.

30. The thrombin derivative according to any one of claims 21 to 24, wherein VIIIA/TMA, a ratio between a blood coagulation factor 8-binding ability (VIIIA) and a thrombomodulin-binding ability (TMA) of the thrombin derivative is 1.5 folds or more of VIIIa/TMa, a ratio between a blood coagulation factor 8-binding ability (VIIIa) and a thrombomodulin-binding ability (TMa) of a thrombin derivative before the amino acids except the active center amino acids are substituted.

31. The thrombin derivative according to claim 21, wherein the amino acids except the active center amino acids are amino acids in an exosite II region of thrombin, and its antithrombotic ability is maintained while its heparin-binding ability is decreased.

32. The thrombin derivative according to claim 31, wherein the amino acids in the exosite II of thrombin are one or more kinds of amino acids selected from the group consisting of arginine at position 98, arginine at position 245, lysine at position 248, and lysine position 252 in a thrombin B chain.

33. The thrombin derivative according to claim 31 or 32, wherein the heparin-binding ability of the thrombin derivative is 90% or less as compared to that of a thrombin before the amino acids except the active center amino acids are substituted.

34. The thrombin derivative according to any one of claims 21 to 33, wherein one or more kinds of antithrombotic effects selected from the group consisting of an activated partial thromboplastin time-prolonging effect, an modified thrombin-induced platelet aggregation-inhibiting effect, and a ristocetin-induced platelet aggregation-inhibiting effect are enhanced.

35. The thrombin derivative according to any one of claims 21 to 33, wherein the activated partial thromboplastin time of the thrombin derivative is 1.1 folds or more as compared to that of anhydrothrombin.

36. The thrombin derivative according to claim 31 or 32, wherein the activated partial thromboplastin time of the thrombin derivative is 1.5 folds or more as compared to that of a thrombin before the amino acids except the active center amino acids are substituted, and its thrombomodulin-binding ability is decreased to 50% or less as compared to that of a thrombin before the amino acids except the active center amino acids are substituted.

37. The thrombin derivative according to any one of claims 1 to 36, wherein the amino acid sequence of the thrombin B chain is an amino acid sequence of a B chain of human wild-type thrombin.

38. The thrombin derivative according to claim 37, wherein the amino acid sequence of the B chain of the human wild-type thrombin is an amino acid sequence from position 50 to position 308 of SEQ ID NO: 2.

39. The thrombin derivative according to any one of claims 1 to 38, wherein its carboxyl group is modified.

40. The thrombin derivative according to claim 39, wherein the carboxyl group is modified by an ester of an amino acid.

41. The thrombin derivative according to claim 39, wherein the carboxyl group is modified by polyethylene glycol.

42. The thrombin derivative according to claim 39, wherein the carboxyl group is modified by polyethylene glycol having an amino group.

43. The thrombin derivative according to claim 41 or 42, wherein the polyethylene glycol is polyethylene glycol having a molecular weight of 1,000 or less.

44. The thrombin derivative according to claim 39, wherein the carboxyl group is modified by carbodiimide.

45. The thrombin derivative according to claim 39, wherein at least 3 carboxyl groups per molecule are modified.

46. The thrombin derivative according to claim 39, wherein 25 or less carboxyl groups per molecule are modified.

47. The thrombin derivative according to claim 39, wherein at least a carboxyl group of glutamic acid at position 25 in the B chain is modified.

48. The thrombin derivative according to any one of claims 1 to 47, having a PAR1 activation-inhibiting effect and/or a ristocetin-induced platelet aggregation-inhibiting effect.

49. A DNA encoding the thrombin derivative according to any one of claims 1 to 38.

50. A pharmaceutical composition, comprising the thrombin derivative according to any one of claims 1 to 48.

51. The pharmaceutical composition according to claim 50, which is an antithrombotic agent.

52. The pharmaceutical composition according to claim 50, which is an anti-inflammatory agent.

53. The pharmaceutical composition according to claim 50, which is a platelet aggregation-inhibiting agent.

54. The pharmaceutical composition according to claim 50, which is a platelet adhesion-inhibiting agent.

55. The pharmaceutical composition according to claim 50, which is an endogenous blood coagulation-inhibiting agent.

56. The pharmaceutical composition according to claim 50, which is a thrombin receptor activation-inhibiting agent.

57. The pharmaceutical composition according to claim 50, having both an anti-blood coagulation effect and an antiplatelet effect.
